(19) Europäisches Patentamt / European Patent Office / Office européen des brevets

(11) **EP 3 166 639 B1**

(12) **EUROPÄISCHE PATENTSCHRIFT**

(45) Veröffentlichungstag und Bekanntmachung des Hinweises auf die Patenterteilung:
**25.03.2020   Patentblatt 2020/13**

(51) Int Cl.:
*A61K 45/06* (2006.01)         *A61K 31/205* (2006.01)
*G01N 33/02* (2006.01)         *G01N 33/50* (2006.01)
*A23L 33/00* (2016.01)         *A23L 33/105* (2016.01)
*A23L 33/15* (2016.01)         *A23L 33/16* (2016.01)
*A23L 33/175* (2016.01)        *A23L 33/21* (2016.01)
*A23L 33/155* (2016.01)

(21) Anmeldenummer: **15734694.1**

(22) Anmeldetag: **08.07.2015**

(86) Internationale Anmeldenummer:
**PCT/EP2015/065551**

(87) Internationale Veröffentlichungsnummer:
**WO 2016/005425 (14.01.2016 Gazette 2016/02)**

(54) **METHODE ZUR BEWERTUNG VON LEBENSMITTELN UND ERNÄHRUNGSSYSTEM ZUR PRÄVENTION UND THERAPIE VON CHRONISCHEN ERKRANKUNGEN**

METHODS FOR EVALUATING FOOD AND FEEDING SYSTEM FOR THE PREVENTION AND TREATMENT OF CHRONIC DISEASES

PROCÉDÉS D'ÉVALUATION DE PRODUITS ALIMENTAIRES ET SYSTÈMES DE NUTRITION DE PRÉVENTION ET THÉRAPIE DE MALADIES CHRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.07.2014   EP 14176455**

(43) Veröffentlichungstag der Anmeldung:
**17.05.2017   Patentblatt 2017/20**

(73) Patentinhaber: **EGB EpiGeneticBalance AG**
**4313 Möhlin (CH)**

(72) Erfinder: **ROHNER, Markus**
**CH-4313 Möhlin (CH)**

(74) Vertreter: **Latscha Schöllhorn Partner AG**
**Grellingerstrasse 60**
**4052 Basel (CH)**

(56) Entgegenhaltungen:
**WO-A1-2010/143053    WO-A1-2013/078658**
**WO-A2-2011/150229**

- SCHUSDZIARRA V ET AL: "Satt essen und abnehmen", MMW-FORTSCHRITTE DER MEDIZIN, Bd. 150, Nr. 28-31, 2008, Seiten 55-58, XP002734163,
- SUSAN B. ROBERTS ET AL: "Effects of dietary factors on energy regulation: Consideration of multiple- versus single-dietary-factor models", PHYSIOLOGY & BEHAVIOR, Bd. 134, 1. Juli 2014 (2014-07-01), Seiten 15-19, XP055160171, ISSN: 0031-9384, DOI: 10.1016/j.physbeh.2014.04.024
- DORA ROMAGUERA ET AL: "Food Composition of the Diet in Relation to Changes in Waist Circumference Adjusted for Body Mass Index", PLOS ONE, Bd. 6, Nr. 8, 17. August 2011 (2011-08-17) , Seite e23384, XP055160173, DOI: 10.1371/journal.pone.0023384
- BURGIO ERNESTO ET AL: "Obesity and diabetes: from genetics to epigenetics", MOLECULAR BIOLOGY REPORTS, SPRINGER NETHERLANDS, NL, Bd. 42, Nr. 4, 25. September 2014 (2014-09-25), Seiten 799-818, XP035466308, ISSN: 0301-4851, DOI: 10.1007/S11033-014-3751-Z [gefunden am 2014-09-25]

- **BEITULLAH ALIPOUR ET AL: "Effect of L-Carnitine Supplementation on Metabolic Status in Obese Diabetic Women With Hypocaloric Diet", HEALTH SCOPE, vol. 3, no. 1, 17 February 2014 (2014-02-17), page 14615, XP55560765, ISSN: 2251-8959, DOI: 10.17795/jhealthscope-14615**

**Beschreibung**

**Technisches Gebiet**

Die Erfindung betrifft eine Methode zur Bewertung von Lebensmitteln.

**Stand der Technik**

Lebensmittelinhaltstoffe oder durch deren Aufnahme und Umwandlung gebildete Metaboliten beeinflussen die Gesundheit und können somit einen Einfluss auf chronische Krankheiten des Menschen haben. Bisher wurde vor allem der glykämische Index bzw. die glykämische Last herangezogen, um den Einfluss von Lebensmitteln auf die Gesundheit vor allem den Einfluss auf Prävention und Therapie von Diabetes Typ 2 zu bewerten. Doch hat sich herausgestellt, dass der glykämische Index bzw. die glykämische Last ungenügend mit dem Risiko für chronische Krankheiten vor allem mit dem Risiko von Diabetes Typ 2 korrelieren (J Nutr. 2013 Jan;143(1):93-9. doi: 10.3945/jn.112.165605. Epub 2012 Nov 28). Schusdziarra et al., MMW-Fortschr.Med Nr. 28-31/2008, beschreiben eine Methode zur Bewertung eines Lebensmittels, wobei die Energiedichte des Lebensmittels und die Kategorisierung des Lebensmittels im Hinblick auf seinen Einfluss auf den Energiestoffwechsel eines Menschen bestimmt wird.

Daher besteht ein Bedarf nach anderen Bewertungssystemen für Lebensmittel um ihren Einfluss auf die Gesundheit beurteilen zu können und insbesondere eine gezielte Auswahl von Lebensmitteln treffen zu können, die für die Prävention und Therapie von chronischen Krankheiten vorteilhaft ist.

**Darstellung der Erfindung**

Der Schutzumfang der Erfindung wird durch die Ansprüche 1 bis 12 definiert. Lebensmittel enthalten verschiedenste Inhaltstoffe je nach Quelle oder Verarbeitung des Lebensmittels, welche die Genexpression über epigenetische Mechanismen und damit über verschiedene epigenetische Reaktionen beeinflussen. Lebensmittel können mit der Bewertungsmethode der vorliegenden Erfindung neu über eine Kategorisierung der epigenetischen Wirkung der Lebensmittel bzw. deren Inhaltsstoffe/Zwischenmetaboliten im Hinblick auf ihren Einfluss auf den Energiestoffwechsel eines Säugers ausgewählt werden. Die Anwendung der auf der vorliegenden Bewertungsmethode basierenden, im folgenden "Epigenetischer Index" genannten Kategorisierung der Lebensmittel bei der Auswahl der Lebensmittels können die Prävention und Therapie chronischer Krankheiten positiv beeinflussen. Dabei wiederspiegelt der epigenetische Index eine Wertigkeit eines Lebensmittels im Hinblick der möglichen Wirkung über verschiedenste epigenetische Reaktionen auf die Genexpression und Aktivierung von Genabschnitten zur Bereitstellung von Enzymsystemen für die auf- und abbauenden Stoffwechselwege. Es wird vermutet, dass die Anwendung der vorliegenden Bewertungmethode von Lebensmitteln und die entsprechende Auswahl von Lebensmittel sogar über transgenerationelle Effekte die Gesundheit des Nachwuchses bereits im Uterus vor allem seine Prädispostion für Übergewicht schon pränatal positiv beeinflusst.

**Kurze Beschreibung der Zeichnungen**

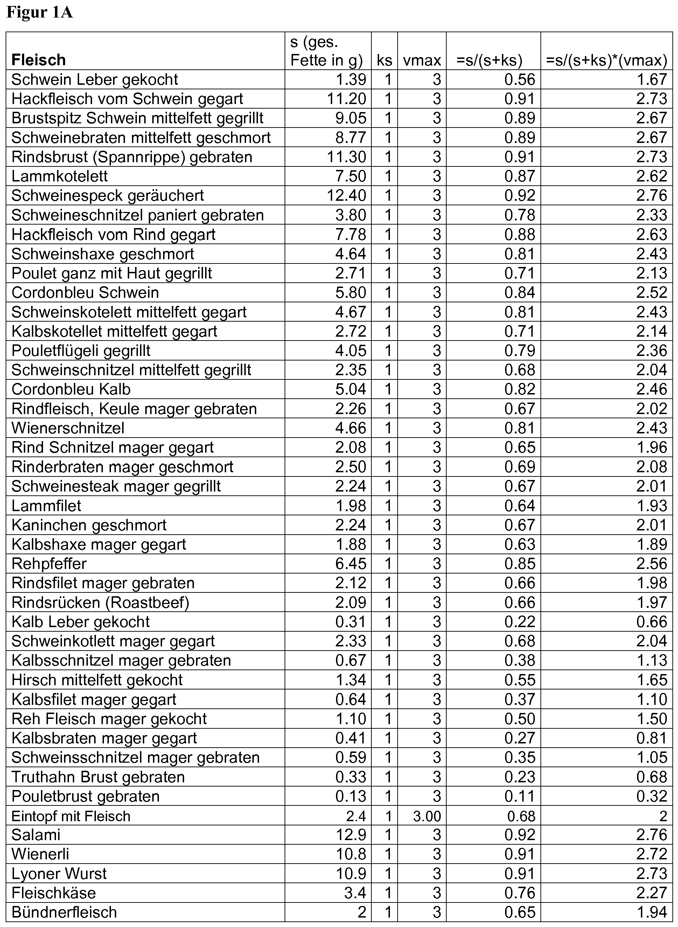

Figur 1A-C zeigt die Konzentrationen von gesättigten Fetten und Cholesterin und die Energiedichte von verschiedenen Lebensmitteln der Lebensmittelklasse A) (Fleischerzeugnisse), die für die Berechung des EPI Index massgeblichen Parameter, sowie den für jedes aufgeführte Lebensmittel berechneten EPI Index.

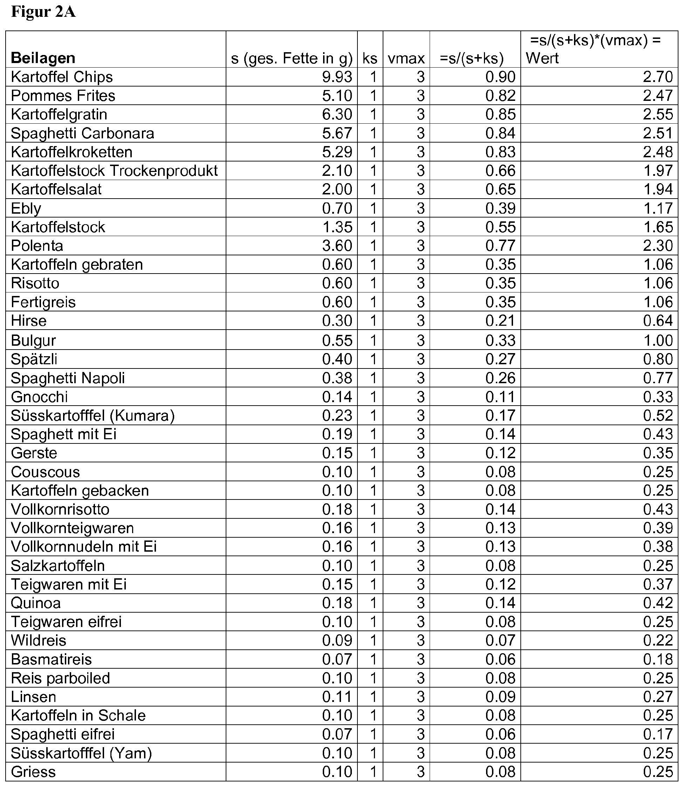

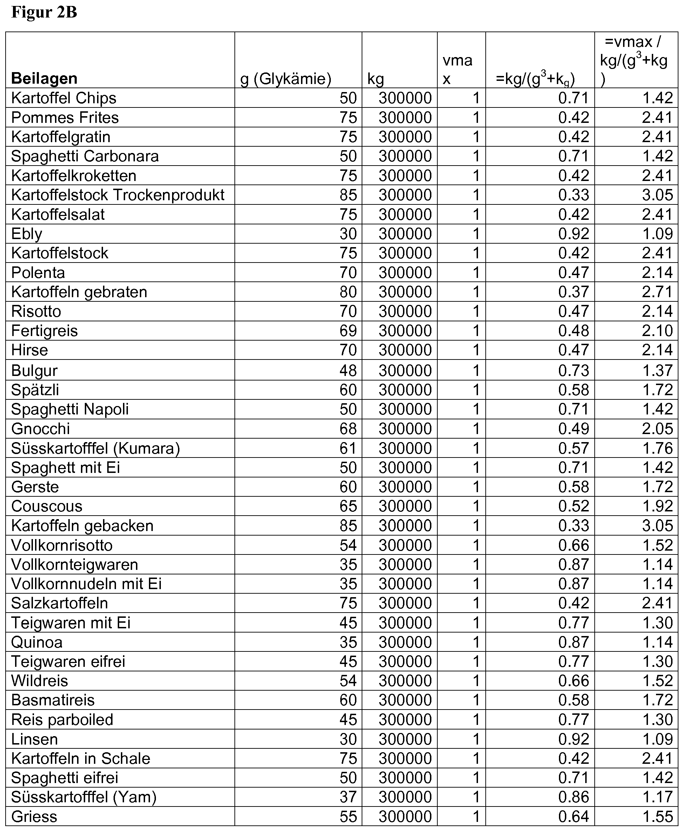

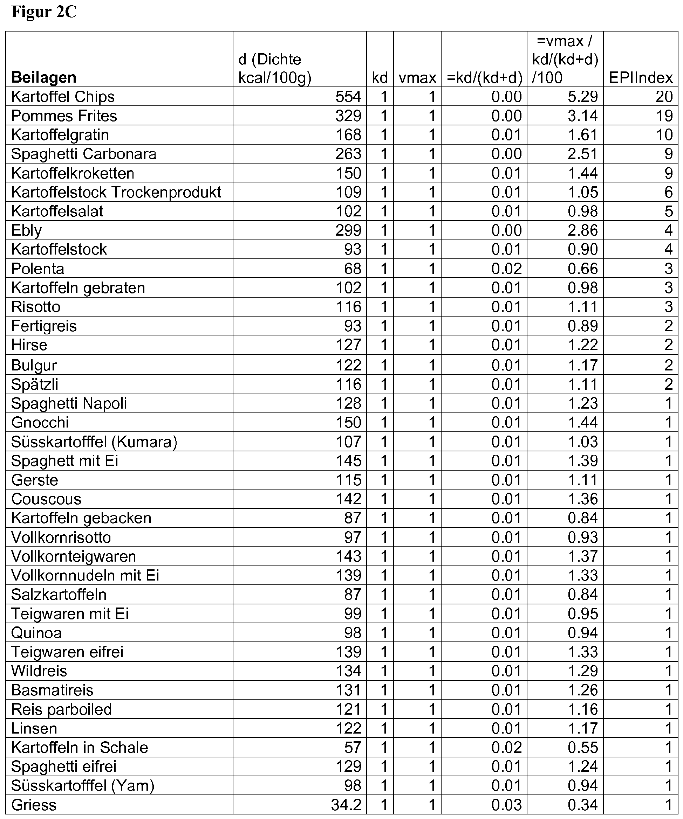

Figur 2A-C zeigt die Konzentration von gesättigten Fetten, den glykämischen Index und die Energiedichte von verschiedenen Lebensmitteln der Lebensmittelklasse B) (Beilagen), die für die Berechung des EPI Index massgeblichen Parameter, sowie den für jedes aufgeführte Lebensmittel berechneten EPI Index.

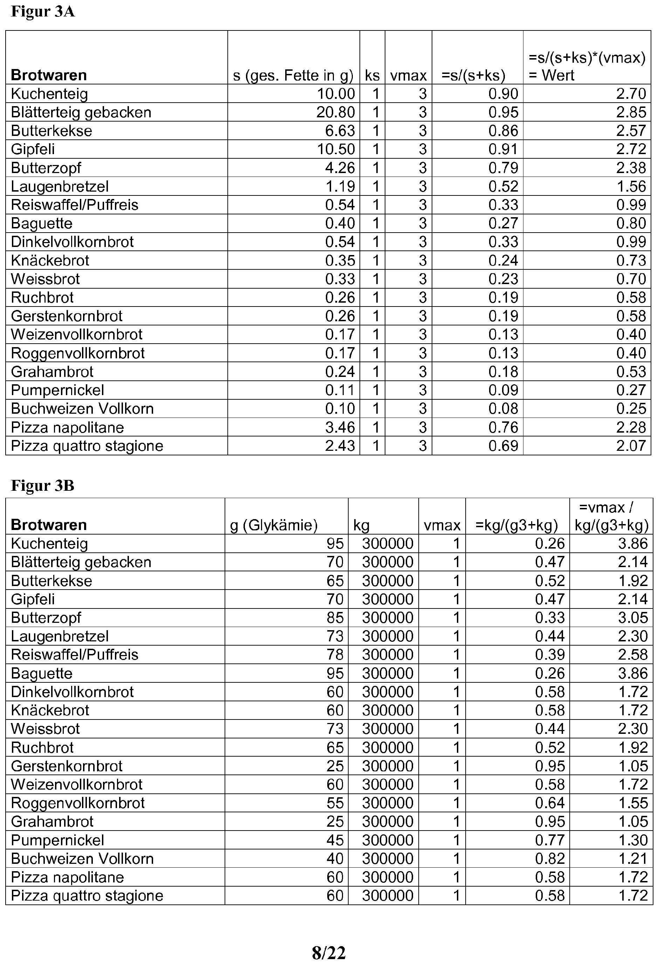

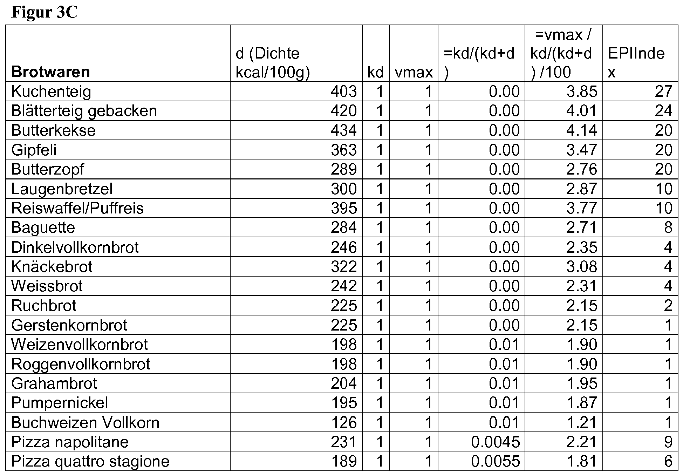

Figur 3A-C zeigt die Konzentration von gesättigten Fetten, den glykämischen Index und die Energiedichte von verschiedenen Lebensmitteln der Lebensmittelklasse B) (Broterzeugnisse), die für die Berechung des EPI Index massgeblichen Parameter, sowie den für jedes aufgeführte Lebensmittel berechneten EPI Index.

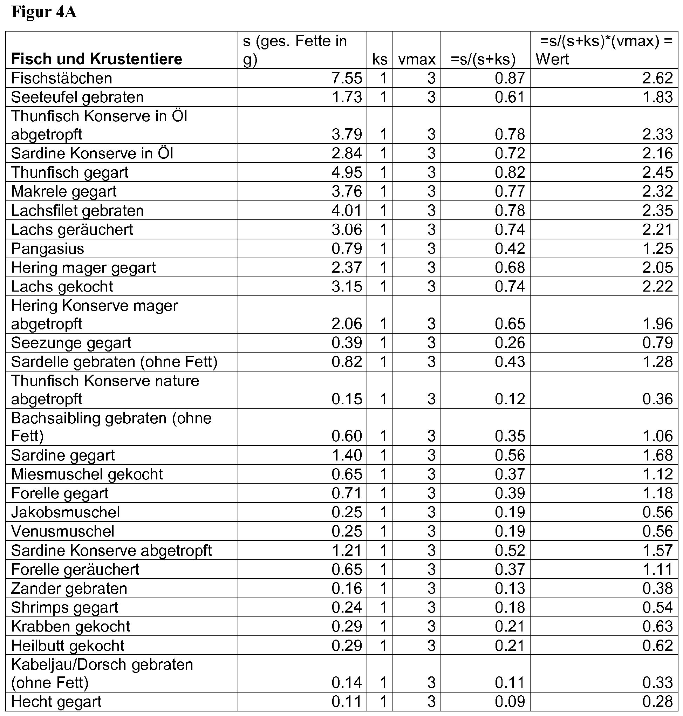

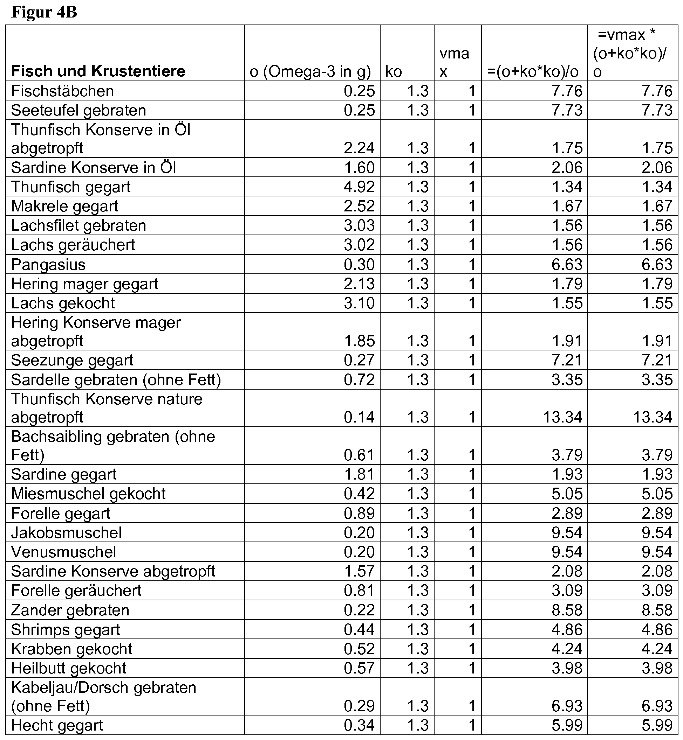

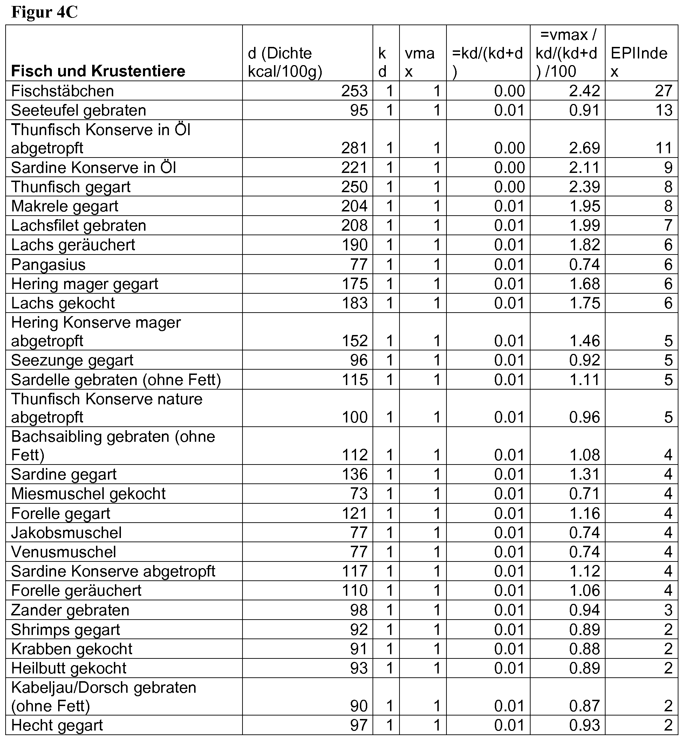

Figur 4A-C zeigt die Konzentrationen von gesättigten Fetten und Omega-3-Fettsäuren und die Energiedichte von verschiedenen Lebensmitteln der Lebensmittelklasse C) (Fischerzeugnisse), die für die Berechung des EPI Index massgeblichen Parameter, sowie den für jedes aufgeführte Lebensmittel berechneten EPI Index.

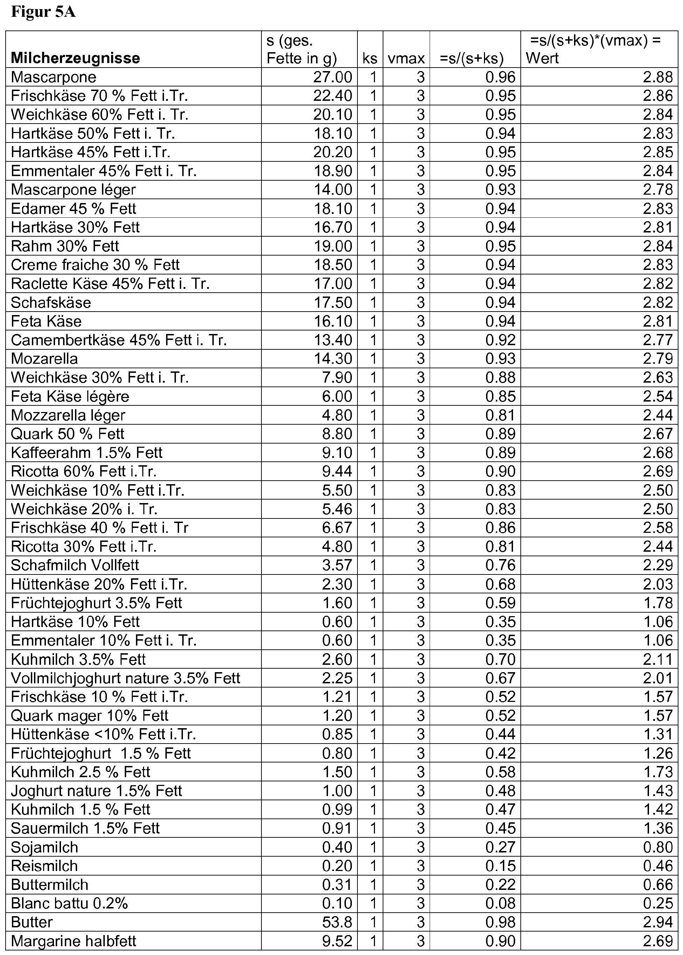

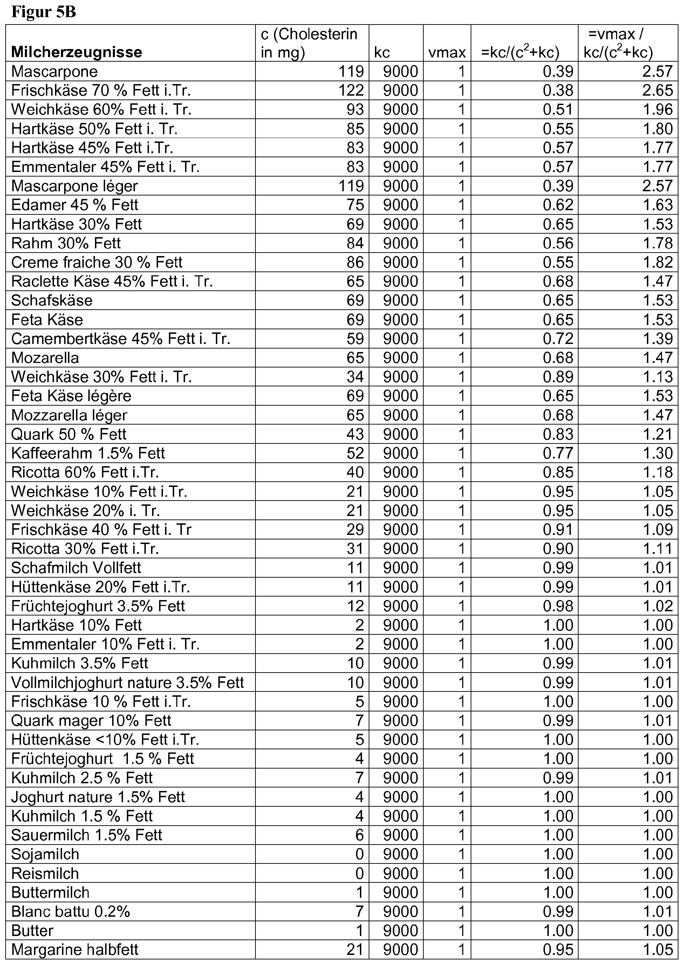

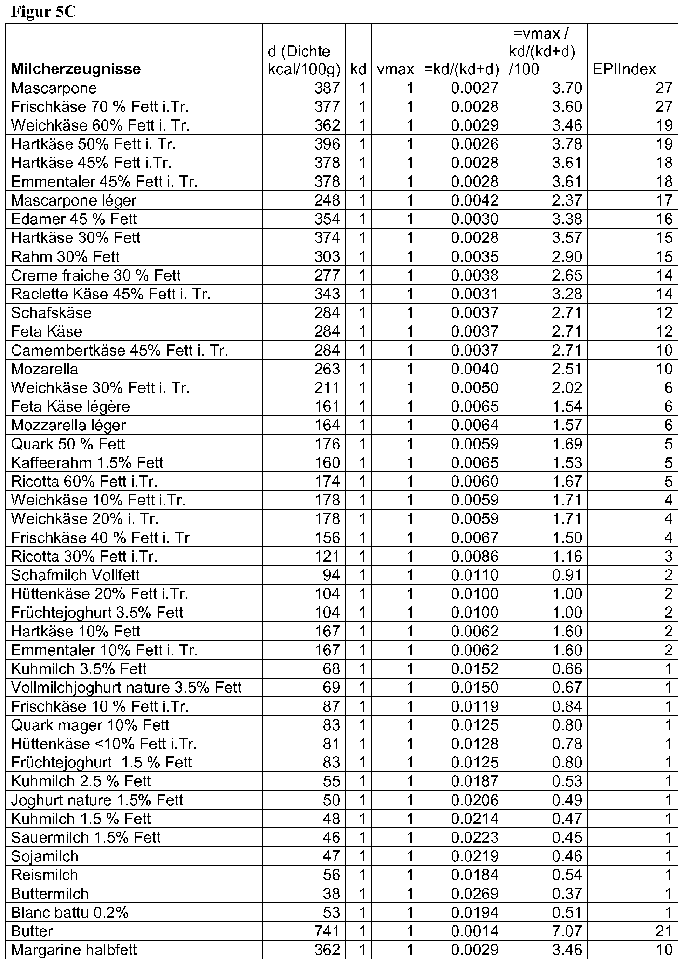

Figur 5A-C zeigt die Konzentrationen von gesättigten Fetten und Cholesterin und die Energiedichte von verschiedenen Lebensmitteln der Lebensmittelklasse D) (Milcherzeugnisse), die für die Berechung des EPI Index massgeblichen Parameter, sowie den für jedes aufgeführte Lebensmittel berechneten EPI Index.

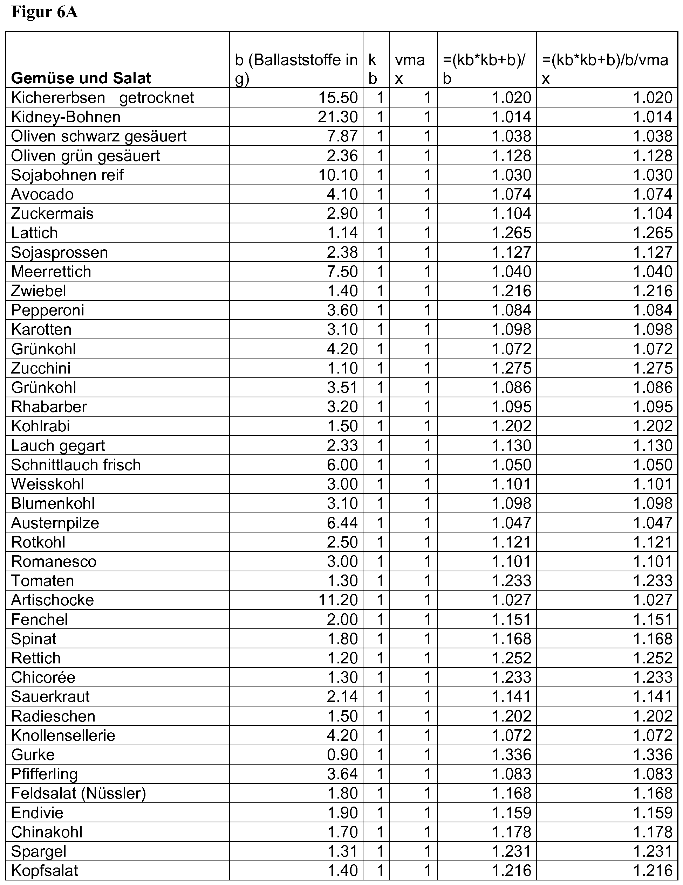

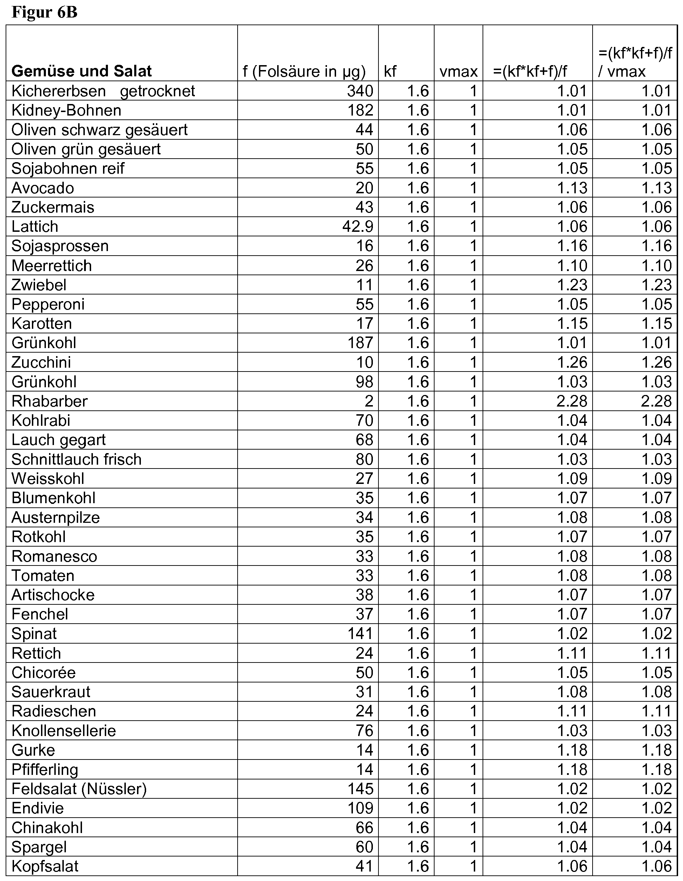

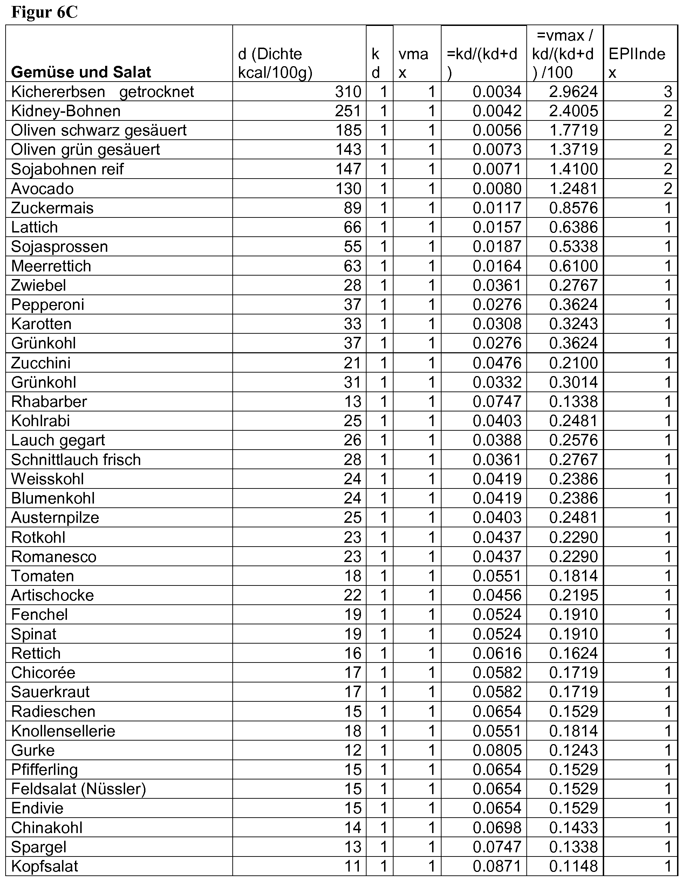

Figur 6A-C zeigt die Konzentrationen von Ballaststoffen und Folsäure und die Energiedichte von verschiedenen Lebensmitteln der Lebensmittelklasse E) (Gemüse), die für die Berechung des EPI Index massgeblichen Parameter, sowie den für jedes aufgeführte Lebensmittel berechneten EPI Index.

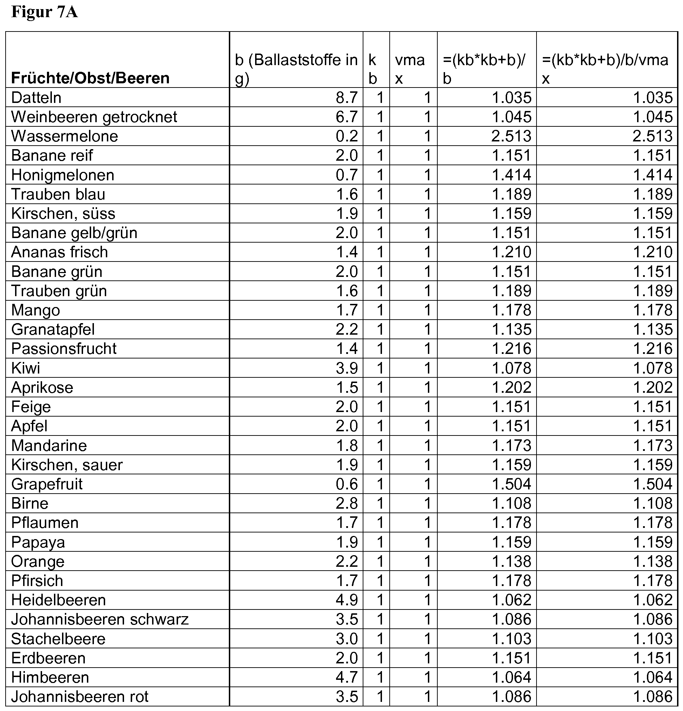

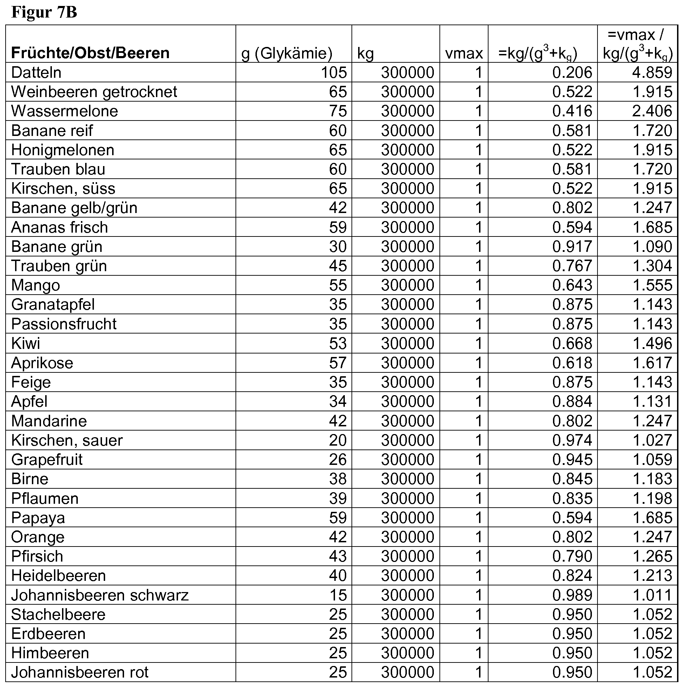

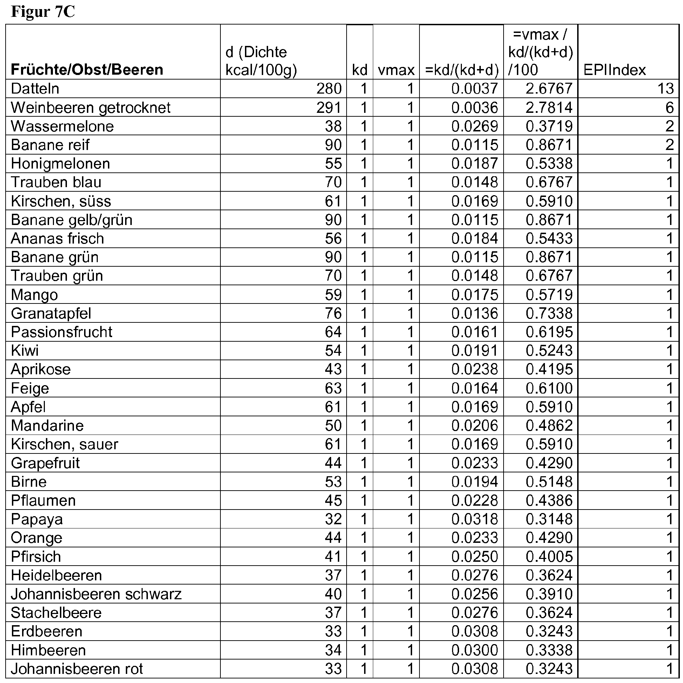

Figur 7A-C zeigt die Konzentration von Ballaststoffen, den glykämischen Index und die Energiedichte von verschiedenen Lebensmitteln der Lebensmittelklasse F) (Früchte), die für die Berechung des EPI Index massgeblichen Parameter, sowie den für jedes aufgeführte Lebensmittel berechneten EPI Index.

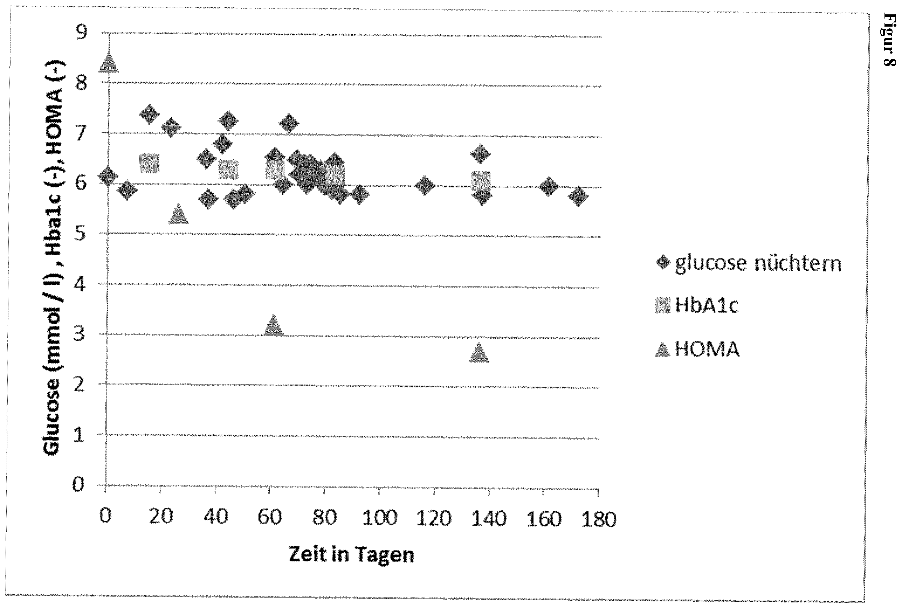

Figur 8 zeigt den zeitlichen Verlauf (in Tagen) der Glucose-Messwerte (Rhomben) jeweils morgens gemessen, den zeitlichen Verlauf (in Tagen) des HbA1c (Quadrate) Messwertes, sowie den zeitlichen Verlauf (in Tagen) des HOMA Indexes (Dreiecke) über die 3 Phasen der Diabetes Typ 2 Therapie. Die Phase I dauerte 63 Tage, die Phase II 74 Tage und die Phase III 2 Monate. Alle Blutwerte sind nüchtern gemessen.

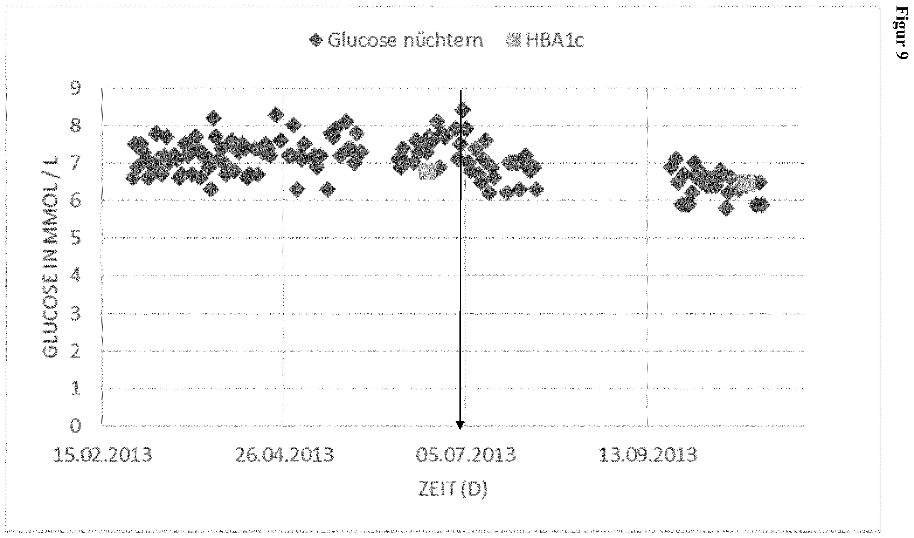

Figur 9 zeigt den zeitlichen Verlauf (in Tagen) der Glucose-Messwerte (Rhomben) jeweils morgens gemessen, sowie den zeitlichen Verlauf (in Tagen) des HbA1c (Quadrate) Messwertes. Der Start der Therapie ist mit dem Pfeil angegeben. Alle Blutwerte sind nüchtern gemessen.

## Deatillierte Beschreibung der Erfindung

Bestimmte Ausdrücke werden in der folgenden Beschreibung aus praktischen Gründen verwendet und sind nicht einschränkend zu verstehen.

Unter Lebensmittel werden hierin Stoffe verstanden, die ein Säuger wie bzw. der Mensch als Nahrungsmittel aufnimmt und im wesentlichen aus den Makronährstoffen Kohlenhydrate, Lipide (Fette) und Proteine bestehen. Zusätzlich sind Mikronährstoffe und Spurenelemente wesentliche Bestandteile von Lebenssmitteln. Jedes Lebensmittel besteht aus einem Anteil, der tatsächlich aufgenommen und verwertet wird. Dieser Anteil wird als "essbarer Anteil" eines Lebensmittels bezeichnet. Darüber hinaus kann das Lebensmittel Anteile wie Knochen und Ähnliches enthalten, die nicht verwertet werden. Die Konzentrations- und Mengenangaben eines im Lebensmittel enthaltenen epigenetisch aktiven Inhaltsstoffs beziehen sich hierin zweckmässig auf den essbaren Anteil des Lebensmittels.

Zweckmässig wird zwischen folgenden Klassen von Lebensmitteln unterschieden:

A) Fleischerzeugnisse, B) Beilagen und/oder Broterzeugnisse, C) Fischerzeugnisse, D) Milcherzeugnisse, E) Gemüse und F) Früchte, wobei die Produkte naturbelassen oder industriell gefertigt sein können.

A) Fleischerzeugnisse umfassen beispielsweise Lebensmittel, die ausschließlich oder überwiegend aus Fleisch, Haarwild, Geflügel und/oder Federwild hergestellt sind. Für die Herstellung von Fleischerzeugnissen finden das Muskelfleisch, Blut, Innereien, Darm, Schwarte und Fett Verwendung. Fleischerzeugnisse sind durch Garen, Säuern, Trocknen, Pökeln, Räuchern oder Kombinationen daraus haltbar gemacht. Zu den Fleischerzeugnissen gehören beispielsweise Speck, Schinken, Wurst und Pökelwaren, Fleischkonserven getückeltes Fleisch wie Gulasch oder Geschnetzeltes, gewolftes Fleisch wie Hackfleisch sowie aus kleineren Fleischstücken geformtes Fleisch, so genanntes Formfleisch. Der mengenmäßig größte Anteil der Fleischerzeugnisse fällt auf Wurst und Pökelwaren. Fleischerzeugnisse können in Fleischwaren und Wurstwaren unterscheiden werden. Fleischwaren unterscheiden sich von Wurstwaren dadurch, dass die Fleischfaser bei der Herstellung ganz bleibt, während bei Wurstwaren die Fleischfasern zerkleinert werden. Wurstwaren unterteilen sich nach den Herstellungververfahren in Kochwurst, Brühwurst und Rohwurst.

B) Beilagen und/oder Broterzeugnisse, umfassen beispielsweise kohlenhydratreiche Lebensmittel, die bei einer Mahlzeit vor allem Kalorien und Ballaststoffe liefern sollen, um zur Sättigung beizutragen. Salate aus Kartoffeln und Nudeln sind verbreitete Beilagen. Beilagen umfassen z.B. auch Teigwaren: Nudeln, Spätzle; Pflanzenknollen: Kartoffel, Süßkartoffel, Topinambur; Klöße wie Knödel und Nocken; verschiedene Getreidearten wie Reis und Hirse sowie Mais (als Polenta; Zuckermais aber zubereitet wie Gemüse), Erbspüree. Broterzeugnisse umfassen Brotmischungen, Brote aller Art wie Maisbrot, Zwiebelbrot, Dampfbrötchen mit Hefe, Kuchenbrot mit Hefe und Sultaninen, Leinsamenbrot mit Hefe, Roggen/Weizen-Mischbrot mit Hefe, Röstbrotwürfel, Weizenschrotbrot mit Hefe, Weizenschrotsemmel mit Hefe, Weizen-Weißbrot, Paniermehl und Semmelbrösel sowie Pizza.

C) Fischerzeugnisse umfassen zum Beispiel Fische, sonstige Meeres- oder Süßwassertiere, Teile dieser Tiere, einschließlich Rogen und Milch und Erzeugnisse aus diesen Tieren, auch in Verbindung mit anderen Lebensmitteln, soweit deren Anteil nicht überwiegt.

D) Milcherzeugnisse umfassen zum Beispiel Milchprodukte oder Molkereiprodukte, deren Zutaten hauptsächlich aus Milch oder Milchbestandteilen (Milcheiweiß, Milchfett oder Milchzucker) besteht. Zu den Milcherzeugnissen gehören einerseits verschieden behandelte Milcharten und andererseits durch Fermentation oder Extraktion von Milchbestandteilen gewonnene Produkte der Milch, wie beispielsweise Butter oder Margarine.

E) Gemüse umfassen zum Beispiel Salate, Kohlsorten, Blütengemüse, Fruchtgemüse, Wurzelgemüse, Knollengemüse, Zwiebelgemüse, und Hülsenfrüchte (*Helianthus tuberosus*) (Korbblütler).

F) Früchte umfassen zum Beispiel Äpfel, Birnen, Kirschen, Zwetschgen, Aprikosen, Erdbeeren, Himbeeren, Heidelbeeren, Brombeeren, und Johannisbeeren.

Unter "Energiedichte" oder "Energiedichte eines Lebensmittels" wird hierin zweckmässig der Energieinhalt (in kcal oder KJ) pro Gewichtseinheit, zweckmässig bezogen auf 100 g essbarer Anteil, verstanden.

Unter "Energiestoffwechsel" oder "Energiestoffwechsel eines Säugers" werden hierin alle Stoffwechselwege verstanden, die zur Energiebereitstellung gezählt werden, insbesondere die Fett- und Zuckerstoffwechsel wie die Glykolyse, sowie der Proteinstoffwechsel.

Unter "epigenetisch aktive Inhaltsstoffe" bzw. "epigenetisch aktive Inhaltsstoffe eines Lebensmittels" werden hierin Stoffe bzw. deren Metabolite verstanden, die bei der Aufnahme durch einen Säuger epigenetische Reaktionen wie Methylierung, Acetylierung und/oder Palmitoylierung eines oder mehrerer Gene oder eines oder mehrerer Histone auslösen oder beeinflussen können. Als epigenetisch aktive Inhaltsstoffe werden zweckmässig gesättigte Fette, Ballaststoffe, Folsäure, Omega-6-Fettsäuren, Omega-3-Fettsäuren, Cholesterin, Kohlenhydrate (hoch und/oder niedrig glykämische Kohlenhydrate), trans-Fettsäuren, Cholin, Vitamin B12, Betain, miRNA29, miRNA 103, miRNA 107, Resveratrol, Epigallocatechin-3-Gallat, Isoflavonoide und Sulforaphane ausgewählt. Ebenso zweckmässig werden epigenetisch aktive Inhaltsstoffe eines Lebensmittels aus der Gruppe bestehend aus gesättigte Fette, Ballaststoffe, Folsäure, Omega-6-Fettsäuren, Omega-3-Fettsäuren, Cholesterin, Kohlenhydrate (hoch und/oder niedrig glykämische Kohlenhydrate), trans-Fettsäuren, Cholin, Vitamin B12, Betain, miRNA29, miRNA 103, miRNA 107, Resveratrol, Epigallocatechin-3-Gallat, Isoflavonoide, Sulforaphane, Vitamin B6, Cystein and Glutathion ausgewählt. Vorteilhaft werden epigenetisch aktive Inhaltsstoffe des Lebensmittels aus der Gruppe bestehend aus gesättigte Fette, Ballaststoffe, Folsäure, Omega-6-Fettsäuren, Omega-3-Fettsäuren, Cholesterin, Kohlenhydrate (hoch und/oder niedrig glykämische Kohlenhydrate), Cholin, Vitamin B12, Betain, miRNA29, miRNA 103, miRNA 107, Resveratrol, Epigallocatechin-3-Gallat, Isoflavonoide, Sulforaphane, Vitamin B6, Cystein and Glutathion ausgewählt. Bevorzugte epigenetisch aktive Inhaltsstoffe sind epigenetisch aktive Inhaltsstoffe ausgewählt aus der Gruppe bestehend aus Palmitin, Myristin, Butyrat, Ballaststoffe, Folsäure, Omega-3-Fettsäuren, Cholesterin, Arachidonsäure, Docosahexaensäure, Fructose, Glucose, Maltose, Cholin Vitamin B12, Betain, miRNA29, miRNA 103, miRNA 107, Resveratrol, Epigallocatechin-3-Gallat, Genistein und Sulforaphane. Ebenso bevorzugte epigenetisch aktive Inhaltsstoffe sind epigenetisch aktive Inhaltsstoffe ausgewählt aus der Gruppe bestehend ausPalmitin, Myristin, Butyrat, Ballaststoffe, Folsäure, Omega-3-Fettsäuren, Cholesterin, Arachidonsäure, Docosahexaensäure, Fructose, Glucose, Maltose, Cholin Vitamin B12, Betain, miRNA29, miRNA 103, miRNA 107, Resveratrol, Epigallocatechin-3-Gallat, Genistein, Sulforaphane, Vitamin B6, Cystein and Glutathion. Besonders bevorzugt sind gesättigte Fette, insbesondere Palmitin oder Myristin, Ballaststoffe, Folsäure, Omega-3-Fettsäuren, Cholesterin sowie Kohlenhdrate, insbesondere hochglykämische Kohlenhdrate wie bspw. Glucose. Für die Berechnung des Einflusses eines Lebensmittels auf den Energiestoffwechsel wird für Kohlenhydrate als epigenetisch aktiver Inhaltsstoff zweckmässig der Glykämische Index eines Lebensmittels eingesetzt. Der Glykämische Index gibt in Zahlen die blutzuckersteigernde Wirkung der Kohlenhydrate bzw. der in den Lebensmitteln enthaltenen Kohlenhydrate an. Die blutzuckersteigernde Wirkung von Traubenzucker dient als Referenzwert (100). Dabei wird von einer Testperson so viel Traubenzucker bzw. so viel des zu testenden Lebensmittels gegessen, dass jeweils 50 g Kohlenhydrate in der verzehrten Portion enthalten sind (s. auch Krumwide K H, 2007, "Kohlenhydrate richtig schätzen und einschätzen". MMW-Fortschritte der Medizin Originalien Nr. III, 149: 91-96).

Ballaststoffe sind weitgehend unverdauliche Nahrungsbestandteile, meist bestehend aus unverdaulichen Polysacchariden, die vorwiegend in pflanzlichen Lebensmitteln vorkommen. Sie kommen unter anderem in Getreide, Obst, Gemüse, Hülsenfrüchten und in geringen Mengen in Milch vor. Der Einfachheit wegen teilt man die Ballaststoffe in wasserlösliche (wie Johannisbrotkernmehl, Guar, Pektin und Dextrine) und wasserunlösliche (zum Beispiel Cellulose) ein. Unter Metabolite bzw. Zwischenmetabolite der epigenetisch aktiven Johannisbrotkernmehl, Guar, Pektin und Dextrine) und wasserunlösliche (zum Beispiel Cellulose) ein. Unter Metabolite bzw. Zwischenmetabolite der epigenetisch aktiven Inhaltsstoffen werden zeckmässig Acetyl-CoA, Malonyl-CoA, NAD / NADH ,ATP/ADP, und SAM/SAH verstanden.

Pflanzenstoffe enthalten miRNA's, die über eine RNA Interferenz die Genexpression epigenetisch beeinflussen und so gewünschte oder unerwünschte Genexpression hervorrufen können. Diese Wirkung kann in die in der Bewertung der Lebensmittel mit der vorliegenden Methode einfliessen.

## Kategorisierung eines Lebensmittels anhand der Bewertungsmethode

In einem ersten Aspekt stellt die vorliegende Erfindung eine Methode zur Bewertung eines Lebensmittels zur Verfügung umfassend

i) Bestimmung der Energiedichte des Lebensmittels;

ii) Auswahl von mindestens einem epigenetisch aktiven Inhaltsstoff des Lebensmittels, der den Energiestoffwechsel eines Säuger beeinflusst;

iii) Bestimmung der Konzentration des mindestens einen epigenetisch aktiven Inhaltsstoffes, der gemäss ii) ausgewählt wurde, im Lebensmittel;

iv) Berechnung des Einflusses des Lebensmittels auf den Energiestoffwechsel des Säugers durch mathematische Kombination der gemäss i) bestimmten Energiedichte des Lebensmittels und der gemäss iii) bestimmten Konzentration des mindestens einen epigenetisch aktiven Inhaltsstoffes im Lebensmittel;

v) Kategorisierung des Lebensmittels im Hinblick auf seinen Einfluss auf den Energiestoffwechsel des Säugers gemäss Berechung iv) wobei zwischen einem positiven, einem neutralen und einem negativen Einfluss des Lebensmittels auf den Energiestoffwechsel des Säugers unterschieden wird,

wobei die Berechnung iv) des Einflusses des Lebensmittels auf den Energiestoffwechsel des Säugers für die Lebensmittelklassen A)-D) gemäss folgender Formel I

$$\text{Epigenetischer Index (EPI)}_{A)-D)} = [v_{max,d} / k_d/(k_d+d) /100] * [s/(s+k_s) * (v_{max,s})],$$

wobei

$k_d$ = Inhibitionskonstante für enzymatische Reaktion mit d
$v_{max,d}$ = Reaktionsgeschwindigkeit für enzymatische Reaktion mit d
d = Energiedichte des Lebensmittels
$v_{max,s}$ = Reaktionsgeschwindigkeit für enzymatische Reaktion mit s
s = Konzentration gesättigte Fette im Lebensmittel
$k_s$ = Sättigungskonstante für enzymatische Reaktion mit s,

und für die Lebensmittelklassen E)-F) gemäss folgender Formel II

$$\text{Epigenetischer Index }_{E)-F)} = [v_{max,d} / k_d/(k_d+d) /100] * [(k_b^2+b)/b /v_{max,b}],$$

wobei

$k_d$ = Inhibitionskonstante für enzymatische Reaktion mit d
$v_{max,d}$ = Reaktionsgeschwindigkeit für enzymatische Reaktion mit d
d = Energiedichte des Lebensmittels
$v_{max,b}$ = Reaktionsgeschwindigkeit für enzymatische Reaktion mit b
b = Konzentration Ballaststoffe im Lebensmittel
$k_b$ = Sättigungskonstante für enzymatische Reaktion mit b,

vorgenommen wird, wobei zwischen folgenden Klassen von Lebensmitteln unterschieden wird: A) Fleischerzeugnisse, B) Beilagen und/oder Broterzeugnisse, C) Fischerzeugnisse, D) Milcherzeugnisse, E) Gemüse und F) Früchte..

Die Lebensmittel werden zur Beurteilung in Klassen eingeteilt und diese Klassen werden aufgrund der epigenetisch aktiven Inhaltstoffe oder deren Metaboliten, die bei der Aufnahme und Abbau der Stoffe im Körper eines Säugers (Mensch, Tier) entstehen und eine epigenetische Wirkung auf den Energiestoffwechsels eines Säugers entfalten können im Hinblick auf die Prävention oder Therapie chronischer Krankheiten bewertet. Ein Lebensmittel besteht aus einer Vielzahl bekannter oder unbekannter chemischer Verbindungen. Die Bewertungsmethode bzw. der epigenetische Index basiert auf epigenetisch aktiven Inhaltsstoffen und bewertet und verknüpft diese im Hinblick auf ihre epigenetische Wirkung. Die epigenetisch aktiven Inhaltsstoffe haben eine dosisabhängige Wirkung auf den Energiestoffwechsel insbesondere den Fett- und Zuckerstoffwechsel. Ohne sich auf eine Theorie festzulegen, wird vermutet, dass das verbindende epigenetische Element dabei die Beeinflussung der Transkription von Sirtuin-1 (SIRT 1) ist, da SIRT1 die Acetylierung des Peroxisome proliferator-activated receptor-gamma coactivator -1alpha (PGC1alpha) direkt beeinflusst und damit die Carnitin-Palmitoyltransferase (CPT) Gene zur Transkription direkt beeinflussen und eine dominante und verbindende Funktion übernimmt und so die Fettstoffwechsel zur Energieerzeugung steigert, und kaskadiert, als Folge des besser funktionierenden Fettstoffwechsel, auch den Zuckerstoffwechsel bezüglich Energiebereitstellung aus Zuckern, verbessert. Dabei ist zu bemerken, dass in den angewendeten Modellen zur Bewertung eines Lebensmittels und der dosisabhängigen Wirkung der epigenetisch aktiven Inhaltsstoffe eine klar führenden Rolle des Fettstoffwechsels zur Regulierung des Zuckerstoffwechsels postuliert wird. Dabei wird vermutet, dass ein enzymatischer Zusammenhang mit dem Epigenom primär via Methylierung und Histonacetylierung sowie auch via Palmitoylierung, sowohl auf die jeweiligen Transkriptionsfaktoren beteiligter Gene als auch auf diese direkt wirkend, existiert, und so über die Verbesserung der Aktivität des Genbereichs des Fettstoffwechsel und folgend des Fettstoffwechsels selbst, ein direkter Zugang zur Verbesserung des Glucosestoffwechsel besteht bzw. der Fettstoffwechsel den Glucosestoffwechsel dominiert, bzw. gesättigte Fette lipoglucotoxisch und nicht umgekehrt wie bisher betrachtet als glucolipotoxisch sind. Die Bewertungsmethode bzw. der epigenetische Index transformieren die epigenetisch aktiven Inhaltsstoffen quantitativ zur Wirkung am Epigenom und darauf basierend werden die Lebensmittel epigenetisch bewertet und kategorisiert.

Dabei werden die verschiedenen epigenetisch aktiven Inhaltstoffe für die Bewertung zweckmässig einem enzymatisch mechanistischen mathematischen Rechenverfahren unterworfen. Die so berechnete Auswirkung auf den Energiestoffwechsel eines Säuger im Hinblick auf die Prävention oder Therapie ernährungsorientierter chronischer Krankheiten und die Korrelation der Inhaltstoffe der Lebensmitteln erlaubt daraus für jedes Lebensmittel einer Lebensmittelklasse eine epigenetische Wertung der Wirkung auf den Energiestoffwechsel eines Säuger zu berechnen.

Für die Bewertungsmethode eines Lebensmittels werden je nach Lebensmittelkategorie unterschiedliche epigenetisch aktive Inhaltsstoffe der Lebensmittel herangezogen. Als quervergleichende Grösse wird immer die energetische Dichte eines Lebensmittels berücksichtigt.

Das Lebensmittel wird zweckmässig in die Klassen bestehend aus A) Fleischerzeugnisse, B) Beilagen und/oder Broterzeugnisse, C) Fischerzeugnisse, D) Milcherzeugnisse, E) Gemüse und F) Früchte unterteilt und je nach Lebensmittelkategorie wird der mindestens eine epigenetisch aktive Inhaltsstoff folgendermassen ausgewählt:

A) Fleischerzeugnisse: epigenetisch aktiver Inhaltsstoff ausgewählt aus der Gruppe bestehend aus gesättigte Fette und Cholesterin, bevorzugt gesättigte Fette;
B) Beilagen und/oder Broterzeugnisse: epigenetisch aktiver Inhaltsstoff ausgewählt aus der Gruppe bestehend aus gesättigte Fette und Kohlenhydrate, bevorzugt gesättigte Fette;
C) Fischerzeugnisse,: epigenetisch aktiver Inhaltsstoff ausgewählt aus der Gruppe bestehend aus gesättigte Fette und Omega-3-Fettsäuren, bevorzugt gesättigte Fette;
D) Milcherzeugnisse: epigenetisch aktiver Inhaltsstoff ausgewählt aus der Gruppe bestehend aus gesättigte Fette und Cholesterin, bevorzugt gesättigte Fette;
E) Gemüse: epigenetisch aktiver Inhaltsstoff ausgewählt aus der Gruppe bestehend aus Ballaststoffe und Folsäure, bevorzugt Ballaststoffe;
F) Früchte: epigenetisch aktiver Inhaltsstoff ausgewählt aus der Gruppe bestehend aus Ballaststoffe und Kohlenhydrate, bevorzugt Ballaststoffe.

Zur Berechnung des Einflusses des Lebensmittels auf den Energiestoffwechsel des Säugers wird zweckmässig die Energiedichte des Lebensmittels und die Konzentration des mindestens einen epigenetisch aktiven Inhaltsstoffes im Lebensmittel mathematisch kombiniert.

Zweckmässig wird die Berechung des Einflusses des Lebensmittels auf den Energiestoffwechsel des Säugers durch Multiplikation der Energiedichte des Lebensmittels und der Konzentration des mindestens einen epigenetisch aktiven Inhaltsstoffes im Lebensmittel vorgenommen. Dabei wird die Energiedichte vorzugsweise in 3 Bereiche eingeteilt (kleiner oder gleich 150, 150 - 250, grösser 250 kcal / 100 g) und vorzugsweise mit der Konzentration von gesättigten Fetten, insbesondere der Konzentration an Palmitinsäure C:16:0 jeweils (von 0-1, 1-5 und 5-10 g / 100 g) multipliziert. Zweckmässig wird das Produkt dieser Multiplikation linear in 3 Kategorien eingeteilt, wobei die tiefste Gruppe einen positiven, die mittlere Gruppe einen neutralen, die höchste Gruppe einen negativen Einfluss auf den Energiestoffwechsel eines Säugers hat.

Bevorzugt wird zur Berechnung des Einflusses des Lebensmittels auf den Energiestoffwechsel des Säugers gemäss der vorliegenden Methode abhängig von der Lebensmittelklasse eine mathematische Formel ausgewählt aus den unten aufgeführten Formeln I-VII eingesetzt. Der so erhaltene, sogenannte "Epigenetische Index" (EPI) kann somit als Grundlage für die Kategorisierung des Lebensmittels im Hinblick auf seinen Einfluss auf den Energiestoffwechsel des Säugers herangezogen werden und die Lebensmittel wie bspw. in Beispiel 1 aufgeführt mit dem so erhaltenen Wert für den Epigenetischen Index kategorisiert werden.

Zur Berechnung des Epigenetischen Index für die Lebensmittelklassen A)-D) bzw. E)-F) wird bevorzugt je nach Lebensmittelklasse die unten aufgeführteFormel I bzw. die Formel II eingesetzt, welche jeweils die Energiedichte und einen epigenetisch aktiven Inhaltsstoff berücksichtigen. Bei der Auswahl von Kohlenhydrate als epigenetisch aktiver Inhaltsstoff für die Berechnung des Epigenetischen Index wie bspw. in unten aufgeführten Formeln IV und VII berücksichtigt, wird zweckmässig der Glykämische Index eines Lebensmittels eingesetzt. Alternativ dazu kann auch die Konzentration von Kohlenhydraten im Lebensmittel, bevorzugt die Konzentration von Glucose im Lebensmittel eingesetzt werden.

Zur Berechnung des Epigenetischen Index für die Lebensmittelklassen A)-D) wird die folgende Formel I eingesetzt:

$$\text{Epigenetischer Index}_{A)-D)} = [v_{max,d} \,/\, k_d/(k_d+d) \,/100] * [s/(s+k_s) * (v_{max,s})],$$

wobei

$k_d$ = Inhibitionskonstante für enzymatische Reaktion mit d
$v_{max,d}$ = Reaktionsgeschwindigkeit für enzymatische Reaktion mit d
d = Energiedichte des Lebensmittels
$v_{max,s}$ = Reaktionsgeschwindigkeit für enzymatische Reaktion mit s
s = Konzentration gesättigte Fette im Lebensmittel
$k_s$ = Sättigungskonstante für enzymatische Reaktion mit s.

Zur Berechnung des Epigenetischen Index für die Lebensmittelklassen E)-F) wird die folgende Formel II eingesetzt:

$$\text{Epigenetischer Index}_{E)-F)} = [v_{max,d} \,/\, k_d/(k_d+d) \,/100] * [(k_b^2+b)/b \,/v_{max,b}],$$

wobei

$k_d$ = Inhibitionskonstante für enzymatische Reaktion mit d
$v_{max,d}$ = Reaktionsgeschwindigkeit für enzymatische Reaktion mit d
d = Energiedichte des Lebensmittels
$v_{max,b}$ = Reaktionsgeschwindigkeit für enzymatische Reaktion mit b
b = Konzentration Ballaststoffe im Lebensmittel
$k_b$ = Sättigungskonstante für enzymatische Reaktion mit b.

Besonders bevorzugt werden zur Berechnung des Einflusses des Lebensmittels auf den Energiestoffwechsel und damit zur Berechnung des Epigenetischen Index zusätzlich weitere epigenetisch aktive Inhaltsstoffe, vorzugsweise die Energiedichte und zwei epigenetisch aktive Inhaltsstoffe ausgewählt.

Insbesondere bevorzugt wird zur Berechnung des Epigenetischen Index für die Lebensmittelklassen A) und D) die folgende Formel III eingesetzt, wobei die Energiedichte und gesättigte Fettsäuren sowie Cholesterin in die Formel einfliessen:

$$\text{Epigenetischer Index}_{A) \text{ und } D)} = [v_{max,d} \,/\, k_d/(k_d+d) \,/100] * [s/(s+k_s) * (v_{max,s})] * [v_{max,c} \,/\, k_c/(c^2+k_c)],$$

wobei

$k_d$ = Inhibitionskonstante für enzymatische Reaktion mit d
$v_{max,d}$ = Reaktionsgeschwindigkeit für enzymatische Reaktion mit d
d = Energiedichte des Lebensmittels
$v_{max,s}$ = Reaktionsgeschwindigkeit für enzymatische Reaktion mit s
s = Konzentration gesättigte Fette im Lebensmittel
$k_s$ = Sättigungskonstante für enzymatische Reaktion mit s
$v_{max,c}$ = Reaktionsgeschwindigkeit für enzymatische Reaktion mit c
c = Konzentration Cholesterin im Lebensmittel
$k_c$ = Reaktionskonstante für enzymatische Reaktion mit c.

Insbesondere bevorzugt wird zur Berechnung des Epigenetischen Index für die Lebensmittelklasse B) die folgende Formel IV eingesetzt, wobei die Energiedichte und gesättigte Fettsäuren sowie Kohlehydrate angegeben als Glykämischer Index in die Formel einfliessen:

$$\text{Epigenetischer Index}_{B)} = [v_{max,d} / k_d/(k_d+d) /100] * [s/(s+k_s) * (v_{max,s})] * [v_{max,g} /k_g/(g^3 +k_g)],$$

wobei

$k_d$ = Inhibitionskonstante für enzymatische Reaktion mit d
$v_{max,d}$ = Reaktionsgeschwindigkeit für enzymatische Reaktion mit d
d = Energiedichte des Lebensmittels
$v_{max,s}$ = Reaktionsgeschwindigkeit für enzymatische Reaktion mit s
s = Konzentration gesättigte Fette im Lebensmittel
$k_s$= Sättigungskonstante für enzymatische Reaktion mit s
$v_{max,g}$ = Reaktionsgeschwindigkeit für enzymatische Reaktion mit g
g = Konzentration Kohlenhydrate im Lebensmittel angegeben als Glykämischer Index des Lebensmittels
$k_g$= Reaktionskonstante für enzymatische Reaktion mit g.

Insbesondere bevorzugt wird zur Berechnung des Epigenetischen Index für die Lebensmittelklasse C) die folgende Formel V eingesetzt, wobei die Energiedichte und gesättigte Fettsäuren sowie Omega-3-Fettsäuren in die Formel einfliessen:

$$\text{Epigenetischer Index}_{C)} = [v_{max,d} / k_d/(k_d+d) /100] * [s/(s+k_s) * (v_{max,s})] * [v_{max,o} *(o+k_o^2)/o],$$

wobei

$k_d$ = Inhibitionskonstante für enzymatische Reaktion mit d
$v_{max,d}$ = Reaktionsgeschwindigkeit für enzymatische Reaktion mit d
d = Energiedichte des Lebensmittels
$v_{max,s}$ = Reaktionsgeschwindigkeit für enzymatische Reaktion s
s = Konzentration gesättigte Fette im Lebensmittel
$k_s$= Sättigungskonstante für enzymatische Reaktion mit s
$v_{max,o}$ = Reaktionsgeschwindigkeit für enzymatische Reaktion mit o
o = Konzentration Omega-3-Fettsäuren im Lebensmittel
$k_o$= Reaktionskonstante für enzymatische Reaktion mit o.

Insbesondere bevorzugt wird zur Berechnung des Epigenetischen Index für die Lebensmittelklasse E) die folgende Formel VI eingesetzt, wobei die Energiedichte und Ballaststoffe sowie Folsäure in die Formel einfliessen:

$$\text{Epigenetischer Index}_{E)} = = [v_{max,d} / k_d/(k_d+d) /100] * [(k_b^2+b)/b/v_{max,b}] * [ (k_f^2+f)/f /v_{max,f}],$$

wobei

$k_d$ = Inhibitionskonstante für enzymatische Reaktion mit d
$v_{max,d}$ = Reaktionsgeschwindigkeit für Reaktion d
d = Energiedichte des Lebensmittels
$v_{max,b}$ = Reaktionsgeschwindigkeit für enzymatische Reaktion mit b
b = Konzentration Ballaststoffe im Lebensmittel
$k_b$ = Reaktionskonstante für enzymatische Reaktion mit b
$v_{max,f}$= Reaktionsgeschwindigkeit für enzymatische Reaktion mit f
f = Konzentration Folsäure im Lebensmittel
$k_f$= Reaktionskonstante für enzymatische Reaktion mit f.

Insbesondere bevorzugt wird zur Berechnung des Epigenetischen Index für die Lebensmittelklassen F) die folgende Formel VII eingesetzt, wobei die Energiedichte und Ballaststoffe sowie Kohlehydrate angegeben als Glykämischer Index in die Formel einfliessen:

$$\text{Epigenetischer Index}_{F)} = [v_{max,d} / k_d/(k_d+d) /100] * [(k_b^2+b)/b/v_{max,b}] * [v_{max,g} /k_g/(g^3 +k_g)],$$

wobei

$k_d$ = Inhibitionskonstante für enzymatische Reaktion mit d
$v_{max,d}$ = Reaktionsgeschwindigkeit für Reaktion d
d = Energiedichte des Lebensmittels
$v_{max,b}$ = Reaktionsgeschwindigkeit für enzymatische Reaktion mit b
b = Konzentration Ballaststoffe im Lebensmittel
$k_b$= Reaktionskonstante für enzymatische Reaktion mit b
$v_{max,g}$ = Reaktionsgeschwindigkeit für enzymatische Reaktion mit g
g = Konzentration Kohlenhydrate im Lebensmittel angegeben als Glykämischer Index des Lebensmittels
kg= Reaktionskonstante für enzymatische Reaktion mit g.

Die für die in den Formeln I-VII enthaltenen Parameter $k_d$, $v_{max,d}$; $k_s$, $v_{max,s}$; $k_b$, $v_{max,b}$; $k_c$, $v_{max,c}$; $k_g$, $v_{max,g}$; $k_o$, $v_{max,o}$; $k_f$, $v_{max,f}$ zu verwenden Zahlenwerte werden zweckmässig der untenstehenden Tabelle 1 entnommen.

Tabelle 1: Zahlenwerte der Parameter $k_d$, $v_{max,d}$; $k_s$, $v_{max,s}$; $k_b$, $v_{max,b}$; $k_c$, $v_{max,c}$; $k_g$, $v_{max,g}$; $k_o$, $v_{max,o}$; $k_f$, $v_{max,f}$

| Parameter | Klasse | Energiedichte (d) | gesättigte Fette (s) | Ballaststoff (b) |
|---|---|---|---|---|
| | | in kcal / 100 g | in g | in g |
| Term | - | $=v_{max,d}$ / $k_d/(k_d+d)$ /100 | $=s/(s+k_s)*(v_{max,s})$ | $= k_b^2+b)/b/v_{max,b}$ |
| $v_{max}$ | - | 1 | 3 | 1 |
| Parametrierung | - | $k_d$ | $k_s$ | $k_b$ |
| Fleisch | A | 1.05 | 1.1 | |
| Brot | B | 1.05 | 1.1 | |
| Beilagen | B | 1.05 | 1.1 | |
| Fisch | C | 1.05 | 1.1 | |
| Milcherzeugnisse | D | 1.05 | 1.1 | |
| Gemüse | E | 1.05 | | 0.55 |
| Früchte | F | 1.05 | | 0.55 |

| Parameter | Klasse | Cholesterin (c) | Glykämie (g) | Omega-3-FS (o) | Folsäure (f) |
|---|---|---|---|---|---|
| | | in mg | in (-) | in g | in µg |
| Term | - | $=v_{max,c}$ / $k_c/(c^2+k_c)$ | $=v_{max,g}$ $/k_g/(g^3+k_g)$ | $=v_{max,o}*(o+k_o^2)/o$ | $= (k_f^2+f)/f$ $/v_{max,f}$ |
| $v_{max}$ | - | 1 | 1 | 1 | 1 |
| Parametrierung | - | $k_c$ | $k_g$ | $k_o$ | $k_f$ |
| Fleisch | A | 9000 | | | |
| Brot | B | | 300000 | | |
| Beilagen | B | | 300000 | | |
| Fisch | C | | | 1.3 | |
| Milcherzeugnisse | D | 9000 | | | |
| Gemüse | E | | | | 1.6 |
| Früchte | F | | 300000 | | |

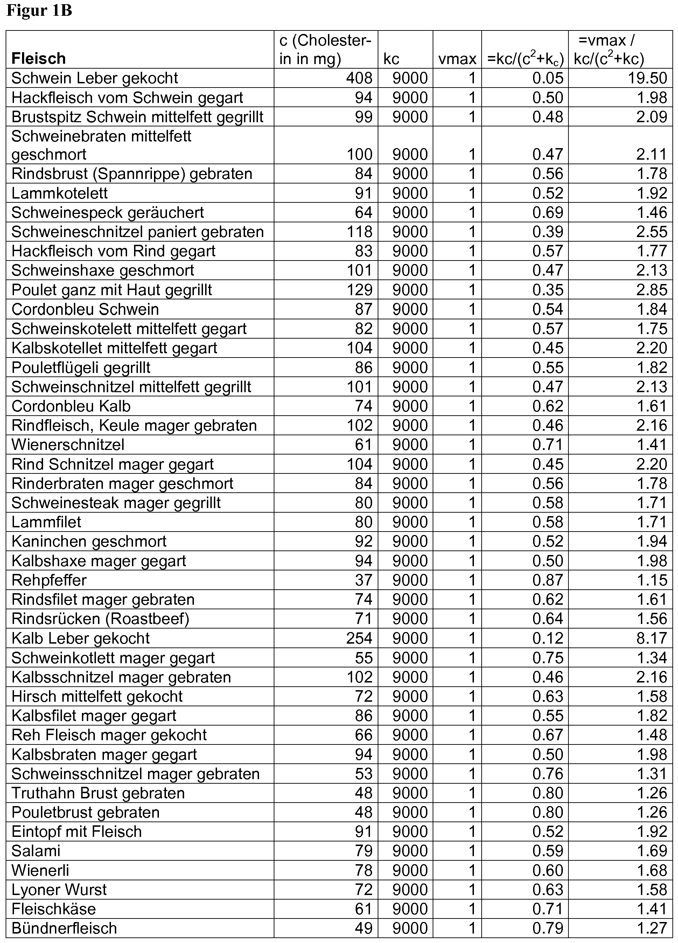

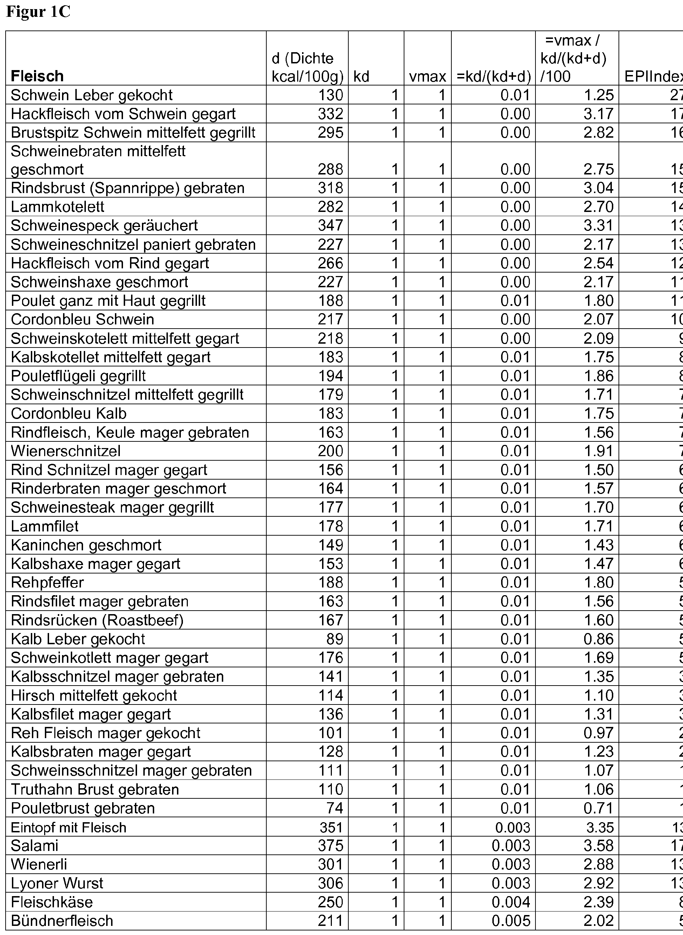

Die Energiedichte d und die Konzentration an epigenetisch aktiven Inhaltsstoffen wie bspw. gesättigte Fette, Ballaststoffe, Cholesterin, Kohlenhydrate, Omega-3-Fettsäuren, bzw. Folsäure eines Lebensmittels kann durch entsprechende in der Literatur bekannte Methoden bestimmt bzw. der Literatur wie beispielsweise dem Standardwerk "Das Kalorien -Nährwert Lexikon" Sven David Müller, Katrin Raschke, 2. Auflage, Schlütersche Verlag, 2004 oder den Tabellen der Figuren 1 bis 7 entnommen werden.

Alle Lebensmittelklassen werden analog behandelt, so dass Quervergleiche von Lebensmitteln im Hinblick auf den Einfluss auf den Energiestoffwechsel des Säugers möglich sind.

## Kategorisierung eines Lebensmittels durch den Epigenetischen Index

Bei der Verwendung der Zahlenwerte aus Tabelle 1 für $k_d$, $v_{max,d}$; $k_s$, $v_{max,s}$; $k_b$, $v_{max,b}$; $k_c$, $v_{max,c}$; $k_g$, $v_{max,g}$; $k_o$, $v_{max,o}$; $k_f$, $v_{max,f}$ zur Berechnung des Epigenetischen Index gemäss einer der Formeln I oder II (Verwendung der Energiedichte und einem epigenetisch aktiven Inhaltsstoff) werden Werte für den Epigenetischen Index eines Lebensmittels zwischen 0.01 und 20 erhalten.

Die einzelnen berechneten Werte sind jeweils auf ganze Zahlen gerundet und zur praktischen Umsetzung auf einen Wertebereich von minmal 1 bis maximal 9 beschränkt (für berechnete Werte < 1 wird jeweils 1 eingesetzt; für berechnete Werte > 9 wird 9 eingesetzt). Drei Klassen werden mit folgender Bewertung definiert:

EPI Index 1-3:     positiver Effekt
EPI Index 3-6:     neutraler Effekt
EPI Index 6-9:     negativer Effekt

Bei der Verwendung der Zahlenwerte aus Tabelle 1 für $k_d$, $v_{max,d}$; $k_s$, $v_{max,s}$; $k_b$, $v_{max,h}$; $k_c$, $v_{max,c}$; $k_g$, $v_{max,g}$; $k_o$, $v_{max,o}$; $k_f$, $v_{max,f}$ zur Berechnung des Epigenetischen Index gemäss einer der Formeln III-VII (Verwendung der Energiedichte und zwei epigenetisch aktiver Inhaltsstoffen) werden Werte für den Epigenetischer Index eines Lebensmittels zwischen 0.01 und 40 erhalten.

Die einzelnen berechneten Werte sind jeweils auf ganze Zahlen gerundet und zur praktischen Umsetzung auf einen Wertebereich von minmal 1 bis maximal 27 beschränkt (für berechnete Werte < 1 wird jeweils 1 eingesetzt; für berechnete Werte > 27 wird 27 eingesetzt). Drei Klassen werden mit folgender Bewertung definiert:

EPI Index 1-3:     positiver Effekt
EPI Index 4-9:     neutraler Effekt
EPI Index 10-27:   negativer Effekt

Gerundete Werte werden dabei für die praktische Umsetzung eingesetzt. Der Epigenetische Index charakterisiert aufgrund der Modellrechnung jedes einzelne Lebensmittel spezifisch und gibt ihm einen berechneten Wert, welcher eine Hierarchiebildung im Hinblick auf die Wirkung des Lebensmittles auf den Energiestoffwechsel ermöglicht. Der Indexbereich von 1-3 gilt als ein vorteilhafter Bereich im Hinblick auf die günstige Wirkung auf den Energiestoffwechsel (dabei insbesondere den Fett- und Zuckerstoffwechsel) und favorisiert Lebensmittel mit diesem Wertbereich zur täglichen Aufnahme. Aus diesem Grund ist das Ziel, möglichst Lebensmittel mit einem möglichst tiefen Epigenetischen Index auszuwählen und aufzunehmen. Der Klassierungsbereich 4 - 9 favorisiert eine mässige Aufnahme der klassierten Lebensmittel da der Energiestoffwechsel positiv wie negativ beeinflusst werden kann und somit ein neutraler Effekt besteht. Der Bereich 10 - 27 bedeutet einen negativen Einfluss der Lebensmittelinhaltstoffe auf den Energiestoffwechsel und aus diesem Grund soll eine Minimierung der Aufnahme der in diesem Bereich klassierten Lebensmittel anzustreben zu sein. Durch eine unterschiedliche Bewertung der Inhaltstoffe lässt sich das vorliegende Bewertungsmodell unter Verwendung des Epigenetischen Index zur Prävention und Therapie unterschiedlicher chronischer Krankheiten breit und universell einsetzen. Ein Berechnungsbeispiel des epigenetischen Index basierend auf der besonders bevorzugten Berechnungsformel III für Lebensmittel tierischer Herkunft (Fleischerzeugnisse) wird am Beispiel Lammfleisch in Beispiel 1 illustriert.

## Epigenetische Last

Um die dosisabhängige (mengenabhängige) Wirkung der epigenetisch aktiven Inhaltstoffe der eingenommen Lebensmittel auf den Energiestoffwechsel zu bestimmen wird die sogenannte "Epigenetische Last" definiert. Das nach dem Epigenetischen Index ausgewählte Lebensmittel wird mit der Menge an gesättigten Fetten des Lebensmittels multipliziert. Dabei wird der erhaltene Wert für den Epigenetischen Index (EPI) mit der Menge an gesättigten Fetten des Lebensmittels bezogen auf die Menge des Lebensmittels multipliziert. Die Menge an gesättigten Fetten des Lebensmittels wird zweckmässig auf 100 g eines Lebemsmittels bezogen (Konzentration an gesättigten Fetten) und mit der aufgenommenen Menge an Lebensmittel multipliziert. Bevorzugt wird die Epigenetische Last gemäss folgender Formel VIII berechnet:

$$\text{Epigenetische Last} = (\text{Epigenetischer Index eines Lebensmittels n}) * (\text{Konzentration an}$$
$$\text{gesättigten Fetten des Lebensmittels n in g / 100 g Lebensmittel n}) * \text{Menge des}$$
$$\text{aufgenommenen Lebensmittels n in g / 100 g;}$$

wobei die Konzentration an gesättigten Fetten des Lebensmittels zweckmässig die Konzentration an gesättigten Fetten in 100 g essbarem Anteil eines Lebensmittels ist.

Für Lebensmittel der Lebensmittelklassen E) (Gemüse) und F) (Früchte) ist die Epigenetische Last unabhängig von der Menge der Lebensmittel null. Beispiel 3 beschreibt beispielhaft die Berechnung der Epigenetischen Last anhand der Formel VIII für zwei verschiedene Lebensmittel. Beispiel 3 zeigt den Vergleich zweier Lebensmittel mit unterschiedlichem epigenetischen Index innerhalb einer Lebensmittelkategorie auf. Die Berechnung des epigenetischen Index und der epigenetischen Last ermöglicht die mengenmässige Auswahl eines Lebensmittels im Hinblick auf die dosisabhängige Wirkung auf den Energiestoffwechsel im Hinblick auf ein zu therapierendes Krankheitsbild mit der Bewertungsmethode und deren Einsatz zur qualitativen und quantitativen Auswahl von Lebensmittel für die Ernährung des Menschen.

**Verwendung der Methode zur Bewertung eines Lebensmittels für die Prävention und/oder Therapie von chronischen Krankheiten**

Zur Verwendung der vorliegenden Bewertungsmethode für die Prävention und/oder Therapie von chronischen Krankheiten wird bevorzugt die Epigenetische Last pro Tag (Epigentische Last/d), d.h. die Epigenetische Last eines pro Tag aufgenommenen Lebensmittels oder die kumulierte, d.h. addierte Epigenetische Last einer oder mehrerer aus verschiedenen Lebensmitteln zusammengesetzten pro Tag aufgenommener Mahlzeiten, eingesetzt. Hierbei kann der positive, neutrale bzw. negative Einfluss des Lebensmittels auf den Energiestoffwechsel des Säugers in Abhängigkeit der Epigentischen Last/d folgendermasssên definiert werden.

Epigentische Last/d von 0 bis 144:      positiver Effekt
Epigentische Last/d von 145 bis 192:    neutraler Effekt
Epigentische Last/d von 193 bis 864:    negativer Effekt

Die Methode zur Bewertung eines Lebensmittels bzw. die daraus resultierende Bestimmung des Epigenetischen Index bzw. der Epigentische Last wie oben beschrieben, können, optional in Kombination mit einer Diät, zur Verwendung für die Prävention und/oder Therapie von chronischen Krankheiten eingesetzt werden. Bevorzugte chronischen Krankheiten sind Krankheiten ausgewählt aus der Gruppe Insulinresistenz, Diabetes Typ 2, Krebserkrankungen wie Darmkrebs, Pankreaskrebs, Brustkrebs, Gefässkrankheiten, Demenz, Alzheimer, Diabetes Typ 1, Gestationsdiabetes, Divertikulitis, Reizdarm, Morbus crohn, Rheumatoide Arthritis, Depression, Burnout, Autismus, Übergewicht, nicht-alkoholische Fettleber, Steatohepatitis, Pankreatitis und Osteoporose. Noch bevorzugtere Krankheiten sind Krankheiten ausgewählt aus der Gruppe Insulinresistenz, Alzheimer, Diabetes Typ 2, Diabetes Typ 1, Gestationsdiabetes, Übergewicht, nicht-alkoholische Fettleber und Steatohepatitis. Besonders bevorzugt sind Insulinresistenz, Diabetes Typ 2 Übergewicht, nicht-alkoholische Fettleber und Steatohepatitis, insbesondere Insulinresistenz, Diabetes Typ 2 und Übergewicht. Am meisten bevorzugt sind Insulinresistenz und/oder Diabetes Typ 2.

Die Bewertungsmethode der vorliegenden Erfindung bzw. der epigenetische Index und die epigenetische Last ermöglichen eine Quantifizierung der Wirkung eines Lebensmittels oder Inhaltsstoffes und dessen epigenetischer Wirkung auf den Energiestoffwechsel eines Säugers zur präventiven und/oder therapeutischen Anwendung bei chronischen Krankheiten insbesondere bei Übergewicht, Insulinresistenz und/oder Diabetes Typ 2. Epigenetisch aktive Inhaltsstoffe des Lebensmittels werden dabei spezifisch zur Berechnung des epigenetischen Index bzw. der epigenetischen Last in Abhängigkeit der zu therapierenden oder präventiv zu verhindernden chronischen Krankheit ausgewählt und eingesetzt.

Abhängig von der spezifischen chronischen Krankheit werden aus den oben genannten epigenetisch aktiven Inhaltsstoffen eines Lebensmittels bevorzugt die folgenden epigenetisch aktiven Inhaltsstoffen für die Bewertungsmethode ausgewählt:

Bei Übergewicht, Insulinresistenz bzw. Diabetes Typ 2 sind dies vorzugsweise gesättigte Fette aufgrund ihrer lipotoxischen Wirkung. Bei Insulinresistenz ist der epigenetisch aktive Inhaltsstoff gesättigte Fette aufgrund ihrer Wirkung auf die wichtigsten Transkriptionsfaktoren bzw. des postulierten Transfers von Methylgruppen dominant im Hinblick auf die Aktivität des Fett- und Zuckerstoffwechsels insbesondere des CPT1 Gens zur Bereitstellung von Energie (ATP). Die epigenetische Last ersetzt die bisher genutzte glykämische Last bei der Therapie von z. B. Diabetes Typ 2, da überraschenderweise in Zusammenhang mit der vorliegenden Erfindung festgestellt wurde, dass die prinzipiellen Auslöseinhaltstoffe für Insulinresistenz und Diabetes Typ 2 nicht alleine Zucker sind, sondern dominant gesättigte Fette insbesondere C16:0.

In der Therapie von Diabetes Typ 2 Patienten wird zur Kurierung des Diabetes Typ 2 eine Ernährungsstrategie mit minimalster Einnahme gesättigter Fette und vorteilhafter epigenetischer Last verfolgt (siehe Beispiel 6), was zur Kurierung des Diabetes Typ 2 in nur 9 Wochen führen kann und bei der eine Langzeitstabilität im Hinblick auf Blutzuckerwerte von vormals Patienten mit Diabetes Typ 2 erzielt werden kann. Die ersten 9 Wochen der Therapie wie auch die Langzeitbehandlung wurde mit der Einnahme einer L-Carnitin enthaltenden Zusammensetzung begleitend zur Ernährungsstrategie unterstützt wie auch das Einnehmen von 3 Hauptmahlzeiten ohne Zwischenmahlzeiten angewendet. Damit steht mit dem epigenetischen Index eine auf den Diabetes Typ 2 ausgerichtete Auswahl von Lebensmitteln und zusammen mit der epigenetischen Last eine neue Ernährungsstrategie zur Verfügung für eine einfache und wissenschaftliche medizinische Patientenführung zur Behandlung von Diabetes Typ 2, einerseits zur Kurierung bei der Anwendung im nicht-insulinabhängigen Diabetes Typ 2 und zur begleitenden Behandlung im insulinabhängigen Diabetes Typ 2 postuliert in der Zukunft auch zu dessen Kurierung. Hiermit eröffnet sich eine neue Ernährungstrategie bei Diabetes Typ 2 zur Therapieunterstützung, welche einen reduzierten Einsatz von Insulin ermöglicht, da die Glucosewerte bei bei der Einnahme von Lebensmitteln mit tiefem epigenetischen Index und tiefer epigenetischer Last einen besseren Glucosestoffwechsel ermöglichen. Zur Prävention von Übergewicht und Insulinresistenz ist die epigenetische Last zwischen den Mahlzeiten vorteilhaft Null.

Für die Prävention und/oder Therapie von chronischen Krankheiten wählt der Konsument vorzugsweise Lebensmittel mit einem positiven Einfluss auf den Energiestoffwechsel, besonders bevorzugt Lebensmittel mit einem tiefen Epigenetischem Index insbesondere Lebensmittel mit einem tiefen Epigenetischem Index im Bereich von 1-3 für eine einzelne oder eine zusammengesetzte Mahlzeit aus. Über die ganze Therapiezeit liegt die epigenetische Last vorteilhaft pro Tag (Epigentische Last/d) zwischen 0 bis 144 und ist zwischen den Mahlzeiten vorteilhaft Null, zweckmässig mit einer Pausenzeit der Essensaufnahme zwischen den Hauptmahlzeiten von 3 bis 6 h.

Für die Prävention und/oder Therapie von chronischen Krankheiten im Rahmen der vorliegenden Beschreibung wird bevorzugt die kumulierte Einnahme von Lebensmitteln über einen Tag vorzugsweise mit Hilfe der Menge an gesättigten Fetten des Lebensmittels kontrolliert, indem die maximale Einnahme pro Tag von gesättigten Fetten wie folgt vorgegeben wird:
Zweckmässig sollte für die Prävention und/oder Therapie von

i) Übergewicht die tägliche Einnahme von gesättigten Fetten durch gesättigte Fette enthaltende Lebensmittels die Menge von 40 g / d gesättigte Fette, bevorzugt die Menge von 32 g / d, besonders bevorzugt die Menge von 13 g / d nicht übersteigen;
ii) Insulinresistenz die tägliche Einnahme von gesättigten Fetten durch gesättigte Fette enthaltende Lebensmittels die Menge von 30 g / d gesättigte Fette bevorzugt die Menge von 23 g / d , besonders bevorzugt die Menge von 9 g /d nicht übersteigen;
iii) Alzheimer, Diabetes Typ 2, Diabetes Typ 1 oder Gestationsdiabetes die tägliche Einnahme von gesättigten Fetten durch gesättigte Fette enthaltende Lebensmittels die Menge von 20 g/ d gesättigte Fette bevorzugt die Menge von 6.5 g/ d besonders bevorzugt die Menge von 4.8 g/ d nicht übersteigen.

Bevorzugt sollte für die Prävention und/oder Therapie von

i) Übergewicht die tägliche Einnahme von gesättigten Fetten durch gesättigte Fette enthaltende Lebensmittels die Menge von 18 g / d gesättigte Fette, bevorzugt die Menge von 12 g / d, besonders bevorzugt die Menge von 9 g / d nicht übersteigen;
ii) Insulinresistenz die tägliche Einnahme von gesättigten Fetten durch gesättigte Fette enthaltende Lebensmittels die Menge von 16 g / d gesättigte Fette bevorzugt die Menge von 12 g / d, besonders bevorzugt die Menge von 9 g /d nicht übersteigen;
iii) Alzheimer, Diabetes Typ 2, Diabetes Typ 1 oder Gestationsdiabetes die tägliche Einnahme von gesättigten Fetten durch gesättigte Fette enthaltende Lebensmittels die Menge von 15 g/ d gesättigte Fette bevorzugt die Menge von 6.5 g/ d besonders bevorzugt die Menge von 4.8 g/ d nicht übersteigen.
ii) Insulinresistenz die tägliche Einnahme von gesättigten Fetten durch gesättigte Fette enthaltende Lebensmittels die Menge von 16 g / d gesättigte Fette bevorzugt die Menge von 12 g / d, besonders bevorzugt die Menge von 9 g /d nicht übersteigen;
iii) Alzheimer, Diabetes Typ 2, Diabetes Typ 1 oder Gestationsdiabetes die tägliche Einnahme von gesättigten Fetten durch gesättigte Fette enthaltende Lebensmittels die Menge von 15 g/ d gesättigte Fette bevorzugt die Menge von 6.5 g/ d besonders bevorzugt die Menge von 4.8 g/ d nicht übersteigen.

Für die Prävention und/oder Therapie von chronischen Krankheiten im Rahmen der vorliegenden Beschreibung wird besonders bevorzugt die kumulierte Einnahme von Lebensmitteln über einen Tag vorzugsweise mit Hilfe der epigenetischen Last der Lebensmittel kontrolliert, indem die maximale Einnahme pro Tag der epigenetischen Last wie unten aufgeführt vorgegeben wird und zweckmässig die gemäss Formel VIII bestimmte epigenetische Last für jede Lebensmittelklasse bzw. für die in den eingenommenen Mahlzeiten enthaltenen Lebensmittelklassen berechnet und entsprechend wie bspw. in Beispiel 5 aufgeführt, addiert wird. Zweckmässig sollte für die Prävention und/oder Therapie von

i) Übergewicht die epigenetische Last von täglich eingenommenen Lebensmitteln 240 / d , bevorzugt 192 / d, besonders bevorzugt 77 / d nicht übersteigen;

ii) Insulinresistenz die epigenetische Last von täglich eingenommenen Lebensmitteln 135 / d bevorzugt 104 / d , besonders bevorzugt 41 /d nicht übersteigen;

iii) Alzheimer, Diabetes Typ 2, Diabetes Typ 1 oder Gestationsdiabetes die tägliche Einnahme von Lebensmittels mit einer epigenetischen Last von 108 / d bevorzugt die epigenetische Last von 86 / d besonders bevorzugt die epigenetische Last von 35 / d nicht übersteigen.

Als L-Carnitin sowie ein physiologisch verträgliches Derivat und/oder Salz davon werden zweckmässig L-Carnitin Base ((3R)-Hydroxy-4-(trimethylammonium)-butyrat), L-Carnitin-L-Tartrat, Acetyl-L-Carnitin, Propionyl-L-Carnitin, Valeroyl-L-Carnitin, Isovaleroyl-L-Carnitin, L-Carnitin-Magnesium-Citrat, L-Carnitin-Fumarat oder weitere bekannte Derivate und/oder Salze von L-Carnitin verwendet. Bevorzugt sind L-Carnitin-L-Tartrat, Acetyl-L-Carnitin und/oder L-Carnitin Base. Besonders bevorzugt ist L-Carnitin-L-Tartrat. Es können auch Mischungen der genannten L-Carnitine eingesetzt werden, bevorzugt wird jedoch jeweils nur ein L-Carnitin eingesetzt. Zweckmässig enthält die Zusammensetzung L-Carnitin, ein physiologisch akzeptables Derivat und/oder Salz davon in einer Menge von 100-10000 mg, bevorzugt in einer Menge von 200-5000 mg, besonders bevorzugt in einer Menge von 300-2000 mg, insbesondere in einer Menge von 500-1000 mg, bezogen auf die Menge an L-Carnitin Base. Zweckmässig werden während der Prävention oder Therapie täglich 500-1000 mg, bevorzugt 1000 mg L-Carnitin, bezogen auf die Menge an L-Carnitin Base eingenommen. Bevorzugt enthält die Zusammensetzung umfassend L-Carnitin die in Tabelle 2 aufgelisteten Inhaltsstoffe, bevorzugt die Summe der in Tabelle 2 aufgelisteten Inhaltstoffe, die zweckmässig während der Prävention oder Therapie auf den Tag verteilt morgens ("Morgenbeutel") und mittags ("Mittagsbeutel") eingenommen werden

Tabelle 2: Zusammensetzung umfassend L-Carnitin (Epigenosan ®)

| Morgen Beutel | Morgen Beutel | Mittagsbeutel | Summe | Angaben in |
|---|---|---|---|---|
| Oligo Fructose | 1250 | 1000 | 2250 | mg |
| L-Carnitin | 500 | 500 | 1000 | mg |
| Magnesium | 100 | | 100 | mg |
| Grüntee Extrakt | 75 | | 75 | mg |
| Folsäure | 30 | 30 | 60 | μg |
| Vitamin B12 | 0.31 | 0.31 | 0.62 | μg |
| Mate Tee Extrakt | | 600 | 600 | mg |
| L-Arginin | | 150 | 150 | mg |
| Soja Extrakt | | 12.5 | 12.5 | mg |
| davon Isoflavone | | 27.5 | 27.5 | mg |
| DHA (Omega-3) | | 1.5 | 1.5 | μg |
| Vitamin D | | 1.8 | 1.8 | mg |
| Vitamin E | | 2.4 | 2.4 | mg |
| Niacin | | 0.21 | 0.21 | mg |
| Vitamin B6 | | 12.5 | 12.5 | μg |
| Biotin | | 1.8 | 1.8 | mg |
| Panthothensäure | | 1.8 | 1.8 | mg |
| Einnahme | morgens | mittags | | |

Bei der hierin erwähnten Diät, wird vorzugsweise kein kalorienhaltiges Nahrungsmittel zwischen den Hauptmahlzeiten zu sich genommen. Bevorzugt wird zwischen den Hauptmahlzeiten überhaupt keine kalorienenthaltende Nahrung aufgenommen. Nahrung, die den Glukosegehalt im Blut erhöht, ist üblicherweise Glukose beziehungsweise allgemein Zucker enthaltende Nahrung. Besonders bevorzugt werden zwischen den Hauptmahlzeiten nur Getränke aufgenommen, die den Glukosegehalt im Blut nicht erhöhen wie Wasser, Mineralwasser oder Tee ohne Zucker, wobei diese Getränke bevorzugt keine Kalorien enthalten. Insbesonders wird zwischen den Hauptmahlzeiten Wasser oder Tee ohne Zucker, aufgenommen. Die üblicherweise 3 Hauptmahlzeiten während eines Tages (24 Stunden) werden zweckmäßig morgens zwischen 6 und 9 Uhr, mittags zwischen 11 und 15 Uhr und abends zwischen 17 und 20 Uhr eingenommen, wobei bevorzugt zwischen drei und sechs Stunden, besonders bevorzugt zwischen vier und sechs Stunden zwischen den Hauptmahlzeiten keine Nahrung aufgenommen wird, die den Glukosegehalt im Blut erhöht, wobei bevorzugt keine Kalorien enthaltende Nahrung während dieser Zeit aufgenommen wird.

Zweckmäßig ist die Energiebilanz der Diät ausgeglichen oder negativ. Eine ausgeglichene Energiebilanz wird erreicht, wenn der Energiegehalt der Nahrung der Anzahl an Kalorien entspricht, die erforderlich ist, um das bestehende Körpergewicht aufrechtzuerhalten. Eine negative Energiebilanz wird erreicht, wenn der Energiegehalt der Nahrung geringer ist als die Anzahl an Kalorien, die erforderlich ist, um das bestehende Körpergewicht aufrechtzuerhalten. Die Anzahl an Kalorien, die erforderlich ist, um das bestehende Körpergewicht aufrechtzuerhalten ist abhängig von Geschlecht, Alter, Körpergewicht, dem Grundumsatz, sowie dem Arbeitsumsatz entsprechend der körperlichen Aktivität eines Säugers. Bevorzugt ist die Energiebilanz des erfindungsgemässen Ernährungs-regimes negativ.

Üblicherweise setzt sich die Anzahl an Kalorien, die erforderlich ist, um das bestehende Körpergewicht aufrechtzuerhalten, bei einem Menschen zusammen aus dem Ruheumsatz (1 kcal pro Kilogramm Körpergewicht und Stunde) und sowie dem Arbeitsumsatz, wobei sich dieser errechnet aus dem Grundumsatz multipliziert mit den entsprechenden Faktoren für die jeweilige körperliche Aktivität (z.B. 1.5 bei leichter körperlicher Aktivität). Der Energiegehalt der Nahrung bei einer negativen Energiebilanz liegt bevorzugt bei weniger als 100 bis 80 %, besonders bevorzugt bei weniger als 80 bis 60 %, insbesondere bei weniger als 60 bis 40 % der Anzahl an Kalorien, die erforderlich ist, um das bestehende Körpergewicht aufrecht zu erhalten. Zweckmäßig wird der Grundumsatz oder Ruheumsatz gemessen, um individuelle Schwankungen zu eliminieren, wobei der Ruheumsatz auch berechnet werden kann.

Besonders bevorzugt übersteigt die pro Tag aufgenommene Kalorienmenge in Zusammenhang mit der Diät die Kalorienmenge von 3000 kcal / d nicht.

## Beispiele

### Beispiel 1: Berechnungsbeispiel epigenetischer Index am Beispiel Lammfleisch

Unter Verwendung von Formel III

(Epigenetischer Index $_{A) und D)}$=[$v_{max,d}/k_d/(k_d+d)/100$]*[$s/(s+k_s)*(v_{max,s})$]*[$v_{max,c}/k_c/(k_c^2+c)$]) und den Zahlenwerten gemäss Tabelle 1 lassen sich die EPIIndices unterschiedlicher Fleischstücke am Tier wie in Tabelle 3 aufgeführt berechnen und bewerten.

Tabelle 3: EPIIndices unterschiedlicher Fleischstücke am Tier

| Angaben | Lammkotelett | Lammfilet |
|---|---|---|
| s (ges. Fette in g) | 7.50 | 1.98 |
| ks | 1.1 | 1.1 |
| vmax | 3 | 3 |
| =s/(s+ks) | 0.87 | 0.64 |
| =s/(s+ks)*(vmax) | 2.62 | 1.93 |
| c (Cholesterin in mg) | 91 | 80 |
| kc | 9000 | 9000 |
| vmax | 1 | 1 |
| =kc/(c$^2$*+k$_c$) | 0.52 | 0.58 |
| =vmax / kc/(c$^2$*+k$_c$) | 1.92 | 1.71 |
| d (Dichte kcal/100g) | 282 | 178 |

(fortgesetzt)

| Angaben | Lammkotelett | Lammfilet |
|---|---|---|
| kd | 1.05 | 1.05 |
| vmax | 1 | 1 |
| =kd/(kd+d) | 0.0037 | 0.0059 |
| =vmax / kd/(kd+d) /100 | 2.70 | 1.71 |
| EPIIndex | 14 | 6 |

Die Bewertung nach dem epigenetischen Index bewertet Lammkoteletts mit einem höheren EPIIndex (14) als Lammfilet mit einem EPIIndex von 6. Das bedeutet, dass die Kategorisierung neutral für Lammfilett eine günstigere Auswahl im Hinblick auf die Wirkung auf den Energiestoffwechsel bedeutet als Lammkoteletts mit der Bewertung für einen negativen Effekt und damit das Lammfilet die geeignetere Auswahl darstellt.

**Beispiel 2: Bestimmung des epigenetischen Index für verschiedene Lebensmittelklassen**

Der epigenetische Index wurde für verschiedene Lebensmittel der Lebensmittelkategorie A) unter Verwendung von Formel III und den Zahlenwerten gemäss Tabelle 1 berechnet und ist in Figur 1A-C dargestellt.
Der epigenetische Index wurde für verschiedene Lebensmittel der Lebensmittelkategorie B) (Beilagen) unter Verwendung von Formel IV und den Zahlenwerten gemäss Tabelle 1 berechnet und ist in Figur 2A-C dargestellt.
Der epigenetische Index wurde für verschiedene Lebensmittel der Lebensmittelkategorie B) (Broterzeugnisse) unter Verwendung von Formel IV und den Zahlenwerten gemäss Tabelle 1 berechnet und ist in Figur 3A-C dargestellt.
Der epigenetische Index wurde für verschiedene Lebensmittel der Lebensmittelkategorie C) unter Verwendung von Formel V und den Zahlenwerten gemäss Tabelle 1 berechnet und ist in Figur 4A-C dargestellt.
Der epigenetische Index wurde für verschiedene Lebensmittel der Lebensmittelkategorie D) unter Verwendung von Formel III und den Zahlenwerten gemäss Tabelle 1 berechnet und ist in Figur 5A-C dargestellt.
Der epigenetische Index wurde für verschiedene Lebensmittel der Lebensmittelkategorie E) unter Verwendung von Formel VI und den Zahlenwerten gemäss Tabelle 1 berechnet und ist in Figur 6A-C dargestellt.
Der epigenetische Index wurde für verschiedene Lebensmittel der Lebensmittelkategorie F) unter Verwendung von Formel VII und den Zahlenwerten gemäss Tabelle 1 berechnet und ist in Figur 7A-C dargestellt.

**Beispiel 3: Bestimmung der epigenetischen Last für eine Lebensmittelklasse**

Die Epigenetische Last von Lammfleisch wurde berechnet basierend auf dem in Beispiel 1 erhaltenen Epigenetischen Index unter Verwendung der Formel VIII:

$$\text{Epigenetische Last} = (\text{Epigenetischer Index eines Lebensmittels n}) * (\text{Konzentration an gesättigten Fetten des Lebensmittels n in g / 100g Lebensmittel n})* \text{Menge des aufgenommenen Lebensmittels n in g / 100 g};$$

wobei die Konzentration an gesättigten Fetten des Lebensmittels zweckmässig die Konzentration an gesättigten Fetten in 100 g essbarem Anteil eines Lebensmittels ist.

Tabelle 4: Epigenetische Last von Lammfleisch

| | Epigenetische Last | EPIIndex | ges. Fette / 100 g | Vergleichsmenge Fleisch |
|---|---|---|---|---|
| Lammkottelett | 105 | 14 | 7.5 | 100 g |
| Lammfilet | 12 | 6 | 2.0 | 100 g |

Die epigenetische Last von Lammkoteletts zur Beeinflussung des Energiestoffwechsel ist deutlich höher als die von Lammfilet, was bedeutet, dass im Vergleich zu 100 g Lammfilet nur gerade 8.8 g Lammkotelletts aufgenommen werden können, um die gleiche Wirkung am Energiestoffwechsel zu erzeugen.

**Beispiel 4: Bewertung von Menüs mit Lebensmittel mit unterschiedlichem epigenetischem Index**

Das Beispiel beschreibt zwei Menüs mit Lebensmittel mit unterschiedlichem epigenetischem Index und demonstriert die Auswahl der Lebensmittel basierend auf dem gemäss Formel III bis VII berechneten Epigenetischen Index sowie die Mengenauswahl der Lebensmittel in Bezug auf gesättigte Fette und die Summierung der gesättigten Fette ("Fettkonto") (s. Tabelle 5).

Tabelle 5: Epigenetische Last von zwei Menüs

| Menü Beispiel | Fettkonto (ges. Fette insgesamt in g) | EPIIndex | ges. Fette / 100 g | Essen in Gramm |
|---|---|---|---|---|
| Lammkottelett | 8 | 14 (berechnet nach Formel III) | 7.5 | 100 |
| Kartoffelkroketten | 11 | 9 (berechnet nach Formel IV) | 5 | 200 |
| Salat | 1 | 1 (berechnet nach Formel VI) | 2 | 30 |
| Summe | 20 | | | |
| | | | | |
| Menübeispiel mit veränderter Beilage und Fleisch | | | | |
| Lammfilet | 2 | 6 (berechnet nach Formel III) | 2 | 100 |
| Reis parboiled | 0.2 | 1 (berechnet nach Formel IV) | 0.1 | 200 |
| Salat | 1 | 1 (berechnet nach Formel VI) | 2 | 30 |
| Summe | 2.8 | | | |

Mithilfe der vereinfachten Berechnung lassen sich zielgerichtet Menüs mit positiver, neutraler oder negativer Wirkung auf den Energiestoffwechsel auswählen.

**Beispiel 5: Bewertung von Menüs enthaltend Lebensmittel unterschiedlicher Lebensmittelklassen**

Das Beispiel beschreibt zwei unterschiedliche Menüs mit unterschiedlicher epigenetischer Last enthaltend Lebensmittel unterschiedlicher Lebensmittelklassen und demonstriert die Anwendung einerseits der Auswahl der Lebensmittel durch den Epigenetischen Index sowie die Mengenauswahl der Lebensmittel in Bezug auf die epigenetische Last ("EPILast-Konto") mit der bevorzugten Berechnung des Epigenetischen Index gemäss Formel III bis VII und der epigenetische Last gemäss Formel VIII (s. Tabelle 6)

Tabelle 6: Epigenetische Last von zwei Menüs enthaltend Lebensmittel unterschiedlicher Lebensmittelklassen

| Menü Beispiel | Epigenetische Last | EPIIndex | ges. Fette / 100 g | Essen in Gramm |
|---|---|---|---|---|
| Lammkotelett | 105 | 14 (berechnet nach Formel III) | 8 | 100 |
| Karto ffelkroketten | 91 | 9 (berechnet nach Formel IV) | 5 | 200 |
| Salat | 1 | 1 (berechnet nach Formel VI) | 2 | 30 |

(fortgesetzt)

| Menü Beispiel | Epigenetische Last | EPIIndex | ges. Fette / 100 g | Essen in Gramm |
|---|---|---|---|---|
| Summe Last Menübeispiel mit verändertem Fleich und Beilage | 197 | | | |
| | | | | |
| Lammfilet | 12 | 6 (berechnet nach Formel III) | 2 | 100 |
| Reis parboiled | 0.2 | 1 (berechnet nach Formel IV) | 0.1 | 200 |
| Salat | 1 | 1 (berechnet nach Formel VI) | 2 | 30 |
| Summe Last | 13 | | | |

Mithilfe der epigenetischen Last lassen sich zielgerichtet Menüs mit positiver oder neutraler Wirkung oder negativer Wirkung auf das Epigenom auswählen. Die Berücksichtung der Formel VIII und die Auswahl der Lebensmittel aufgrund der epigenetischen Last berücksichtigt die Berechnung des Epigenetischen Index und bringt die lipoglucotoxische Wirkung der gesättigten Fette besonders bevorzugt ins Spiel und berücksichtigt so die ganze Bandbreite der epigenetischen Bewertungsmethode und nicht nur alleine gesättigte Fettsäuren.

**Beispiel 6: Diabetes Typ 2 Therapie basierend auf der gemäss der Bewertungsmethode kategorisierten Lebensmittel**

Verlauf der Therapie von einer vormals an Diabetes Typ 2 erkrankten Patientin (Figur 8), welche seit 5 Jahren diagnostiziert ist und die folgende Medikation vor der Therapie Ihres Diabetes Typ 2 aufwies:

Metfin® 1000 (1-0-1)
Meto Zeroc ® 50 (1-0-0)
Candesartan ® 16/12.5 (1-0-0)
Vitctoza ® 1.2 mg (0-0-1)
Atorvastatin ® 5 mg (0-0-1)

Die Therapie von Diabetes Typ 2 teilte sich in 3 Phasen auf, wobei die epigenetische Last in Phase I < 20 / d betrug , die Kalorienaufnahme mit Protiline und ausgewählten Gemüsen auf insgesamt 630 kcal / d beschränkt ist und 8 bis 9 Wochen dauert und keine anderen Lebensmittel aufgenommen werden, Phase II eine epigenetische Last von < 62 und in einem Kalorienbereich von anfänglich 900 kcal / d bis auf 1100 kcal / d gehalten wird und eine Ernährung mit Lebensmitteln bevorzugt in einem epigenetischen Indexbereich von 1 bis 3 und max. bis 9 erlaubt sind und auch aufgenommen wurden und 5 bis 7 Wochen dauert und schliesslich die Phase III eine maximale epigenetische Last von 108 eingestellt war, und der Kalorienbereich sich bei 1200 bis 1300 kcal / d eingestellt war. Die Auswahl der Lebensmittel ist auf einen epigenetischen Index von 1 bis 10 beschränkt. Die Phase III dauerte knapp 2 Monate. Verlaufsdaten (HOMA Index (-); HbA1c (-); Glucose nüchtern (mmol/l)

In den ersten 5 Wochen der Kurierung des Diabetes Typ 2 wurden sämtliche Medikamente bis auf das Blutdruckmittel Candesartan ® abgesetzt, der HOMA Index verbessert sich und fällt << 5, was die Grenze von Diabetes Typ 2 darstellt. In der Phase II wurde das Blutdruckmittel ebenfalls abgesetzt. Die Patientin ist in Phase III von Diabetes Typ 2 kuriert und ohne Medikamente. Die ersten 9 Wochen der Therapie wie auch die Langzeitbehandlung wurde mit L-Carnitin, durch Einnnahme der unten genannten Zusammensetzung begleitend zur Ernährungsstrategie unterstützt. Die Einnahme erfolgte wie angegeben morgens ("Morgenbeutel") und mittags ("Mittagsbeutel") über die ganze Therapiezeit.

| Morgen Beutel | Morgen Beutel | Mittagsbeutel | Summe | Angaben in |
|---|---|---|---|---|
| Oligo Fructose | 1250 | 1000 | 2250 | mg |

(fortgesetzt)

| Morgen Beutel | Morgen Beutel | Mittagsbeutel | Summe | Angaben in |
|---|---|---|---|---|
| L-Carnitin | 500 | 500 | 1000 | mg |
| Magnesium | 100 | | 100 | mg |
| Grüntee Extrakt | 75 | | 75 | mg |
| Folsäure | 30 | 30 | 60 | μg |
| Vitamin B12 | 0.31 | 0.31 | 0.62 | μg |
| Mate Tee Extrakt | | 600 | 600 | mg |
| L-Arginin | | 150 | 150 | mg |
| Soja Extrakt | | 12.5 | 12.5 | mg |
| davon Isoflavone | | 27.5 | 27.5 | mg |
| DHA (Omega-3) | | 1.5 | 1.5 | μg |
| Vitamin D | | 1.8 | 1.8 | mg |
| Vitamin E | | 2.4 | 2.4 | mg |
| Niacin | | 0.21 | 0.21 | mg |
| Vitamin B6 | | 12.5 | 12.5 | μg |
| Biotin | | 1.8 | 1.8 | mg |
| Panthothensäure | | 1.8 | 1.8 | mg |
| Einnahme | morgens | mittags | | |

**Beispiel 7: Diabetes Typ 2 Therapie basierend auf der gemäss der Bewertungsmethode kategorisierten Lebensmittel**

Therapie einer Kundin (weiblich, 65 Jahre, keine Medikation, BMI bei Start Therapie von 30), basierend auf einer geänderten Auswahl der Lebensmittel anhand des epigenetischen Index und der epigenetischen Last leidend an Diabetes Typ 2 aufgrund einer ungeeigneten langandauernden Lebensmittelauswahl im epigenetisch ungünstigen Bereich und ungünstiger zirkadianer Lebensmittelaufnahme führend zu erhöhter epigenetischer Last pro Tag.

Die Lebensmittelaufnahme war vor der Therapie geprägt von Lebensmittels mit einem epigenetischen Index > 10 und einer aufgenommen epigenetischen Last von >>192. Die Energiebilanz wurde leicht reduziert für eine Körpergewichtsreduktion von 0.3 kg /Woche und die Auswahl der Lebensmittel auf einen epigenetischen Index von max. 10 beschränkt. Über die angegebene Therapiezeit von ca. 3 Monaten hat die Patientin das Körpergewicht um ca. 3 kg reduziert. Die epigenetische Last wurde auf < 144 / d beschränkt. Die Patientin hat deutliche Esspausen von 4- 6 h zwischen den Hauptmahlzeiten eingehalten. Die Glucose Werte nüchtern gemessen vor Therapiebeginn waren schwankend und oft > 7 und die Patientin war eine beginnend mit Diabetes Typ 2 mit einem HbA1c von 6.8 (Figur 9). Nach 3 Monaten Therapie mit der beschriebenen epigenetischen Essensauswahl zeigt der HbA1c noch einen Wert von 6.3 auf und die Glucose Messwerte waren stabil immer <7. Die Gewichtsreduktion ist minimal und die Verbesserung der Blutwerte kann nicht allein auf die minimale Gewichtsreduktion von 3 kg Körpergewicht zurückzuführen sein sondern ist primär auf die reduzierte epigenetische Last und der Lebensmittelauswahl nach dem epigenetischen Index zurückzuführen. Die Therapie wurde mit L-Carnitin wie in Beispiel 6 beschrieben begleitend zur Ernährungsstrategie unterstützt.

**Beispiel 8: Insulinresistenz Therapie basierend auf der gemäss der Bewertungsmethode kategorisierten Lebensmittel**

Therapie eines Kunden (männlich, 53 Jahre) basierend auf einer geänderten Auswahl der Lebensmittel anhand des epigenetischen Index und der epigenetischen Last leidend an Insulinresistenz mit ausgeprägtem Bauchfett bei Normalgewicht (BMI < oberer Wert Norm BMI) aufgrund einer ungeeigneten langandauernden Lebensmittelauswahl im epigenetisch ungünstigen Bereich von 10- 27) führend zu Herzrasen, Schlaflosigkeit und ausgeprägter körperlicher Müdigkeit aufgrund täglich überhöhter epigenetischer Last über mehrere Jahre.

Tabelle 7: Blutwerte

| Messwerte | 1. Analyse (31.1.2012) | 2. Analyse (5.7.2012) |
|---|---|---|
| HDL (in mmol/l) | 1.25 | 1.25 |
| Cholesterin (in mmol/l) | 5.9 | 4.73 |
| Triglyceride (in mmol/l) | 2.15 | 0.8 |
| LDL (in mmol/l) | 3.7 | 3.13 |
| Glucose (in mmol/l) | 5.1 | 4.81 |
| BMI (-) | 23.5 | 23 |

Die Blutwerte (s. Tabelle 7) zum Start der Therapie (1. Analyse) zeigen überhöhte Blutfettwerte insbesondere des Triglycerides sowie LDL bei normalen Glucosewerten auf. Eine über Jahre Aufnahme an Lebensmitteln aus dem ungeeigneten epigenetischen Index Bereich > 10 und einer epigenetischen Last von >> 192 führten zur chronischen Erkrankung mit der Folge der Unfähigkeit zur Arbeitsausführung.

Eine Veränderung der Ernährung basierend auf einer maximalen epigenetischen Last von < 144, der Einführung von Essenspausen von 4-6 h zwischen den Hauptmahlzeiten und einer Auswahl der Lebensmittel bei einem epigenetischen Index <10 führten zur Verbesserung des Wohlbefindens nach ca. 2 Monaten und Aufnahme der Arbeitstätigkeit nach 4 Wochen. Die Blutwerte (2. Analyse) zeigten aufgrund der epigenetischen Essensauswahl mit der Auswahl von Lebensmitteln mit einem epigenetischen Index < 10 und einer epigenetischen Last pro Tag von <144 bei einer energieseitig bilanzierten Kalorienaufnahme passend zum BMI und der Tätigkeit, nach 6 Monaten eine dramatische Veränderung insbesondere bei den Blutfettwerten Triglyceriden und LDL sowie Gesamtcholesterin. Die Ernährungsweise nach dem epigenetischen Index sowie der epigenetischen Last therapiert normalgewichtige Personen mit chronischen Krankheitszeichen eines beginnen Burnouts mit Arbeitsunfähigkeit innert 6 Monaten nachhaltig und ermöglichten eine Weiterführung des Lebens mithilfe des neu erlernten Lebensstils mit der Auswahl der Lebensmittel nach epigenetischen Kriterien.

Die Therapie wurde mit L-Carnitin wie in Beispiel 6 beschrieben begleitend zur Ernährungsstrategie unterstützt.

**Beispiel 9: Insulinresistenz und Übergewichts-Therapie basierend auf der gemäss der Bewertungsmethode kategorisierten Lebensmittel**

Therapie eines Kunden (männlich, 30 Jahre) basierend auf einer geänderten Auswahl der Lebensmittel anhand des epigenetischen Index und der epigenetischen Last leidend an Insulinresistenz mit ausgeprägtem Bauchfett bei Übergewicht (BMI > oberer Wert Norm BMI) aufgrund einer ungeeigneten langandauernden Lebensmittelauswahl im epigenetisch ungünstigen Bereich von 10- 27) führend zu ausgeprägter körperlicher Müdigkeit und Übergewicht aufgrund täglich überhöhter epigenetischer Last über mehrere Jahre.

Tabelle 8: Blutwerte

| Analyse | 1. Analyse 23.04.13 | 2. Analyse 04.10.13 | Einheit |
|---|---|---|---|
| Cholesterin | 5.19 | 4.24 | mmol/l |
| HDL-Cholesterin | 0.97 | 1.03 | mmol/l |
| LDL-Cholesterin | 2.93 | 2.65 | mmol/l |
| Triglyceride | 2.82 | 1.22 | mmol/l |
| Glucose | 5.35 | 4.19 | mmol/l |
| Bauchumfang | 113 | 102 | cm |
| BMI | 33.2 | 28.7 | kg/m$^2$ |
| EPITyp® | 8 | 4 | |

Die Blutwerte (s. Tabelle 8) zum Start der Therapie (1. Analyse) zeigen überhöhte Blutfettwerte insbesondere des Triglycerides sowie LDL bei normalen Glucosewerten auf. Eine über Jahre Aufnahme an Lebensmitteln aus dem ungeeigneten epigenetischen Index Bereich > 10 und einer epigenetischen Last von >> 192 führten zu Insulinresistenz und Übergewicht. Eine Veränderung der Ernährung basierend auf einer maximalen epigenetischen Last von < 144, der Einführung von Essenspausen von 4-6 h zwischen den Hauptmahlzeiten und einer Auswahl der Lebensmittel bei einem epigenetischen Index <10 führten zur Verbesserung des Wohlbefindens nach ca. 1 Monat und deutlicher Reduktion des Körpergewichtes und Bauchumfanges nach 6 Monaten sowie Verbesserung der Insulinsensitivität. Die Blutwerte (2. Analyse) zeigten aufgrund der epigenetischen Essensauswahl mit der Auswahl von Lebensmitteln mit einem epigenetischen Index < 10 und einer epigenetischen Last pro Tag von <144 bei einer energieseitig leicht reduzierten Kalorienaufnahme (ca. 500 kcal / d) nach 6 Monaten eine dramatische Veränderung insbesondere bei den Blutfettwerten Triglyceriden und Verbesserung des LDL sowie Gesamtcholesterin. Die Ernährungsweise nach dem epigenetischen Index sowie der epigenetischen Last therapiert übergewichtie Personen mit chronischen Krankheitszeichen eines beginnen Prädiabetes (Insulinresistenz) innert 6 Monaten nachhaltig und ermöglichten eine Weiterführung des Lebens mithilfe des neu erlernten Lebensstils mit der Auswahl der Lebensmittel nach epigenetischen Kriterien. Die Therapie wurde mit L-Carnitin wie in Beispiel 6 beschriebenbegleitend zur Ernährungsstrategie unterstützt.

## Patentansprüche

1. Methode zur Bewertung eines Lebensmittels umfassend

    i) Bestimmung der Energiedichte des Lebensmittels;
    ii) Auswahl von mindestens einem epigenetisch aktiven Inhaltsstoff des Lebensmittels, der den Energiestoffwechsel eines Säuger beeinflusst;
    iii) Bestimmung der Konzentration des mindestens einen epigenetisch aktiven Inhaltsstoffes, der gemäss ii) ausgewählt wurde, im Lebensmittel;
    iv) Berechnung des Einflusses des Lebensmittels auf den Energiestoffwechsel des Säugers durch mathematische Kombination der gemäss i) bestimmten Energiedichte des Lebensmittels und der gemäss iii) bestimmten Konzentration des mindestens einen epigenetisch aktiven Inhaltsstoffes im Lebensmittel;
    v) Kategorisierung des Lebensmittels im Hinblick auf seinen Einfluss auf den Energiestoffwechsel des Säugers gemäss Berechung iv) wobei zwischen einem positiven, einem neutralen und einem negativen Einfluss des Lebensmittels auf den Energiestoffwechsel des Säugers unterschieden wird,

wobei die Berechnung iv) des Einflusses des Lebensmittels auf den Energiestoffwechsel des Säugers für die Lebensmittelklassen A)-D) gemäss folgender Formel I

$$\text{Epigenetischer Index (EPI)}_{A)-D)} = [v_{max,d} / k_d/(k_d+d) /100] * [s/(s+k_s) * (v_{max,s})],$$

wobei

$k_d$ = Inhibitionskonstante für enzymatische Reaktion mit d
$v_{max,d}$ = Reaktionsgeschwindigkeit für enzymatische Reaktion mit d
d = Energiedichte des Lebensmittels
$v_{max,s}$ = Reaktionsgeschwindigkeit für enzymatische Reaktion mit s
s = Konzentration gesättigte Fette im Lebensmittel
$k_s$ = Sättigungskonstante für enzymatische Reaktion mit s,

und für die Lebensmittelklassen E)-F) gemäss folgender Formel II

$$\text{Epigenetischer Index }_{E)-F)} = [v_{max,d} / k_d/(k_d+d) /100] * [(k_b^2+b)/b /v_{max,b}],$$

wobei

$k_d$ = Inhibitionskonstante für enzymatische Reaktion mit d
$v_{max,d}$ = Reaktionsgeschwindigkeit für enzymatische Reaktion mit d
d = Energiedichte des Lebensmittels
$v_{max,b}$ = Reaktionsgeschwindigkeit für enzymatische Reaktion mit b
b = Konzentration Ballaststoffe im Lebensmittel
$k_b$=Sättigungskonstante für enzymatische Reaktion mit b,

vorgenommen wird, wobei zwischen folgenden Klassen von Lebensmitteln unterschieden wird: A) Fleischerzeugnisse, B) Beilagen und/oder Broterzeugnisse, C) Fischerzeugnisse, D) Milcherzeugnisse, E) Gemüse und F) Früchte.

2. Methode zur Bewertung eines Lebensmittels gemäss Anspruch 1, wobei je nach Lebensmittelklasse der mindestens eine epigenetisch aktive Inhaltsstoff folgendermassen ausgewählt wird:

A) Fleischerzeugnisse: epigenetisch aktiver Inhaltsstoff ausgewählt aus der Gruppe bestehend aus gesättigte Fetten und Cholesterin;
B) Beilagen und/oder Broterzeugnisse: epigenetisch aktiver Inhaltsstoff ausgewählt aus der Gruppe bestehend aus gesättigte Fette und Kohlenhydrate;
C) Fischerzeugnisse: epigenetisch aktiver Inhaltsstoff ausgewählt aus der Gruppe bestehend aus gesättigte Fette und Omega-3-Fettsäuren;
D) Milcherzeugnisse: epigenetisch aktiver Inhaltsstoff ausgewählt aus der Gruppe bestehend aus gesättigte Fette und Cholesterin;
E) Gemüse: epigenetisch aktiver Inhaltsstoff ausgewählt aus der Gruppe bestehend aus Ballaststoffe und Folsäure;
F) Früchte: epigenetisch aktiver Inhaltsstoff ausgewählt aus der Gruppe bestehend aus Ballaststoffe und Kohlenhydrate.

3. Methode zur Bewertung eines Lebensmittels gemäss Anspruch 2, wobei das Lebensmittel aus den Klassen A) und D) ausgewählt wird und zur Berechnung des Epigenetischen Index für die Lebensmittelklassen A) und D) die folgende Formel III eingesetzt wird, wobei die Energiedichte und gesättigte Fettsäuren sowie Cholesterin in die Formel einfliessen:

$$\text{Epigenetischer Index}_{A) \text{ und } D)} = [v_{max,d} \, / \, k_d/(k_d+d) \, /100] * [s/(s+k_s) * (v_{max,s})] * [v_{max,c} \, / \, k_c/(c^2 +k_c)],$$

wobei

$k_d$ = Inhibitionskonstante für enzymatische Reaktion mit d
$v_{max,d}$ = Reaktionsgeschwindigkeit für enzymatische Reaktion mit d
d = Energiedichte des Lebensmittels
$v_{max,s}$ = Reaktionsgeschwindigkeit für enzymatische Reaktion mit s
s = Konzentration gesättigte Fette im Lebensmittel
$k_s$ = Sättigungskonstante für enzymatische Reaktion mit s
$v_{max,c}$ = Reaktionsgeschwindigkeit für enzymatische Reaktion mit c
c = Konzentration Cholesterin im Lebensmittel.
$k_c$ = Reaktionskonstante für enzymatische Reaktion mit c.

4. Methode zur Bewertung eines Lebensmittels gemäss Anspruch 2, wobei das Lebensmittel aus der Klasse B) ausgewählt wird und zur Berechnung des Epigenetischen Index für die Lebensmittelklasse B) die folgende Formel IV eingesetzt, wobei die Energiedichte und gesättigte Fettsäuren sowie Kohlehydrate angegeben als Glykämischer Index in die Formel einfliessen:

$$\text{Epigenetischer Index}_{B)} = [v_{max,d} \, / \, k_d/(k_d+d) \, /100] * [s/(s+k_s) * (v_{max,s})] * [v_{max,g} \, /k_g/(g^3 +k_g)],$$

wobei

$k_d$ = Inhibitionskonstante für enzymatische Reaktion mit d
$v_{max,d}$ = Reaktionsgeschwindigkeit für enzymatische Reaktion mit d
d = Energiedichte des Lebensmittels
$v_{max,s}$ = Reaktionsgeschwindigkeit für enzymatische Reaktion mit s

s = Konzentration gesättigte Fette im Lebensmittel

$k_s$ = Sättigungskonstante für enzymatische Reaktion mit s

$v_{max,g}$ = Reaktionsgeschwindigkeit für enzymatische Reaktion mit g

g = Konzentration Kohlenhydrate im Lebensmittel angegeben als Glykämischer Index des Lebensmittels

$k_g$ = Reaktionskonstante für enzymatische Reaktion mit g.

5. Methode zur Bewertung eines Lebensmittels gemäss Anspruch 2, wobei das Lebensmittel aus der Klasse C) ausgewählt wird und zur Berechnung des Epigenetischen Index für die Lebensmittelklasse C) die folgende Formel V eingesetzt, wobei die Energiedichte und gesättigte Fettsäuren sowie Omega-3-Fettsäuren in die Formel einfliessen:

$$\text{Epigenetischer Index}_{C)} = [v_{max,d} / k_d/(k_d+d) /100] * [s/(s+k_s) * (v_{max,s})] * [v_{max,o} *(o+k_o^2)/o],$$

wobei

$k_d$ = Inhibitionskonstante für enzymatische Reaktion mit d

$v_{max,d}$ = Reaktionsgeschwindigkeit für enzymatische Reaktion mit d

d = Energiedichte des Lebensmittels

$v_{max,s}$ = Reaktionsgeschwindigkeit für enzymatische Reaktion s

s = Konzentration gesättigte Fette im Lebensmittel

$k_s$ = Sättigungskonstante für enzymatische Reaktion mit s

$v_{max,o}$ = Reaktionsgeschwindigkeit für enzymatische Reaktion mit o

o = Konzentration Omega-3-Fettsäuren im Lebensmittel

$k_o$ = Reaktionskonstante für enzymatische Reaktion mit o.

6. Methode zur Bewertung eines Lebensmittels gemäss Anspruch 2, wobei das Lebensmittel aus der Klasse E) ausgewählt wird und zur Berechnung des Epigenetischen Index für die Lebensmittelklasse E) die folgende Formel VI eingesetzt, wobei die Energiedichte und Ballaststoffe sowie Folsäure in die Formel einfliessen:

$$\text{Epigenetischer Index}_{E)} = = [v_{max,d} / k_d/(k_d+d) /100] * [(k_b^2+b)/b/v_{max,b}] * [ (k_f^2+f)/f /v_{max,f}],$$

wobei

$k_d$ = Inhibitionskonstante für enzymatische Reaktion mit d

$v_{max,d}$ = Reaktionsgeschwindigkeit für Reaktion d

d = Energiedichte des Lebensmittels

$v_{max,b}$ = Reaktionsgeschwindigkeit für enzymatische Reaktion mit b

b = Konzentration Ballaststoffe im Lebensmittel

$k_b$ = Reaktionskonstante für enzymatische Reaktion mit b

$v_{max,f}$ = Reaktionsgeschwindigkeit für enzymatische Reaktion mit f

f = Konzentration Folsäure im Lebensmittel

$k_f$ = Reaktionskonstante für enzymatische Reaktion mit f.

7. Methode zur Bewertung eines Lebensmittels gemäss Anspruch 2, wobei das Lebensmittel aus der Klasse F) ausgewählt wird und zur Berechnung des Epigenetischen Index für die Lebensmittelklassen F) die folgende Formel VII eingesetzt, wobei die Energiedichte und Ballaststoffe sowie Kohlehydrate angegeben als Glykämischer Index in die Formel einfliessen:

$$\text{Epigenetischer Index}_{F)} = [v_{max,d} / k_d/(k_d+d) /100] * [(k_b^2+b)/b/v_{max,b}] * [v_{max,g} /k_g/(g^3 +k_g)],$$

wobei

$k_d$ = Inhibitionskonstante für enzymatische Reaktion mit d
$v_{max,d}$ = Reaktionsgeschwindigkeit für Reaktion d
d = Energiedichte des Lebensmittels
$v_{max,b}$ = Reaktionsgeschwindigkeit für enzymatische Reaktion mit b
b = Konzentration Ballaststoffe im Lebensmittel
$k_b$ = Reaktionskonstante für enzymatische Reaktion mit b
$v_{max,g}$ = Reaktionsgeschwindigkeit für enzymatische Reaktion mit g
g = Konzentration Kohlenhydrate im Lebensmittel angegeben als Glykämischer Index des Lebensmittels
$k_g$ = Reaktionskonstante für enzymatische Reaktion mit g.

8. Methode zur Bewertung eines Lebensmittels gemäss Anspruch 2, wobei das Lebensmittel aus den Klassen A) bis D) ausgewählt wird und der mindestens eine epigenetisch aktive Inhaltsstoff des Lebensmittels gesättigte Fette sind.

9. Methode zur Bewertung eines Lebensmittels gemäss Anspruch 8, wobei eine Energiedichte des Lebensmittels von höher als 250kcal/100g und eine Konzentration von gesättigten Fetten von 0-5g/100g oder grösser als 5g/100g einen negativen, eine Energiedichte des Lebensmittels von 150 bis 250kcal/100g und eine Konzentration von gesättigten Fetten von 0-5g/100g oder grösser als 5g/100g einen neutralen, eine Energiedichte des Lebensmittels bis 150kcal/100g und eine Konzentration von gesättigten Fetten grösser als 5g/100g einen neutralen, und eine Energiedichte des Lebensmittels bis 150kcal/100g und eine Konzentration von gesättigten Fetten von 0-5g/100g einen positiven Einfluss des Lebensmittels auf den Energiestoffwechsel des Säugers bedeutet.

10. Methode zur Bewertung eines Lebensmittels gemäss Anspruch 2, wobei das Lebensmittel aus den Klassen E) bis F) ausgewählt wird und der mindestens eine epigenetisch aktive Inhaltsstoff des Lebensmittels Ballaststoffe sind.

11. Methode zur Bewertung eines Lebensmittels gemäss Anspruch 10, wobei eine Energiedichte von höher als 250kcal/100g und eine Konzentration von Ballaststoffen von 0-0,5g/100g einen negativen, eine Energiedichte des Lebensmittels von höher als 250kcal/100g oder eine Energiedichte des Lebensmittels von 150 bis 250kcal/100g und eine Konzentration von Ballaststoffen von 0-0,5g/100g oder grösser als 0,5g/100g einen neutralen, eine Energiedichte des Lebensmittels bis 150kcal/100g und eine Konzentration von Ballaststoffen von 0-0,1g/100g einen neutralen, und eine Energiedichte des Lebensmittels bis 150kcal/100g und eine Konzentration von Ballaststoffen von 0,1-5g/100g oder grösser als 0,5g/100g einen positiven Einfluss des Lebensmittels auf den Energiestoffwechsel des Säugers bedeutet.

12. Methode zur Bewertung eines Lebensmittels gemäss Anspruch 1, wobei der gemäss Formel I erhaltene Wert für den Epigenetischen Index $(EPI)_{A)-D)}$ mit der Menge an gesättigten Fetten des Lebensmittels bezogen auf die Menge des Lebensmittels multipliziert wird.

**Claims**

1. Method for evaluating a foodstuff, comprising

   i) determining the energy density of the foodstuff;
   ii) selecting at least one epigenetically active ingredient of the foodstuff that influences the energy metabolism of a mammal;
   iii) determining the concentration of the at least one epigenetically active ingredient that was selected as per ii) in the foodstuff;
   iv) calculating the influence of the foodstuff on the energy metabolism of the mammal by mathematical combination of the energy density of the foodstuff that was determined as per i) and the concentration of the at least one epigenetically active ingredient in the foodstuff that was determined as per iii);
   v) categorizing the foodstuff with respect to its influence on the energy metabolism of the mammal as per calculation iv), with a distinction being made between a positive, a neutral and a negative influence of the foodstuff on the energy metabolism of the mammal,

   with the calculation iv) of the influence of the foodstuff on the energy metabolism of the mammal being done as per the following formula I for foodstuff classes A)-D)

$$\text{Epigenetic Index } (EPI)_{A)-D)} = [v_{max,d} / k_d/(k_d+d) /100] * [s/(s+k_s) * (v_{max,s})],$$

where

$k_d$ = inhibition constant for enzymatic reaction with d
$v_{max,d}$ = reaction rate for enzymatic reaction with d
d = energy density of the foodstuff
$v_{max,s}$ = reaction rate for enzymatic reaction with s
s = concentration of saturated fats in the foodstuff
$k_s$ = saturation constant for enzymatic reaction with s,

and as per the following formula II for foodstuff classes E)-F)

$$\text{Epigenetic Index }_{E)-F)} = [v_{max,d} / k_d/(k_d+d) /100] * [(k_b^2+b)/b /v_{max,b}],$$

where

$k_d$ = inhibition constant for enzymatic reaction with d
$v_{max,d}$ = reaction rate for enzymatic reaction with d
d = energy density of the foodstuff
$v_{max,b}$ = reaction rate for enzymatic reaction with b
b = concentration of dietary fibres in the foodstuff
$k_b$ = saturation constant for enzymatic reaction with b,

with a distinction being made between the following classes of foodstuffs: A) meat products, B) sides and/or bread products, C) fish products, D) milk products, E) vegetables and F) fruits.

2. Method for evaluating a foodstuff according to Claim 1, wherein the at least one epigenetically active ingredient is selected depending on foodstuff class as follows:

A) meat products: epigenetically active ingredient selected from the group consisting of saturated fats and cholesterol;
B) sides and/or bread products: epigenetically active ingredient selected from the group consisting of saturated fats and carbohydrates;
C) fish products: epigenetically active ingredient selected from the group consisting of saturated fats and omega-3 fatty acids;
D) milk products: epigenetically active ingredient selected from the group consisting of saturated fats and cholesterol;
E) vegetables: epigenetically active ingredient selected from the group consisting of dietary fibres and folic acid;
F) fruits: epigenetically active ingredient selected from the group consisting of dietary fibres and carbohydrates.

3. Method for evaluating a foodstuff according to Claim 2, wherein the foodstuff is selected from classes A) and D) and the following formula III is used for the calculation of the epigenetic index for foodstuff classes A) and D), with the energy density and saturated fatty acids and cholesterol being incorporated in the formula:

$$\text{Epigenetic Index }_{A) \text{ and } D)} = [v_{max,d} / k_d/(k_d+d) /100] * [s/(s+k_s) * (v_{max,s})] * [v_{max,c} / kc/(c^2 +k_c)],$$

where

$k_d$ = inhibition constant for enzymatic reaction with d
$v_{max,d}$ = reaction rate for enzymatic reaction with d
d = energy density of the foodstuff

$v_{max,s}$ = reaction rate for enzymatic reaction with s

s = concentration of saturated fats in the foodstuff

$k_s$ = saturation constant for enzymatic reaction with s

$v_{max,c}$ = reaction rate for enzymatic reaction with c

c = concentration of cholesterol in the foodstuff

$k_c$ = reaction constant for enzymatic reaction with c.

4. Method for evaluating a foodstuff according to Claim 2, wherein the foodstuff is selected from class B) and the following formula IV is used for the calculation of the epigenetic index for foodstuff class B), with the energy density and saturated fatty acids and carbohydrates specified as glycaemic index being incorporated in the formula:

$$\text{Epigenetic Index}_{B)} = [v_{max,d} / k_d/(k_d+d) /100] * [s/(s+k_s) * (v_{max,s})] * [v_{max,g} /k_g/(g^3+k_g],$$

where

$k_d$ = inhibition constant for enzymatic reaction with d

$v_{max,d}$ = reaction rate for enzymatic reaction with d

d = energy density of the foodstuff

$v_{max,s}$ = reaction rate for enzymatic reaction with s

s = concentration of saturated fats in the foodstuff

$k_s$ = saturation constant for enzymatic reaction with s

$v_{max,g}$ = reaction rate for enzymatic reaction with g

g = concentration of carbohydrates in the foodstuff specified as glycaemic index of the foodstuff

$k_g$ = reaction constant for enzymatic reaction with g.

5. Method for evaluating a foodstuff according to Claim 2, wherein the foodstuff is selected from class C) and the following formula V is used for the calculation of the epigenetic index for foodstuff class C), with the energy density and saturated fatty acids and omega-3 fatty acids being incorporated in the formula:

$$\text{Epigenetic Index}_{C)} = [v_{max,d} / k_d/(k_d+d) /100] * [s/(s+k_s) * (v_{max,s})] * [v_{max,o} * (o+k_o^2)/o],$$

where

$k_d$ = inhibition constant for enzymatic reaction with d

$v_{max,d}$ = reaction rate for enzymatic reaction with d

d = energy density of the foodstuff

$v_{max,s}$ = reaction rate for enzymatic reaction s

s = concentration of saturated fats in the foodstuff

$k_s$ = saturation constant for enzymatic reaction with s

$v_{max,o}$ = reaction rate for enzymatic reaction with o

o = concentration of omega-3 fatty acids in the foodstuff

$k_o$ = reaction constant for enzymatic reaction with o.

6. Method for evaluating a foodstuff according to Claim 2, wherein the foodstuff is selected from class E) and the following formula VI is used for the calculation of the epigenetic index for foodstuff class E), with the energy density and dietary fibres and folic acid being incorporated in the formula:

$$\text{Epigenetic Index}_{E)} = [v_{max,d} / k_d/(k_d+d) /100] * [(k_b^2+b)/b/v_{max,b}] * [(k_f^2+f)/f/v_{max,f}],$$

where

$k_d$ = inhibition constant for enzymatic reaction with d

$v_{max,d}$ = reaction rate for reaction d
d = energy density of the foodstuff
$v_{max,b}$ = reaction rate for enzymatic reaction with b
b = concentration of dietary fibres in the foodstuff
$k_b$ = reaction constant for enzymatic reaction with b
$v_{max,f}$ = reaction rate for enzymatic reaction with f
f = concentration of folic acid in the foodstuff
$k_f$ = reaction constant for enzymatic reaction with f.

7. Method for evaluating a foodstuff according to Claim 2, wherein the foodstuff is selected from class F) and the following formula VII is used for the calculation of the epigenetic index for foodstuff classes F), with the energy density and dietary fibres and carbohydrates specified as glycaemic index being incorporated in the formula:

$$\text{Epigenetic Index}_{F)} = [v_{max,d} / k_d/(k_d+d) /100] * [(k_b^2+b)/b/v_{max,b}] * [v_{max,g}/k_g/(g^3+k_g)],$$

where

$k_d$ = inhibition constant for enzymatic reaction with d
$v_{max,d}$ = reaction rate for reaction d
d = energy density of the foodstuff
$v_{max,b}$ = reaction rate for enzymatic reaction with b
b = concentration of dietary fibres in the foodstuff
$k_b$ = reaction constant for enzymatic reaction with b
$v_{max,g}$ = reaction rate for enzymatic reaction with g
g = concentration of carbohydrates in the foodstuff specified as glycaemic index of the foodstuff
$k_g$ = reaction constant for enzymatic reaction with g.

8. Method for evaluating a foodstuff according to Claim 2, wherein the foodstuff is selected from classes A) to D) and the at least one epigenetically active ingredient of the foodstuff is saturated fats.

9. Method for evaluating a foodstuff according to Claim 8, wherein an energy density of the foodstuff of higher than 250 kcal/100 g and a concentration of saturated fats of 0-5 g/100 g or greater than 5 g/100 g means a negative influence of the foodstuff on the energy metabolism of the mammal, an energy density of the foodstuff of 150 to 250 kcal/100 g and a concentration of saturated fats of 0-5 g/100 g or greater than 5 g/100 g means a neutral one, an energy density of the foodstuff up to 150 kcal/100 g and a concentration of saturated fats greater than 5 g/100 g means a neutral one, and an energy density of the foodstuff up to 150 kcal/100 g and a concentration of saturated fats of 0-5 g/100 g means a positive one.

10. Method for evaluating a foodstuff according to Claim 2, wherein the foodstuff is selected from classes E) to F) and the at least one epigenetically active ingredient of the foodstuff is dietary fibres.

11. Method for evaluating a foodstuff according to Claim 10, wherein an energy density of higher than 250 kcal/100 g and a concentration of dietary fibres of 0-0.5 g/100 g means a negative influence of the foodstuff on the energy metabolism of the mammal, an energy density of the foodstuff of higher than 250 kcal/100 g or an energy density of the foodstuff of 150 to 250 kcal/100 g and a concentration of dietary fibres of 0-0.5 g/100 g or greater than 0.5 g/100 g means a neutral one, an energy density of the foodstuff up to 150 kcal/100 g and a concentration of dietary fibres of 0-0.1 g/100 g means a neutral one, and an energy density of the foodstuff up to 150 kcal/100 g and a concentration of dietary fibres of 0.1-5 g/100 g or greater than 0.5 g/100 g means a positive one.

12. Method for evaluating a foodstuff according to Claim 1, wherein the value for the epigenetic index $(EPI)_{A)-D)}$ that was obtained as per formula I is multiplied by the amount of saturated fats in the foodstuff based on the amount of the foodstuff.

**Revendications**

1. Procédé pour l'évaluation d'un produit alimentaire comprenant

    i) la détermination de la densité d'énergie du produit alimentaire ;
    ii) la sélection d'au moins un ingrédient épigénétiquement actif du produit alimentaire, qui influence le métabolisme énergétique d'un mammifère ;
    iii) la détermination de la concentration de l'au moins un ingrédient épigénétiquement actif, qui a été sélectionné selon ii), dans le produit alimentaire ;
    iv) le calcul de l'influence du produit alimentaire sur le métabolisme énergétique du mammifère par une combinaison mathématique de la densité d'énergie du produit alimentaire déterminée selon i) et de la concentration de l'au moins un ingrédient épigénétiquement actif dans le produit alimentaire déterminée selon iii) ;
    v) la catégorisation du produit alimentaire en ce qui concerne son influence sur le métabolisme énergétique du mammifère selon le calcul iv), dans laquelle on différencie entre un effet positif, un effet neutre et un effet négatif du produit alimentaire sur le métabolisme énergétique du mammifère,

    dans lequel on effectue le calcul iv) de l'influence du produit alimentaire sur le métabolisme énergétique du mammifère pour les classes de produits alimentaires A)-D) d'après la formule suivante I

    $$\text{index épigénétique (IEP)}_{A)-D)} = [v_{max,d}/k_d/(k_d+d)/100]*[s/(s+k_s)*(v_{max,s})],$$

    dans laquelle

    $k_d$ = constante d'inhibition pour la réaction enzymatique avec d
    $v_{max,d}$ = vitesse de réaction pour la réaction enzymatique avec d
    d = densité d'énergie du produit alimentaire
    $v_{max,s}$ = vitesse de réaction pour la réaction enzymatique avec s
    s = concentration en graisses saturées dans le produit alimentaire
    $k_s$ = constante de saturation pour la réaction enzymatique avec s,

    et pour les classes de produits alimentaires E)-F) d'après la formule suivante II

    $$\text{index épigénétique}_{E)-F)} = [v_{max,d}/k_d/(k_d+d)/100]*[(k_b^2+b)/b/v_{max,b}],$$

    dans laquelle

    $k_d$ = constante d'inhibition pour la réaction enzymatique avec d
    $v_{max,d}$ = vitesse de réaction pour la réaction enzymatique avec d
    d = densité d'énergie du produit alimentaire
    $v_{max,b}$ = vitesse de réaction pour la réaction enzymatique avec b
    b = concentration en fibres alimentaires dans le produit alimentaire
    $k_b$ = constante de saturation pour la réaction enzymatique avec b,

    dans lesquelles on différencie entre les classes suivantes de produits alimentaires : A) produits carnés, B) garnitures et/ou produits de panification, C) produits à base de poisson, D) produits à base de lait, E) légumes et F) fruits.

2. Procédé pour l'évaluation d'un produit alimentaire selon la revendication 1, l'au moins un ingrédient épigénétiquement actif, selon la classe de produits alimentaires, étant choisi de la manière suivante :

    A) produits carnés : ingrédient épigénétiquement actif choisi dans le groupe constitué par les graisses saturées et le cholestérol ;
    B) garnitures et/ou produits de panification : ingrédient épigénétiquement actif choisi dans le groupe constitué par les graisses saturées et les glucides ;
    C) produits à base de poisson : ingrédient épigénétiquement actif choisi dans le groupe constitué par les graisses saturées et les acides gras oméga-3 ;

D) produits à base de lait : ingrédient épigénétiquement actif choisi dans le groupe constitué par les graisses saturées et le cholestérol ;

E) légumes : ingrédient épigénétiquement actif choisi dans le groupe constitué par les fibres alimentaires et l'acide folique ;

F) fruits : ingrédient épigénétiquement actif choisi dans le groupe constitué par les fibres alimentaires et les glucides.

3. Procédé pour l'évaluation d'un produit alimentaire selon la revendication 2, le produit alimentaire des classes A) et D) étant choisi et pour le calcul de l'index épigénétique pour les classes de produits alimentaires A) et D) la formule III suivante étant utilisée, la densité d'énergie et les acides gras saturés ainsi que le cholestérol étant introduits dans la formule :

$$\text{index épigénétique}_{A) \text{ et } D)} = [v_{max,d}/k_d/(k_d+d)/100]*[s/(s+k_s)*(v_{max,s})]*[v_{max,c}/k_c/(c^2 + k_c)],$$

dans laquelle

$k_d$ = constante d'inhibition pour la réaction enzymatique avec d
$V_{max,d}$ = vitesse de réaction pour la réaction enzymatique avec d
d = densité d'énergie du produit alimentaire
$v_{max,s}$ = vitesse de réaction pour la réaction enzymatique avec s
s = concentration en graisses saturées dans le produit alimentaire
$k_s$ = constante de saturation pour la réaction enzymatique avec s
$v_{max,c}$ = vitesse de réaction pour la réaction enzymatique avec c
c = concentration en cholestérol dans le produit alimentaire.
$k_c$ = constante de réaction pour la réaction enzymatique avec c.

4. Procédé pour l'évaluation d'un produit alimentaire selon la revendication 2, le produit alimentaire de la classe B) étant choisi et pour le calcul de l'index épigénétique pour la classe de produits alimentaires B), la formule IV suivante étant utilisée, la densité d'énergie et les acides gras saturés ainsi que les glucides indiqués en tant qu'indice glycémique étant introduits dans la formule :

$$\text{index épigénétique}_{B)} = [v_{max,d}/k_d/(k_d+d)/100]*[s/(s+k_s)*(v_{max,s})]*[v_{max,g}/k_g/(g^3 + k_g)],$$

dans laquelle

$k_d$ = constante d'inhibition pour la réaction enzymatique avec d
$V_{max,d}$ = vitesse de réaction pour la réaction enzymatique avec d
d = densité d'énergie du produit alimentaire
$v_{max,s}$ = vitesse de réaction pour la réaction enzymatique avec s
s = concentration en graisses saturées dans le produit alimentaire
$k_s$ = constante de saturation pour la réaction enzymatique avec s
$v_{max,g}$ = vitesse de réaction pour la réaction enzymatique avec g
g = concentration en glucides dans le produit alimentaire indiquée en tant que qu'indice glycémique du produit alimentaire
$k_g$ = constante de réaction pour la réaction enzymatique avec g.

5. Procédé pour l'évaluation d'un produit alimentaire selon la revendication 2, le produit alimentaire de la classe C) étant choisi et pour le calcul de l'index épigénétique pour la classe de produits alimentaires C), la formule V suivante étant utilisée, la densité d'énergie et les acides gras saturés ainsi que les acides gras oméga-3 étant introduits dans

la formule :

$$\text{index épigénétique}_{C)} = [V_{max,d}/k_d/(k_d + d)/100] * [s/(s+k_s)*(V_{max,s})] * [V_{max,o}*(o + k_o^2)/o],$$

dans laquelle

$k_d$ = constante d'inhibition pour la réaction enzymatique avec d
$V_{max,d}$ = vitesse de réaction pour la réaction enzymatique avec d
d = densité d'énergie du produit alimentaire
$v_{max,s}$ = vitesse de réaction pour la réaction enzymatique s
s = concentration en graisses saturées dans le produit alimentaire
$k_s$ = constante de saturation pour la réaction enzymatique avec s,
$V_{max,o}$ = vitesse de réaction pour la réaction enzymatique avec o
o = concentration en acide gras oméga-3 dans le produit alimentaire
$k_o$ = constante de réaction pour la réaction enzymatique avec o.

**6.** Procédé pour l'évaluation d'un produit alimentaire selon la revendication 2, le produit alimentaire de la classe E) étant choisi et pour le calcul de l'index épigénétique pour la classe de produits alimentaires E), la formule VI suivante étant utilisée, la densité d'énergie et les fibres alimentaires ainsi que l'acide folique étant introduits dans la formule :

$$\text{index épigénétique}_{E)} = [V_{max,d}/k_d/(k_d + d)/100] * [(k_b^2 + b)/b/v_{max,b}] * [(k_f^2 + f)/f/v_{max,f}],$$

dans laquelle

$k_d$ = constante d'inhibition pour la réaction enzymatique avec d
$V_{max,d}$ = vitesse de réaction pour la réaction enzymatique d
d = densité d'énergie du produit alimentaire
$v_{max,b}$ = vitesse de réaction pour la réaction enzymatique avec b
b = concentration en fibres alimentaires dans le produit alimentaire
$k_b$ = constante de réaction pour la réaction enzymatique avec b
$v_{max,f}$ = vitesse de réaction pour la réaction enzymatique avec f
f = concentration en acide folique dans le produit alimentaire
$k_f$ = constante de réaction pour la réaction enzymatique avec f.

**7.** Procédé pour l'évaluation d'un produit alimentaire selon la revendication 2, le produit alimentaire de la classe F) étant choisi et pour le calcul de l'index épigénétique pour les classes de produits alimentaires F), la formule VII suivante étant utilisée, la densité d'énergie et les fibres alimentaires ainsi que les glucides indiqués en tant qu'indice glycémique étant introduits dans la formule :

$$\text{index épigénétique}_{F)} = [V_{max,d}/k_d/(k_d+d)/100] * [(k_b^2+b)/b/v_{max,b}] * [V_{max,g}/k_g/(g^3 + k_g)],$$

dans laquelle

$k_d$ = constante d'inhibition pour la réaction enzymatique avec d
$v_{max,d}$ = vitesse de réaction pour la réaction d
d = densité d'énergie du produit alimentaire

$v_{max,b}$ = vitesse de réaction pour la réaction enzymatique avec b

b = concentration en fibres alimentaires dans le produit alimentaire

$k_b$ = constante de réaction pour la réaction enzymatique avec b

$v_{max,g}$ = vitesse de réaction pour la réaction enzymatique avec g

g = concentration en glucides dans le produit alimentaire indiquée en tant que qu'indice glycémique du produit alimentaire

$k_g$ = constante de réaction pour la réaction enzymatique avec g.

8. Procédé pour l'évaluation d'un produit alimentaire selon la revendication 2, le produit alimentaire des classes A) à D) étant choisi et l'au moins un ingrédient épigénétiquement actif du produit alimentaire étant les graisses saturées.

9. Procédé pour l'évaluation d'un produit alimentaire selon la revendication 8, une densité d'énergie du produit alimentaire supérieure à 250 kcal/100 g et une concentration en graisses saturées de 0 à 5 g/100 g ou supérieure à 5 g/100 g signifiant un effet négatif du produit alimentaire sur le métabolisme énergétique du mammifère, une densité d'énergie du produit alimentaire de 150 à 250 kcal/ 100 g et une concentration de graisses saturées de 0 à 5 g/100 g ou supérieure à 5 g/100 g signifiant un effet neutre du produit alimentaire sur le métabolisme énergétique du mammifère, une densité d'énergie du produit alimentaire jusqu'à 150 kcal/100 g et une concentration de graisses saturées supérieure à 5 g/100 g signifiant un effet neutre du produit alimentaire sur le métabolisme énergétique du mammifère, et une densité d'énergie du produit alimentaire jusqu'à 150 kcal/100 g et une concentration en graisses saturées de 0 à 5 g/100 g signifiant un effet positif du produit alimentaire sur le métabolisme énergétique du mammifère.

10. Procédé pour l'évaluation d'un produit alimentaire selon la revendication 2, le produit alimentaire des classes E) à F) étant choisi et l'au moins un ingrédient épigénétiquement actif du produit alimentaire étant les fibres alimentaires.

11. Procédé pour l'évaluation d'un produit alimentaire selon la revendication 10, une densité d'énergie supérieure à 250 kcal/100 g et une concentration en fibres alimentaires de 0 à 0,5 g/100 g signifiant un effet négatif du produit alimentaire sur le métabolisme énergétique du mammifère, une densité énergétique du produit alimentaire supérieure à 250 kcal/100 g ou une densité énergétique du produit alimentaire de 150 à 250 kcal/100 g et une concentration en fibres alimentaires de 0 à 0,5 /100 g ou supérieure à 0,5 g/100 g signifiant un effet neutre du produit alimentaire sur le métabolisme énergétique du mammifère, une densité d'énergie du produit alimentaire de 150 kcal/100 g et une concentration en fibres alimentaires de 0 à 0,1 g/100 g signifiant un effet neutre du produit alimentaire sur le métabolisme énergétique du mammifère, et une densité d'énergie du produit alimentaire jusqu'à 150 kcal/100 g et une concentration en fibres alimentaires de 0,1 à 5 g/100 g ou supérieure à 0,5 g/100 g signifiant un effet positif du produit alimentaire sur le métabolisme énergétique du mammifère.

12. Procédé pour l'évaluation d'un produit alimentaire selon la revendication 1, la valeur obtenue pour l'index épigénétique (IPE)$_{A)-D)}$ d'après la formule I étant multipliée par la quantité de graisses saturées du produit alimentaire par rapport à la quantité du produit alimentaire.

**Figur 1A**

| Fleisch | s (ges. Fette in g) | ks | vmax | =s/(s+ks) | =s/(s+ks)*(vmax) |
|---|---|---|---|---|---|
| Schwein Leber gekocht | 1.39 | 1 | 3 | 0.56 | 1.67 |
| Hackfleisch vom Schwein gegart | 11.20 | 1 | 3 | 0.91 | 2.73 |
| Brustspitz Schwein mittelfett gegrillt | 9.05 | 1 | 3 | 0.89 | 2.67 |
| Schweinebraten mittelfett geschmort | 8.77 | 1 | 3 | 0.89 | 2.67 |
| Rindsbrust (Spannrippe) gebraten | 11.30 | 1 | 3 | 0.91 | 2.73 |
| Lammkotelett | 7.50 | 1 | 3 | 0.87 | 2.62 |
| Schweinespeck geräuchert | 12.40 | 1 | 3 | 0.92 | 2.76 |
| Schweineschnitzel paniert gebraten | 3.80 | 1 | 3 | 0.78 | 2.33 |
| Hackfleisch vom Rind gegart | 7.78 | 1 | 3 | 0.88 | 2.63 |
| Schweinshaxe geschmort | 4.64 | 1 | 3 | 0.81 | 2.43 |
| Poulet ganz mit Haut gegrillt | 2.71 | 1 | 3 | 0.71 | 2.13 |
| Cordonbleu Schwein | 5.80 | 1 | 3 | 0.84 | 2.52 |
| Schweinskotelett mittelfett gegart | 4.67 | 1 | 3 | 0.81 | 2.43 |
| Kalbskotellet mittelfett gegart | 2.72 | 1 | 3 | 0.71 | 2.14 |
| Pouletflügeli gegrillt | 4.05 | 1 | 3 | 0.79 | 2.36 |
| Schweinschnitzel mittelfett gegrillt | 2.35 | 1 | 3 | 0.68 | 2.04 |
| Cordonbleu Kalb | 5.04 | 1 | 3 | 0.82 | 2.46 |
| Rindfleisch, Keule mager gebraten | 2.26 | 1 | 3 | 0.67 | 2.02 |
| Wienerschnitzel | 4.66 | 1 | 3 | 0.81 | 2.43 |
| Rind Schnitzel mager gegart | 2.08 | 1 | 3 | 0.65 | 1.96 |
| Rinderbraten mager geschmort | 2.50 | 1 | 3 | 0.69 | 2.08 |
| Schweinesteak mager gegrillt | 2.24 | 1 | 3 | 0.67 | 2.01 |
| Lammfilet | 1.98 | 1 | 3 | 0.64 | 1.93 |
| Kaninchen geschmort | 2.24 | 1 | 3 | 0.67 | 2.01 |
| Kalbshaxe mager gegart | 1.88 | 1 | 3 | 0.63 | 1.89 |
| Rehpfeffer | 6.45 | 1 | 3 | 0.85 | 2.56 |
| Rindsfilet mager gebraten | 2.12 | 1 | 3 | 0.66 | 1.98 |
| Rindsrücken (Roastbeef) | 2.09 | 1 | 3 | 0.66 | 1.97 |
| Kalb Leber gekocht | 0.31 | 1 | 3 | 0.22 | 0.66 |
| Schweinkotlett mager gegart | 2.33 | 1 | 3 | 0.68 | 2.04 |
| Kalbsschnitzel mager gebraten | 0.67 | 1 | 3 | 0.38 | 1.13 |
| Hirsch mittelfett gekocht | 1.34 | 1 | 3 | 0.55 | 1.65 |
| Kalbsfilet mager gegart | 0.64 | 1 | 3 | 0.37 | 1.10 |
| Reh Fleisch mager gekocht | 1.10 | 1 | 3 | 0.50 | 1.50 |
| Kalbsbraten mager gegart | 0.41 | 1 | 3 | 0.27 | 0.81 |
| Schweinsschnitzel mager gebraten | 0.59 | 1 | 3 | 0.35 | 1.05 |
| Truthahn Brust gebraten | 0.33 | 1 | 3 | 0.23 | 0.68 |
| Pouletbrust gebraten | 0.13 | 1 | 3 | 0.11 | 0.32 |
| Eintopf mit Fleisch | 2.4 | 1 | 3.00 | 0.68 | 2 |
| Salami | 12.9 | 1 | 3 | 0.92 | 2.76 |
| Wienerli | 10.8 | 1 | 3 | 0.91 | 2.72 |
| Lyoner Wurst | 10.9 | 1 | 3 | 0.91 | 2.73 |
| Fleischkäse | 3.4 | 1 | 3 | 0.76 | 2.27 |
| Bündnerfleisch | 2 | 1 | 3 | 0.65 | 1.94 |

## Figur 1B

| Fleisch | c (Cholesterin in mg) | kc | vmax | $=kc/(c^2+k_c)$ | $=vmax /$ $kc/(c^2+kc)$ |
|---|---|---|---|---|---|
| Schwein Leber gekocht | 408 | 9000 | 1 | 0.05 | 19.50 |
| Hackfleisch vom Schwein gegart | 94 | 9000 | 1 | 0.50 | 1.98 |
| Brustspitz Schwein mittelfett gegrillt | 99 | 9000 | 1 | 0.48 | 2.09 |
| Schweinebraten mittelfett geschmort | 100 | 9000 | 1 | 0.47 | 2.11 |
| Rindsbrust (Spannrippe) gebraten | 84 | 9000 | 1 | 0.56 | 1.78 |
| Lammkotelett | 91 | 9000 | 1 | 0.52 | 1.92 |
| Schweinespeck geräuchert | 64 | 9000 | 1 | 0.69 | 1.46 |
| Schweineschnitzel paniert gebraten | 118 | 9000 | 1 | 0.39 | 2.55 |
| Hackfleisch vom Rind gegart | 83 | 9000 | 1 | 0.57 | 1.77 |
| Schweinshaxe geschmort | 101 | 9000 | 1 | 0.47 | 2.13 |
| Poulet ganz mit Haut gegrillt | 129 | 9000 | 1 | 0.35 | 2.85 |
| Cordonbleu Schwein | 87 | 9000 | 1 | 0.54 | 1.84 |
| Schweinskotelett mittelfett gegart | 82 | 9000 | 1 | 0.57 | 1.75 |
| Kalbskotellet mittelfett gegart | 104 | 9000 | 1 | 0.45 | 2.20 |
| Pouletflügeli gegrillt | 86 | 9000 | 1 | 0.55 | 1.82 |
| Schweinschnitzel mittelfett gegrillt | 101 | 9000 | 1 | 0.47 | 2.13 |
| Cordonbleu Kalb | 74 | 9000 | 1 | 0.62 | 1.61 |
| Rindfleisch, Keule mager gebraten | 102 | 9000 | 1 | 0.46 | 2.16 |
| Wienerschnitzel | 61 | 9000 | 1 | 0.71 | 1.41 |
| Rind Schnitzel mager gegart | 104 | 9000 | 1 | 0.45 | 2.20 |
| Rinderbraten mager geschmort | 84 | 9000 | 1 | 0.56 | 1.78 |
| Schweinesteak mager gegrillt | 80 | 9000 | 1 | 0.58 | 1.71 |
| Lammfilet | 80 | 9000 | 1 | 0.58 | 1.71 |
| Kaninchen geschmort | 92 | 9000 | 1 | 0.52 | 1.94 |
| Kalbshaxe mager gegart | 94 | 9000 | 1 | 0.50 | 1.98 |
| Rehpfeffer | 37 | 9000 | 1 | 0.87 | 1.15 |
| Rindsfilet mager gebraten | 74 | 9000 | 1 | 0.62 | 1.61 |
| Rindsrücken (Roastbeef) | 71 | 9000 | 1 | 0.64 | 1.56 |
| Kalb Leber gekocht | 254 | 9000 | 1 | 0.12 | 8.17 |
| Schweinkotlett mager gegart | 55 | 9000 | 1 | 0.75 | 1.34 |
| Kalbsschnitzel mager gebraten | 102 | 9000 | 1 | 0.46 | 2.16 |
| Hirsch mittelfett gekocht | 72 | 9000 | 1 | 0.63 | 1.58 |
| Kalbsfilet mager gegart | 86 | 9000 | 1 | 0.55 | 1.82 |
| Reh Fleisch mager gekocht | 66 | 9000 | 1 | 0.67 | 1.48 |
| Kalbsbraten mager gegart | 94 | 9000 | 1 | 0.50 | 1.98 |
| Schweinsschnitzel mager gebraten | 53 | 9000 | 1 | 0.76 | 1.31 |
| Truthahn Brust gebraten | 48 | 9000 | 1 | 0.80 | 1.26 |
| Pouletbrust gebraten | 48 | 9000 | 1 | 0.80 | 1.26 |
| Eintopf mit Fleisch | 91 | 9000 | 1 | 0.52 | 1.92 |
| Salami | 79 | 9000 | 1 | 0.59 | 1.69 |
| Wienerli | 78 | 9000 | 1 | 0.60 | 1.68 |
| Lyoner Wurst | 72 | 9000 | 1 | 0.63 | 1.58 |
| Fleischkäse | 61 | 9000 | 1 | 0.71 | 1.41 |
| Bündnerfleisch | 49 | 9000 | 1 | 0.79 | 1.27 |

**Figur 1C**

| Fleisch | d (Dichte kcal/100g) | kd | vmax | =kd/(kd+d) | =vmax / kd/(kd+d) /100 | EPIlnde> |
|---|---|---|---|---|---|---|
| Schwein Leber gekocht | 130 | 1 | 1 | 0.01 | 1.25 | 27 |
| Hackfleisch vom Schwein gegart | 332 | 1 | 1 | 0.00 | 3.17 | 17 |
| Brustspitz Schwein mittelfett gegrillt | 295 | 1 | 1 | 0.00 | 2.82 | 16 |
| Schweinebraten mittelfett geschmort | 288 | 1 | 1 | 0.00 | 2.75 | 15 |
| Rindsbrust (Spannrippe) gebraten | 318 | 1 | 1 | 0.00 | 3.04 | 15 |
| Lammkotelett | 282 | 1 | 1 | 0.00 | 2.70 | 14 |
| Schweinespeck geräuchert | 347 | 1 | 1 | 0.00 | 3.31 | 13 |
| Schweineschnitzel paniert gebraten | 227 | 1 | 1 | 0.00 | 2.17 | 13 |
| Hackfleisch vom Rind gegart | 266 | 1 | 1 | 0.00 | 2.54 | 12 |
| Schweinshaxe geschmort | 227 | 1 | 1 | 0.00 | 2.17 | 11 |
| Poulet ganz mit Haut gegrillt | 188 | 1 | 1 | 0.01 | 1.80 | 11 |
| Cordonbleu Schwein | 217 | 1 | 1 | 0.00 | 2.07 | 10 |
| Schweinskotelett mittelfett gegart | 218 | 1 | 1 | 0.00 | 2.09 | 9 |
| Kalbskotelett mittelfett gegart | 183 | 1 | 1 | 0.01 | 1.75 | 8 |
| Pouletflügeli gegrillt | 194 | 1 | 1 | 0.01 | 1.86 | 8 |
| Schweinschnitzel mittelfett gegrillt | 179 | 1 | 1 | 0.01 | 1.71 | 7 |
| Cordonbleu Kalb | 183 | 1 | 1 | 0.01 | 1.75 | 7 |
| Rindfleisch, Keule mager gebraten | 163 | 1 | 1 | 0.01 | 1.56 | 7 |
| Wienerschnitzel | 200 | 1 | 1 | 0.01 | 1.91 | 7 |
| Rind Schnitzel mager gegart | 156 | 1 | 1 | 0.01 | 1.50 | 6 |
| Rinderbraten mager geschmort | 164 | 1 | 1 | 0.01 | 1.57 | 6 |
| Schweinesteak mager gegrillt | 177 | 1 | 1 | 0.01 | 1.70 | 6 |
| Lammfilet | 178 | 1 | 1 | 0.01 | 1.71 | 6 |
| Kaninchen geschmort | 149 | 1 | 1 | 0.01 | 1.43 | 6 |
| Kalbshaxe mager gegart | 153 | 1 | 1 | 0.01 | 1.47 | 6 |
| Rehpfeffer | 188 | 1 | 1 | 0.01 | 1.80 | 5 |
| Rindsfilet mager gebraten | 163 | 1 | 1 | 0.01 | 1.56 | 5 |
| Rindsrücken (Roastbeef) | 167 | 1 | 1 | 0.01 | 1.60 | 5 |
| Kalb Leber gekocht | 89 | 1 | 1 | 0.01 | 0.86 | 5 |
| Schweinkotlett mager gegart | 176 | 1 | 1 | 0.01 | 1.69 | 5 |
| Kalbsschnitzel mager gebraten | 141 | 1 | 1 | 0.01 | 1.35 | 3 |
| Hirsch mittelfett gekocht | 114 | 1 | 1 | 0.01 | 1.10 | 3 |
| Kalbsfilet mager gegart | 136 | 1 | 1 | 0.01 | 1.31 | 3 |
| Reh Fleisch mager gekocht | 101 | 1 | 1 | 0.01 | 0.97 | 2 |
| Kalbsbraten mager gegart | 128 | 1 | 1 | 0.01 | 1.23 | 2 |
| Schweinsschnitzel mager gebraten | 111 | 1 | 1 | 0.01 | 1.07 | 1 |
| Truthahn Brust gebraten | 110 | 1 | 1 | 0.01 | 1.06 | 1 |
| Pouletbrust gebraten | 74 | 1 | 1 | 0.01 | 0.71 | 1 |
| Eintopf mit Fleisch | 351 | 1 | 1 | 0.003 | 3.35 | 13 |
| Salami | 375 | 1 | 1 | 0.003 | 3.58 | 17 |
| Wienerli | 301 | 1 | 1 | 0.003 | 2.88 | 13 |
| Lyoner Wurst | 306 | 1 | 1 | 0.003 | 2.92 | 13 |
| Fleischkäse | 250 | 1 | 1 | 0.004 | 2.39 | 8 |
| Bündnerfleisch | 211 | 1 | 1 | 0.005 | 2.02 | 5 |

**Figur 2A**

| Beilagen | s (ges. Fette in g) | ks | vmax | =s/(s+ks) | =s/(s+ks)*(vmax) = Wert |
|---|---|---|---|---|---|
| Kartoffel Chips | 9.93 | 1 | 3 | 0.90 | 2.70 |
| Pommes Frites | 5.10 | 1 | 3 | 0.82 | 2.47 |
| Kartoffelgratin | 6.30 | 1 | 3 | 0.85 | 2.55 |
| Spaghetti Carbonara | 5.67 | 1 | 3 | 0.84 | 2.51 |
| Kartoffelkroketten | 5.29 | 1 | 3 | 0.83 | 2.48 |
| Kartoffelstock Trockenprodukt | 2.10 | 1 | 3 | 0.66 | 1.97 |
| Kartoffelsalat | 2.00 | 1 | 3 | 0.65 | 1.94 |
| Ebly | 0.70 | 1 | 3 | 0.39 | 1.17 |
| Kartoffelstock | 1.35 | 1 | 3 | 0.55 | 1.65 |
| Polenta | 3.60 | 1 | 3 | 0.77 | 2.30 |
| Kartoffeln gebraten | 0.60 | 1 | 3 | 0.35 | 1.06 |
| Risotto | 0.60 | 1 | 3 | 0.35 | 1.06 |
| Fertigreis | 0.60 | 1 | 3 | 0.35 | 1.06 |
| Hirse | 0.30 | 1 | 3 | 0.21 | 0.64 |
| Bulgur | 0.55 | 1 | 3 | 0.33 | 1.00 |
| Spätzli | 0.40 | 1 | 3 | 0.27 | 0.80 |
| Spaghetti Napoli | 0.38 | 1 | 3 | 0.26 | 0.77 |
| Gnocchi | 0.14 | 1 | 3 | 0.11 | 0.33 |
| Süsskartofffel (Kumara) | 0.23 | 1 | 3 | 0.17 | 0.52 |
| Spaghett mit Ei | 0.19 | 1 | 3 | 0.14 | 0.43 |
| Gerste | 0.15 | 1 | 3 | 0.12 | 0.35 |
| Couscous | 0.10 | 1 | 3 | 0.08 | 0.25 |
| Kartoffeln gebacken | 0.10 | 1 | 3 | 0.08 | 0.25 |
| Vollkornrisotto | 0.18 | 1 | 3 | 0.14 | 0.43 |
| Vollkornteigwaren | 0.16 | 1 | 3 | 0.13 | 0.39 |
| Vollkornnudeln mit Ei | 0.16 | 1 | 3 | 0.13 | 0.38 |
| Salzkartoffeln | 0.10 | 1 | 3 | 0.08 | 0.25 |
| Teigwaren mit Ei | 0.15 | 1 | 3 | 0.12 | 0.37 |
| Quinoa | 0.18 | 1 | 3 | 0.14 | 0.42 |
| Teigwaren eifrei | 0.10 | 1 | 3 | 0.08 | 0.25 |
| Wildreis | 0.09 | 1 | 3 | 0.07 | 0.22 |
| Basmatireis | 0.07 | 1 | 3 | 0.06 | 0.18 |
| Reis parboiled | 0.10 | 1 | 3 | 0.08 | 0.25 |
| Linsen | 0.11 | 1 | 3 | 0.09 | 0.27 |
| Kartoffeln in Schale | 0.10 | 1 | 3 | 0.08 | 0.25 |
| Spaghetti eifrei | 0.07 | 1 | 3 | 0.06 | 0.17 |
| Süsskartofffel (Yam) | 0.10 | 1 | 3 | 0.08 | 0.25 |
| Griess | 0.10 | 1 | 3 | 0.08 | 0.25 |

**Figur 2B**

| Beilagen | g (Glykämie) | kg | vmax | $=kg/(g^3+k_g)$ | $=vmax / kg/(g^3+kg)$ |
|---|---|---|---|---|---|
| Kartoffel Chips | 50 | 300000 | 1 | 0.71 | 1.42 |
| Pommes Frites | 75 | 300000 | 1 | 0.42 | 2.41 |
| Kartoffelgratin | 75 | 300000 | 1 | 0.42 | 2.41 |
| Spaghetti Carbonara | 50 | 300000 | 1 | 0.71 | 1.42 |
| Kartoffelkroketten | 75 | 300000 | 1 | 0.42 | 2.41 |
| Kartoffelstock Trockenprodukt | 85 | 300000 | 1 | 0.33 | 3.05 |
| Kartoffelsalat | 75 | 300000 | 1 | 0.42 | 2.41 |
| Ebly | 30 | 300000 | 1 | 0.92 | 1.09 |
| Kartoffelstock | 75 | 300000 | 1 | 0.42 | 2.41 |
| Polenta | 70 | 300000 | 1 | 0.47 | 2.14 |
| Kartoffeln gebraten | 80 | 300000 | 1 | 0.37 | 2.71 |
| Risotto | 70 | 300000 | 1 | 0.47 | 2.14 |
| Fertigreis | 69 | 300000 | 1 | 0.48 | 2.10 |
| Hirse | 70 | 300000 | 1 | 0.47 | 2.14 |
| Bulgur | 48 | 300000 | 1 | 0.73 | 1.37 |
| Spätzli | 60 | 300000 | 1 | 0.58 | 1.72 |
| Spaghetti Napoli | 50 | 300000 | 1 | 0.71 | 1.42 |
| Gnocchi | 68 | 300000 | 1 | 0.49 | 2.05 |
| Süsskartofffel (Kumara) | 61 | 300000 | 1 | 0.57 | 1.76 |
| Spaghett mit Ei | 50 | 300000 | 1 | 0.71 | 1.42 |
| Gerste | 60 | 300000 | 1 | 0.58 | 1.72 |
| Couscous | 65 | 300000 | 1 | 0.52 | 1.92 |
| Kartoffeln gebacken | 85 | 300000 | 1 | 0.33 | 3.05 |
| Vollkornrisotto | 54 | 300000 | 1 | 0.66 | 1.52 |
| Vollkornteigwaren | 35 | 300000 | 1 | 0.87 | 1.14 |
| Vollkornnudeln mit Ei | 35 | 300000 | 1 | 0.87 | 1.14 |
| Salzkartoffeln | 75 | 300000 | 1 | 0.42 | 2.41 |
| Teigwaren mit Ei | 45 | 300000 | 1 | 0.77 | 1.30 |
| Quinoa | 35 | 300000 | 1 | 0.87 | 1.14 |
| Teigwaren eifrei | 45 | 300000 | 1 | 0.77 | 1.30 |
| Wildreis | 54 | 300000 | 1 | 0.66 | 1.52 |
| Basmatireis | 60 | 300000 | 1 | 0.58 | 1.72 |
| Reis parboiled | 45 | 300000 | 1 | 0.77 | 1.30 |
| Linsen | 30 | 300000 | 1 | 0.92 | 1.09 |
| Kartoffeln in Schale | 75 | 300000 | 1 | 0.42 | 2.41 |
| Spaghetti eifrei | 50 | 300000 | 1 | 0.71 | 1.42 |
| Süsskartofffel (Yam) | 37 | 300000 | 1 | 0.86 | 1.17 |
| Griess | 55 | 300000 | 1 | 0.64 | 1.55 |

**Figur 2C**

| Beilagen | d (Dichte kcal/100g) | kd | vmax | =kd/(kd+d) | =vmax / kd/(kd+d) /100 | EPIIndex |
|---|---|---|---|---|---|---|
| Kartoffel Chips | 554 | 1 | 1 | 0.00 | 5.29 | 20 |
| Pommes Frites | 329 | 1 | 1 | 0.00 | 3.14 | 19 |
| Kartoffelgratin | 168 | 1 | 1 | 0.01 | 1.61 | 10 |
| Spaghetti Carbonara | 263 | 1 | 1 | 0.00 | 2.51 | 9 |
| Kartoffelkroketten | 150 | 1 | 1 | 0.01 | 1.44 | 9 |
| Kartoffelstock Trockenprodukt | 109 | 1 | 1 | 0.01 | 1.05 | 6 |
| Kartoffelsalat | 102 | 1 | 1 | 0.01 | 0.98 | 5 |
| Ebly | 299 | 1 | 1 | 0.00 | 2.86 | 4 |
| Kartoffelstock | 93 | 1 | 1 | 0.01 | 0.90 | 4 |
| Polenta | 68 | 1 | 1 | 0.02 | 0.66 | 3 |
| Kartoffeln gebraten | 102 | 1 | 1 | 0.01 | 0.98 | 3 |
| Risotto | 116 | 1 | 1 | 0.01 | 1.11 | 3 |
| Fertigreis | 93 | 1 | 1 | 0.01 | 0.89 | 2 |
| Hirse | 127 | 1 | 1 | 0.01 | 1.22 | 2 |
| Bulgur | 122 | 1 | 1 | 0.01 | 1.17 | 2 |
| Spätzli | 116 | 1 | 1 | 0.01 | 1.11 | 2 |
| Spaghetti Napoli | 128 | 1 | 1 | 0.01 | 1.23 | 1 |
| Gnocchi | 150 | 1 | 1 | 0.01 | 1.44 | 1 |
| Süsskartofffel (Kumara) | 107 | 1 | 1 | 0.01 | 1.03 | 1 |
| Spaghett mit Ei | 145 | 1 | 1 | 0.01 | 1.39 | 1 |
| Gerste | 115 | 1 | 1 | 0.01 | 1.11 | 1 |
| Couscous | 142 | 1 | 1 | 0.01 | 1.36 | 1 |
| Kartoffeln gebacken | 87 | 1 | 1 | 0.01 | 0.84 | 1 |
| Vollkornrisotto | 97 | 1 | 1 | 0.01 | 0.93 | 1 |
| Vollkornteigwaren | 143 | 1 | 1 | 0.01 | 1.37 | 1 |
| Vollkornnudeln mit Ei | 139 | 1 | 1 | 0.01 | 1.33 | 1 |
| Salzkartoffeln | 87 | 1 | 1 | 0.01 | 0.84 | 1 |
| Teigwaren mit Ei | 99 | 1 | 1 | 0.01 | 0.95 | 1 |
| Quinoa | 98 | 1 | 1 | 0.01 | 0.94 | 1 |
| Teigwaren eifrei | 139 | 1 | 1 | 0.01 | 1.33 | 1 |
| Wildreis | 134 | 1 | 1 | 0.01 | 1.29 | 1 |
| Basmatireis | 131 | 1 | 1 | 0.01 | 1.26 | 1 |
| Reis parboiled | 121 | 1 | 1 | 0.01 | 1.16 | 1 |
| Linsen | 122 | 1 | 1 | 0.01 | 1.17 | 1 |
| Kartoffeln in Schale | 57 | 1 | 1 | 0.02 | 0.55 | 1 |
| Spaghetti eifrei | 129 | 1 | 1 | 0.01 | 1.24 | 1 |
| Süsskartofffel (Yam) | 98 | 1 | 1 | 0.01 | 0.94 | 1 |
| Griess | 34.2 | 1 | 1 | 0.03 | 0.34 | 1 |

**Figur 3A**

| Brotwaren | s (ges. Fette in g) | ks | vmax | =s/(s+ks) | =s/(s+ks)*(vmax) = Wert |
|---|---|---|---|---|---|
| Kuchenteig | 10.00 | 1 | 3 | 0.90 | 2.70 |
| Blätterteig gebacken | 20.80 | 1 | 3 | 0.95 | 2.85 |
| Butterkekse | 6.63 | 1 | 3 | 0.86 | 2.57 |
| Gipfeli | 10.50 | 1 | 3 | 0.91 | 2.72 |
| Butterzopf | 4.26 | 1 | 3 | 0.79 | 2.38 |
| Laugenbretzel | 1.19 | 1 | 3 | 0.52 | 1.56 |
| Reiswaffel/Puffreis | 0.54 | 1 | 3 | 0.33 | 0.99 |
| Baguette | 0.40 | 1 | 3 | 0.27 | 0.80 |
| Dinkelvollkornbrot | 0.54 | 1 | 3 | 0.33 | 0.99 |
| Knäckebrot | 0.35 | 1 | 3 | 0.24 | 0.73 |
| Weissbrot | 0.33 | 1 | 3 | 0.23 | 0.70 |
| Ruchbrot | 0.26 | 1 | 3 | 0.19 | 0.58 |
| Gerstenkornbrot | 0.26 | 1 | 3 | 0.19 | 0.58 |
| Weizenvollkornbrot | 0.17 | 1 | 3 | 0.13 | 0.40 |
| Roggenvollkornbrot | 0.17 | 1 | 3 | 0.13 | 0.40 |
| Grahambrot | 0.24 | 1 | 3 | 0.18 | 0.53 |
| Pumpernickel | 0.11 | 1 | 3 | 0.09 | 0.27 |
| Buchweizen Vollkorn | 0.10 | 1 | 3 | 0.08 | 0.25 |
| Pizza napolitane | 3.46 | 1 | 3 | 0.76 | 2.28 |
| Pizza quattro stagione | 2.43 | 1 | 3 | 0.69 | 2.07 |

**Figur 3B**

| Brotwaren | g (Glykämie) | kg | vmax | =kg/(g3+kg) | =vmax / kg/(g3+kg) |
|---|---|---|---|---|---|
| Kuchenteig | 95 | 300000 | 1 | 0.26 | 3.86 |
| Blätterteig gebacken | 70 | 300000 | 1 | 0.47 | 2.14 |
| Butterkekse | 65 | 300000 | 1 | 0.52 | 1.92 |
| Gipfeli | 70 | 300000 | 1 | 0.47 | 2.14 |
| Butterzopf | 85 | 300000 | 1 | 0.33 | 3.05 |
| Laugenbretzel | 73 | 300000 | 1 | 0.44 | 2.30 |
| Reiswaffel/Puffreis | 78 | 300000 | 1 | 0.39 | 2.58 |
| Baguette | 95 | 300000 | 1 | 0.26 | 3.86 |
| Dinkelvollkornbrot | 60 | 300000 | 1 | 0.58 | 1.72 |
| Knäckebrot | 60 | 300000 | 1 | 0.58 | 1.72 |
| Weissbrot | 73 | 300000 | 1 | 0.44 | 2.30 |
| Ruchbrot | 65 | 300000 | 1 | 0.52 | 1.92 |
| Gerstenkornbrot | 25 | 300000 | 1 | 0.95 | 1.05 |
| Weizenvollkornbrot | 60 | 300000 | 1 | 0.58 | 1.72 |
| Roggenvollkornbrot | 55 | 300000 | 1 | 0.64 | 1.55 |
| Grahambrot | 25 | 300000 | 1 | 0.95 | 1.05 |
| Pumpernickel | 45 | 300000 | 1 | 0.77 | 1.30 |
| Buchweizen Vollkorn | 40 | 300000 | 1 | 0.82 | 1.21 |
| Pizza napolitane | 60 | 300000 | 1 | 0.58 | 1.72 |
| Pizza quattro stagione | 60 | 300000 | 1 | 0.58 | 1.72 |

**8/22**

**Figur 3C**

| Brotwaren | d (Dichte kcal/100g) | kd | vmax | =kd/(kd+d) | =vmax / kd/(kd+d) /100 | EPIIndex |
|---|---|---|---|---|---|---|
| Kuchenteig | 403 | 1 | 1 | 0.00 | 3.85 | 27 |
| Blätterteig gebacken | 420 | 1 | 1 | 0.00 | 4.01 | 24 |
| Butterkekse | 434 | 1 | 1 | 0.00 | 4.14 | 20 |
| Gipfeli | 363 | 1 | 1 | 0.00 | 3.47 | 20 |
| Butterzopf | 289 | 1 | 1 | 0.00 | 2.76 | 20 |
| Laugenbretzel | 300 | 1 | 1 | 0.00 | 2.87 | 10 |
| Reiswaffel/Puffreis | 395 | 1 | 1 | 0.00 | 3.77 | 10 |
| Baguette | 284 | 1 | 1 | 0.00 | 2.71 | 8 |
| Dinkelvollkornbrot | 246 | 1 | 1 | 0.00 | 2.35 | 4 |
| Knäckebrot | 322 | 1 | 1 | 0.00 | 3.08 | 4 |
| Weissbrot | 242 | 1 | 1 | 0.00 | 2.31 | 4 |
| Ruchbrot | 225 | 1 | 1 | 0.00 | 2.15 | 2 |
| Gerstenkornbrot | 225 | 1 | 1 | 0.00 | 2.15 | 1 |
| Weizenvollkornbrot | 198 | 1 | 1 | 0.01 | 1.90 | 1 |
| Roggenvollkornbrot | 198 | 1 | 1 | 0.01 | 1.90 | 1 |
| Grahambrot | 204 | 1 | 1 | 0.01 | 1.95 | 1 |
| Pumpernickel | 195 | 1 | 1 | 0.01 | 1.87 | 1 |
| Buchweizen Vollkorn | 126 | 1 | 1 | 0.01 | 1.21 | 1 |
| Pizza napolitane | 231 | 1 | 1 | 0.0045 | 2.21 | 9 |
| Pizza quattro stagione | 189 | 1 | 1 | 0.0055 | 1.81 | 6 |

**Figur 4A**

| Fisch und Krustentiere | s (ges. Fette in g) | ks | vmax | =s/(s+ks) | =s/(s+ks)*(vmax) = Wert |
|---|---|---|---|---|---|
| Fischstäbchen | 7.55 | 1 | 3 | 0.87 | 2.62 |
| Seeteufel gebraten | 1.73 | 1 | 3 | 0.61 | 1.83 |
| Thunfisch Konserve in Öl abgetropft | 3.79 | 1 | 3 | 0.78 | 2.33 |
| Sardine Konserve in Öl | 2.84 | 1 | 3 | 0.72 | 2.16 |
| Thunfisch gegart | 4.95 | 1 | 3 | 0.82 | 2.45 |
| Makrele gegart | 3.76 | 1 | 3 | 0.77 | 2.32 |
| Lachsfilet gebraten | 4.01 | 1 | 3 | 0.78 | 2.35 |
| Lachs geräuchert | 3.06 | 1 | 3 | 0.74 | 2.21 |
| Pangasius | 0.79 | 1 | 3 | 0.42 | 1.25 |
| Hering mager gegart | 2.37 | 1 | 3 | 0.68 | 2.05 |
| Lachs gekocht | 3.15 | 1 | 3 | 0.74 | 2.22 |
| Hering Konserve mager abgetropft | 2.06 | 1 | 3 | 0.65 | 1.96 |
| Seezunge gegart | 0.39 | 1 | 3 | 0.26 | 0.79 |
| Sardelle gebraten (ohne Fett) | 0.82 | 1 | 3 | 0.43 | 1.28 |
| Thunfisch Konserve nature abgetropft | 0.15 | 1 | 3 | 0.12 | 0.36 |
| Bachsaibling gebraten (ohne Fett) | 0.60 | 1 | 3 | 0.35 | 1.06 |
| Sardine gegart | 1.40 | 1 | 3 | 0.56 | 1.68 |
| Miesmuschel gekocht | 0.65 | 1 | 3 | 0.37 | 1.12 |
| Forelle gegart | 0.71 | 1 | 3 | 0.39 | 1.18 |
| Jakobsmuschel | 0.25 | 1 | 3 | 0.19 | 0.56 |
| Venusmuschel | 0.25 | 1 | 3 | 0.19 | 0.56 |
| Sardine Konserve abgetropft | 1.21 | 1 | 3 | 0.52 | 1.57 |
| Forelle geräuchert | 0.65 | 1 | 3 | 0.37 | 1.11 |
| Zander gebraten | 0.16 | 1 | 3 | 0.13 | 0.38 |
| Shrimps gegart | 0.24 | 1 | 3 | 0.18 | 0.54 |
| Krabben gekocht | 0.29 | 1 | 3 | 0.21 | 0.63 |
| Heilbutt gekocht | 0.29 | 1 | 3 | 0.21 | 0.62 |
| Kabeljau/Dorsch gebraten (ohne Fett) | 0.14 | 1 | 3 | 0.11 | 0.33 |
| Hecht gegart | 0.11 | 1 | 3 | 0.09 | 0.28 |

**Figur 4B**

| Fisch und Krustentiere | o (Omega-3 in g) | ko | vmax | =(o+ko*ko)/o | =vmax * (o+ko*ko)/o |
|---|---|---|---|---|---|
| Fischstäbchen | 0.25 | 1.3 | 1 | 7.76 | 7.76 |
| Seeteufel gebraten | 0.25 | 1.3 | 1 | 7.73 | 7.73 |
| Thunfisch Konserve in Öl abgetropft | 2.24 | 1.3 | 1 | 1.75 | 1.75 |
| Sardine Konserve in Öl | 1.60 | 1.3 | 1 | 2.06 | 2.06 |
| Thunfisch gegart | 4.92 | 1.3 | 1 | 1.34 | 1.34 |
| Makrele gegart | 2.52 | 1.3 | 1 | 1.67 | 1.67 |
| Lachsfilet gebraten | 3.03 | 1.3 | 1 | 1.56 | 1.56 |
| Lachs geräuchert | 3.02 | 1.3 | 1 | 1.56 | 1.56 |
| Pangasius | 0.30 | 1.3 | 1 | 6.63 | 6.63 |
| Hering mager gegart | 2.13 | 1.3 | 1 | 1.79 | 1.79 |
| Lachs gekocht | 3.10 | 1.3 | 1 | 1.55 | 1.55 |
| Hering Konserve mager abgetropft | 1.85 | 1.3 | 1 | 1.91 | 1.91 |
| Seezunge gegart | 0.27 | 1.3 | 1 | 7.21 | 7.21 |
| Sardelle gebraten (ohne Fett) | 0.72 | 1.3 | 1 | 3.35 | 3.35 |
| Thunfisch Konserve nature abgetropft | 0.14 | 1.3 | 1 | 13.34 | 13.34 |
| Bachsaibling gebraten (ohne Fett) | 0.61 | 1.3 | 1 | 3.79 | 3.79 |
| Sardine gegart | 1.81 | 1.3 | 1 | 1.93 | 1.93 |
| Miesmuschel gekocht | 0.42 | 1.3 | 1 | 5.05 | 5.05 |
| Forelle gegart | 0.89 | 1.3 | 1 | 2.89 | 2.89 |
| Jakobsmuschel | 0.20 | 1.3 | 1 | 9.54 | 9.54 |
| Venusmuschel | 0.20 | 1.3 | 1 | 9.54 | 9.54 |
| Sardine Konserve abgetropft | 1.57 | 1.3 | 1 | 2.08 | 2.08 |
| Forelle geräuchert | 0.81 | 1.3 | 1 | 3.09 | 3.09 |
| Zander gebraten | 0.22 | 1.3 | 1 | 8.58 | 8.58 |
| Shrimps gegart | 0.44 | 1.3 | 1 | 4.86 | 4.86 |
| Krabben gekocht | 0.52 | 1.3 | 1 | 4.24 | 4.24 |
| Heilbutt gekocht | 0.57 | 1.3 | 1 | 3.98 | 3.98 |
| Kabeljau/Dorsch gebraten (ohne Fett) | 0.29 | 1.3 | 1 | 6.93 | 6.93 |
| Hecht gegart | 0.34 | 1.3 | 1 | 5.99 | 5.99 |

**Figur 4C**

| Fisch und Krustentiere | d (Dichte kcal/100g) | k d | vma x | =kd/(kd+d ) | =vmax / kd/(kd+d ) /100 | EPIInde x |
|---|---|---|---|---|---|---|
| Fischstäbchen | 253 | 1 | 1 | 0.00 | 2.42 | 27 |
| Seeteufel gebraten | 95 | 1 | 1 | 0.01 | 0.91 | 13 |
| Thunfisch Konserve in Öl abgetropft | 281 | 1 | 1 | 0.00 | 2.69 | 11 |
| Sardine Konserve in Öl | 221 | 1 | 1 | 0.00 | 2.11 | 9 |
| Thunfisch gegart | 250 | 1 | 1 | 0.00 | 2.39 | 8 |
| Makrele gegart | 204 | 1 | 1 | 0.01 | 1.95 | 8 |
| Lachsfilet gebraten | 208 | 1 | 1 | 0.01 | 1.99 | 7 |
| Lachs geräuchert | 190 | 1 | 1 | 0.01 | 1.82 | 6 |
| Pangasius | 77 | 1 | 1 | 0.01 | 0.74 | 6 |
| Hering mager gegart | 175 | 1 | 1 | 0.01 | 1.68 | 6 |
| Lachs gekocht | 183 | 1 | 1 | 0.01 | 1.75 | 6 |
| Hering Konserve mager abgetropft | 152 | 1 | 1 | 0.01 | 1.46 | 5 |
| Seezunge gegart | 96 | 1 | 1 | 0.01 | 0.92 | 5 |
| Sardelle gebraten (ohne Fett) | 115 | 1 | 1 | 0.01 | 1.11 | 5 |
| Thunfisch Konserve nature abgetropft | 100 | 1 | 1 | 0.01 | 0.96 | 5 |
| Bachsaibling gebraten (ohne Fett) | 112 | 1 | 1 | 0.01 | 1.08 | 4 |
| Sardine gegart | 136 | 1 | 1 | 0.01 | 1.31 | 4 |
| Miesmuschel gekocht | 73 | 1 | 1 | 0.01 | 0.71 | 4 |
| Forelle gegart | 121 | 1 | 1 | 0.01 | 1.16 | 4 |
| Jakobsmuschel | 77 | 1 | 1 | 0.01 | 0.74 | 4 |
| Venusmuschel | 77 | 1 | 1 | 0.01 | 0.74 | 4 |
| Sardine Konserve abgetropft | 117 | 1 | 1 | 0.01 | 1.12 | 4 |
| Forelle geräuchert | 110 | 1 | 1 | 0.01 | 1.06 | 4 |
| Zander gebraten | 98 | 1 | 1 | 0.01 | 0.94 | 3 |
| Shrimps gegart | 92 | 1 | 1 | 0.01 | 0.89 | 2 |
| Krabben gekocht | 91 | 1 | 1 | 0.01 | 0.88 | 2 |
| Heilbutt gekocht | 93 | 1 | 1 | 0.01 | 0.89 | 2 |
| Kabeljau/Dorsch gebraten (ohne Fett) | 90 | 1 | 1 | 0.01 | 0.87 | 2 |
| Hecht gegart | 97 | 1 | 1 | 0.01 | 0.93 | 2 |

**Figur 5A**

| Milcherzeugnisse | s (ges. Fette in g) | ks | vmax | =s/(s+ks) | =s/(s+ks)*(vmax) = Wert |
|---|---|---|---|---|---|
| Mascarpone | 27.00 | 1 | 3 | 0.96 | 2.88 |
| Frischkäse 70 % Fett i.Tr. | 22.40 | 1 | 3 | 0.95 | 2.86 |
| Weichkäse 60% Fett i. Tr. | 20.10 | 1 | 3 | 0.95 | 2.84 |
| Hartkäse 50% Fett i. Tr. | 18.10 | 1 | 3 | 0.94 | 2.83 |
| Hartkäse 45% Fett i.Tr. | 20.20 | 1 | 3 | 0.95 | 2.85 |
| Emmentaler 45% Fett i. Tr. | 18.90 | 1 | 3 | 0.95 | 2.84 |
| Mascarpone léger | 14.00 | 1 | 3 | 0.93 | 2.78 |
| Edamer 45 % Fett | 18.10 | 1 | 3 | 0.94 | 2.83 |
| Hartkäse 30% Fett | 16.70 | 1 | 3 | 0.94 | 2.81 |
| Rahm 30% Fett | 19.00 | 1 | 3 | 0.95 | 2.84 |
| Creme fraiche 30 % Fett | 18.50 | 1 | 3 | 0.94 | 2.83 |
| Raclette Käse 45% Fett i. Tr. | 17.00 | 1 | 3 | 0.94 | 2.82 |
| Schafskäse | 17.50 | 1 | 3 | 0.94 | 2.82 |
| Feta Käse | 16.10 | 1 | 3 | 0.94 | 2.81 |
| Camembertkäse 45% Fett i. Tr. | 13.40 | 1 | 3 | 0.92 | 2.77 |
| Mozarella | 14.30 | 1 | 3 | 0.93 | 2.79 |
| Weichkäse 30% Fett i. Tr. | 7.90 | 1 | 3 | 0.88 | 2.63 |
| Feta Käse légère | 6.00 | 1 | 3 | 0.85 | 2.54 |
| Mozzarella léger | 4.80 | 1 | 3 | 0.81 | 2.44 |
| Quark 50 % Fett | 8.80 | 1 | 3 | 0.89 | 2.67 |
| Kaffeerahm 1.5% Fett | 9.10 | 1 | 3 | 0.89 | 2.68 |
| Ricotta 60% Fett i.Tr. | 9.44 | 1 | 3 | 0.90 | 2.69 |
| Weichkäse 10% Fett i.Tr. | 5.50 | 1 | 3 | 0.83 | 2.50 |
| Weichkäse 20% i. Tr. | 5.46 | 1 | 3 | 0.83 | 2.50 |
| Frischkäse 40 % Fett i. Tr | 6.67 | 1 | 3 | 0.86 | 2.58 |
| Ricotta 30% Fett i.Tr. | 4.80 | 1 | 3 | 0.81 | 2.44 |
| Schafmilch Vollfett | 3.57 | 1 | 3 | 0.76 | 2.29 |
| Hüttenkäse 20% Fett i.Tr. | 2.30 | 1 | 3 | 0.68 | 2.03 |
| Früchtejoghurt 3.5% Fett | 1.60 | 1 | 3 | 0.59 | 1.78 |
| Hartkäse 10% Fett | 0.60 | 1 | 3 | 0.35 | 1.06 |
| Emmentaler 10% Fett i. Tr. | 0.60 | 1 | 3 | 0.35 | 1.06 |
| Kuhmilch 3.5% Fett | 2.60 | 1 | 3 | 0.70 | 2.11 |
| Vollmilchjoghurt nature 3.5% Fett | 2.25 | 1 | 3 | 0.67 | 2.01 |
| Frischkäse 10 % Fett i.Tr. | 1.21 | 1 | 3 | 0.52 | 1.57 |
| Quark mager 10% Fett | 1.20 | 1 | 3 | 0.52 | 1.57 |
| Hüttenkäse <10% Fett i.Tr. | 0.85 | 1 | 3 | 0.44 | 1.31 |
| Früchtejoghurt  1.5 % Fett | 0.80 | 1 | 3 | 0.42 | 1.26 |
| Kuhmilch 2.5 % Fett | 1.50 | 1 | 3 | 0.58 | 1.73 |
| Joghurt nature 1.5% Fett | 1.00 | 1 | 3 | 0.48 | 1.43 |
| Kuhmilch 1.5 % Fett | 0.99 | 1 | 3 | 0.47 | 1.42 |
| Sauermilch 1.5% Fett | 0.91 | 1 | 3 | 0.45 | 1.36 |
| Sojamilch | 0.40 | 1 | 3 | 0.27 | 0.80 |
| Reismilch | 0.20 | 1 | 3 | 0.15 | 0.46 |
| Buttermilch | 0.31 | 1 | 3 | 0.22 | 0.66 |
| Blanc battu 0.2% | 0.10 | 1 | 3 | 0.08 | 0.25 |
| Butter | 53.8 | 1 | 3 | 0.98 | 2.94 |
| Margarine halbfett | 9.52 | 1 | 3 | 0.90 | 2.69 |

**Figur 5B**

| Milcherzeugnisse | c (Cholesterin in mg) | kc | vmax | =kc/(c$^2$+kc) | =vmax / kc/(c$^2$+kc) |
|---|---|---|---|---|---|
| Mascarpone | 119 | 9000 | 1 | 0.39 | 2.57 |
| Frischkäse 70 % Fett i.Tr. | 122 | 9000 | 1 | 0.38 | 2.65 |
| Weichkäse 60% Fett i. Tr. | 93 | 9000 | 1 | 0.51 | 1.96 |
| Hartkäse 50% Fett i. Tr. | 85 | 9000 | 1 | 0.55 | 1.80 |
| Hartkäse 45% Fett i.Tr. | 83 | 9000 | 1 | 0.57 | 1.77 |
| Emmentaler 45% Fett i. Tr. | 83 | 9000 | 1 | 0.57 | 1.77 |
| Mascarpone léger | 119 | 9000 | 1 | 0.39 | 2.57 |
| Edamer 45 % Fett | 75 | 9000 | 1 | 0.62 | 1.63 |
| Hartkäse 30% Fett | 69 | 9000 | 1 | 0.65 | 1.53 |
| Rahm 30% Fett | 84 | 9000 | 1 | 0.56 | 1.78 |
| Creme fraiche 30 % Fett | 86 | 9000 | 1 | 0.55 | 1.82 |
| Raclette Käse 45% Fett i. Tr. | 65 | 9000 | 1 | 0.68 | 1.47 |
| Schafskäse | 69 | 9000 | 1 | 0.65 | 1.53 |
| Feta Käse | 69 | 9000 | 1 | 0.65 | 1.53 |
| Camembertkäse 45% Fett i. Tr. | 59 | 9000 | 1 | 0.72 | 1.39 |
| Mozarella | 65 | 9000 | 1 | 0.68 | 1.47 |
| Weichkäse 30% Fett i. Tr. | 34 | 9000 | 1 | 0.89 | 1.13 |
| Feta Käse légère | 69 | 9000 | 1 | 0.65 | 1.53 |
| Mozzarella léger | 65 | 9000 | 1 | 0.68 | 1.47 |
| Quark 50 % Fett | 43 | 9000 | 1 | 0.83 | 1.21 |
| Kaffeerahm 1.5% Fett | 52 | 9000 | 1 | 0.77 | 1.30 |
| Ricotta 60% Fett i.Tr. | 40 | 9000 | 1 | 0.85 | 1.18 |
| Weichkäse 10% Fett i.Tr. | 21 | 9000 | 1 | 0.95 | 1.05 |
| Weichkäse 20% i. Tr. | 21 | 9000 | 1 | 0.95 | 1.05 |
| Frischkäse 40 % Fett i. Tr | 29 | 9000 | 1 | 0.91 | 1.09 |
| Ricotta 30% Fett i.Tr. | 31 | 9000 | 1 | 0.90 | 1.11 |
| Schafmilch Vollfett | 11 | 9000 | 1 | 0.99 | 1.01 |
| Hüttenkäse 20% Fett i.Tr. | 11 | 9000 | 1 | 0.99 | 1.01 |
| Früchtejoghurt 3.5% Fett | 12 | 9000 | 1 | 0.98 | 1.02 |
| Hartkäse 10% Fett | 2 | 9000 | 1 | 1.00 | 1.00 |
| Emmentaler 10% Fett i. Tr. | 2 | 9000 | 1 | 1.00 | 1.00 |
| Kuhmilch 3.5% Fett | 10 | 9000 | 1 | 0.99 | 1.01 |
| Vollmilchjoghurt nature 3.5% Fett | 10 | 9000 | 1 | 0.99 | 1.01 |
| Frischkäse 10 % Fett i.Tr. | 5 | 9000 | 1 | 1.00 | 1.00 |
| Quark mager 10% Fett | 7 | 9000 | 1 | 0.99 | 1.01 |
| Hüttenkäse <10% Fett i.Tr. | 5 | 9000 | 1 | 1.00 | 1.00 |
| Früchtejoghurt 1.5 % Fett | 4 | 9000 | 1 | 1.00 | 1.00 |
| Kuhmilch 2.5 % Fett | 7 | 9000 | 1 | 0.99 | 1.01 |
| Joghurt nature 1.5% Fett | 4 | 9000 | 1 | 1.00 | 1.00 |
| Kuhmilch 1.5 % Fett | 4 | 9000 | 1 | 1.00 | 1.00 |
| Sauermilch 1.5% Fett | 6 | 9000 | 1 | 1.00 | 1.00 |
| Sojamilch | 0 | 9000 | 1 | 1.00 | 1.00 |
| Reismilch | 0 | 9000 | 1 | 1.00 | 1.00 |
| Buttermilch | 1 | 9000 | 1 | 1.00 | 1.00 |
| Blanc battu 0.2% | 7 | 9000 | 1 | 0.99 | 1.01 |
| Butter | 1 | 9000 | 1 | 1.00 | 1.00 |
| Margarine halbfett | 21 | 9000 | 1 | 0.95 | 1.05 |

**Figur 5C**

| Milcherzeugnisse | d (Dichte kcal/100g) | kd | vmax | =kd/(kd+d) | =vmax / kd/(kd+d) /100 | EPIIndex |
|---|---|---|---|---|---|---|
| Mascarpone | 387 | 1 | 1 | 0.0027 | 3.70 | 27 |
| Frischkäse 70 % Fett i.Tr. | 377 | 1 | 1 | 0.0028 | 3.60 | 27 |
| Weichkäse 60% Fett i. Tr. | 362 | 1 | 1 | 0.0029 | 3.46 | 19 |
| Hartkäse 50% Fett i. Tr. | 396 | 1 | 1 | 0.0026 | 3.78 | 19 |
| Hartkäse 45% Fett i.Tr. | 378 | 1 | 1 | 0.0028 | 3.61 | 18 |
| Emmentaler 45% Fett i. Tr. | 378 | 1 | 1 | 0.0028 | 3.61 | 18 |
| Mascarpone léger | 248 | 1 | 1 | 0.0042 | 2.37 | 17 |
| Edamer 45 % Fett | 354 | 1 | 1 | 0.0030 | 3.38 | 16 |
| Hartkäse 30% Fett | 374 | 1 | 1 | 0.0028 | 3.57 | 15 |
| Rahm 30% Fett | 303 | 1 | 1 | 0.0035 | 2.90 | 15 |
| Creme fraiche 30 % Fett | 277 | 1 | 1 | 0.0038 | 2.65 | 14 |
| Raclette Käse 45% Fett i. Tr. | 343 | 1 | 1 | 0.0031 | 3.28 | 14 |
| Schafskäse | 284 | 1 | 1 | 0.0037 | 2.71 | 12 |
| Feta Käse | 284 | 1 | 1 | 0.0037 | 2.71 | 12 |
| Camembertkäse 45% Fett i. Tr. | 284 | 1 | 1 | 0.0037 | 2.71 | 10 |
| Mozarella | 263 | 1 | 1 | 0.0040 | 2.51 | 10 |
| Weichkäse 30% Fett i. Tr. | 211 | 1 | 1 | 0.0050 | 2.02 | 6 |
| Feta Käse légère | 161 | 1 | 1 | 0.0065 | 1.54 | 6 |
| Mozzarella léger | 164 | 1 | 1 | 0.0064 | 1.57 | 6 |
| Quark 50 % Fett | 176 | 1 | 1 | 0.0059 | 1.69 | 5 |
| Kaffeerahm 1.5% Fett | 160 | 1 | 1 | 0.0065 | 1.53 | 5 |
| Ricotta 60% Fett i.Tr. | 174 | 1 | 1 | 0.0060 | 1.67 | 5 |
| Weichkäse 10% Fett i.Tr. | 178 | 1 | 1 | 0.0059 | 1.71 | 4 |
| Weichkäse 20% i. Tr. | 178 | 1 | 1 | 0.0059 | 1.71 | 4 |
| Frischkäse 40 % Fett i. Tr | 156 | 1 | 1 | 0.0067 | 1.50 | 4 |
| Ricotta 30% Fett i.Tr. | 121 | 1 | 1 | 0.0086 | 1.16 | 3 |
| Schafmilch Vollfett | 94 | 1 | 1 | 0.0110 | 0.91 | 2 |
| Hüttenkäse 20% Fett i.Tr. | 104 | 1 | 1 | 0.0100 | 1.00 | 2 |
| Früchtejoghurt 3.5% Fett | 104 | 1 | 1 | 0.0100 | 1.00 | 2 |
| Hartkäse 10% Fett | 167 | 1 | 1 | 0.0062 | 1.60 | 2 |
| Emmentaler 10% Fett i. Tr. | 167 | 1 | 1 | 0.0062 | 1.60 | 2 |
| Kuhmilch 3.5% Fett | 68 | 1 | 1 | 0.0152 | 0.66 | 1 |
| Vollmilchjoghurt nature 3.5% Fett | 69 | 1 | 1 | 0.0150 | 0.67 | 1 |
| Frischkäse 10 % Fett i.Tr. | 87 | 1 | 1 | 0.0119 | 0.84 | 1 |
| Quark mager 10% Fett | 83 | 1 | 1 | 0.0125 | 0.80 | 1 |
| Hüttenkäse <10% Fett i.Tr. | 81 | 1 | 1 | 0.0128 | 0.78 | 1 |
| Früchtejoghurt  1.5 % Fett | 83 | 1 | 1 | 0.0125 | 0.80 | 1 |
| Kuhmilch 2.5 % Fett | 55 | 1 | 1 | 0.0187 | 0.53 | 1 |
| Joghurt nature 1.5% Fett | 50 | 1 | 1 | 0.0206 | 0.49 | 1 |
| Kuhmilch 1.5 % Fett | 48 | 1 | 1 | 0.0214 | 0.47 | 1 |
| Sauermilch 1.5% Fett | 46 | 1 | 1 | 0.0223 | 0.45 | 1 |
| Sojamilch | 47 | 1 | 1 | 0.0219 | 0.46 | 1 |
| Reismilch | 56 | 1 | 1 | 0.0184 | 0.54 | 1 |
| Buttermilch | 38 | 1 | 1 | 0.0269 | 0.37 | 1 |
| Blanc battu 0.2% | 53 | 1 | 1 | 0.0194 | 0.51 | 1 |
| Butter | 741 | 1 | 1 | 0.0014 | 7.07 | 21 |
| Margarine halbfett | 362 | 1 | 1 | 0.0029 | 3.46 | 10 |

**Figur 6A**

| Gemüse und Salat | b (Ballaststoffe in g) | k b | vma x | =(kb*kb+b)/ b | =(kb*kb+b)/b/vma x |
|---|---|---|---|---|---|
| Kichererbsen getrocknet | 15.50 | 1 | 1 | 1.020 | 1.020 |
| Kidney-Bohnen | 21.30 | 1 | 1 | 1.014 | 1.014 |
| Oliven schwarz gesäuert | 7.87 | 1 | 1 | 1.038 | 1.038 |
| Oliven grün gesäuert | 2.36 | 1 | 1 | 1.128 | 1.128 |
| Sojabohnen reif | 10.10 | 1 | 1 | 1.030 | 1.030 |
| Avocado | 4.10 | 1 | 1 | 1.074 | 1.074 |
| Zuckermais | 2.90 | 1 | 1 | 1.104 | 1.104 |
| Lattich | 1.14 | 1 | 1 | 1.265 | 1.265 |
| Sojasprossen | 2.38 | 1 | 1 | 1.127 | 1.127 |
| Meerrettich | 7.50 | 1 | 1 | 1.040 | 1.040 |
| Zwiebel | 1.40 | 1 | 1 | 1.216 | 1.216 |
| Pepperoni | 3.60 | 1 | 1 | 1.084 | 1.084 |
| Karotten | 3.10 | 1 | 1 | 1.098 | 1.098 |
| Grünkohl | 4.20 | 1 | 1 | 1.072 | 1.072 |
| Zucchini | 1.10 | 1 | 1 | 1.275 | 1.275 |
| Grünkohl | 3.51 | 1 | 1 | 1.086 | 1.086 |
| Rhabarber | 3.20 | 1 | 1 | 1.095 | 1.095 |
| Kohlrabi | 1.50 | 1 | 1 | 1.202 | 1.202 |
| Lauch gegart | 2.33 | 1 | 1 | 1.130 | 1.130 |
| Schnittlauch frisch | 6.00 | 1 | 1 | 1.050 | 1.050 |
| Weisskohl | 3.00 | 1 | 1 | 1.101 | 1.101 |
| Blumenkohl | 3.10 | 1 | 1 | 1.098 | 1.098 |
| Austernpilze | 6.44 | 1 | 1 | 1.047 | 1.047 |
| Rotkohl | 2.50 | 1 | 1 | 1.121 | 1.121 |
| Romanesco | 3.00 | 1 | 1 | 1.101 | 1.101 |
| Tomaten | 1.30 | 1 | 1 | 1.233 | 1.233 |
| Artischocke | 11.20 | 1 | 1 | 1.027 | 1.027 |
| Fenchel | 2.00 | 1 | 1 | 1.151 | 1.151 |
| Spinat | 1.80 | 1 | 1 | 1.168 | 1.168 |
| Rettich | 1.20 | 1 | 1 | 1.252 | 1.252 |
| Chicorée | 1.30 | 1 | 1 | 1.233 | 1.233 |
| Sauerkraut | 2.14 | 1 | 1 | 1.141 | 1.141 |
| Radieschen | 1.50 | 1 | 1 | 1.202 | 1.202 |
| Knollensellerie | 4.20 | 1 | 1 | 1.072 | 1.072 |
| Gurke | 0.90 | 1 | 1 | 1.336 | 1.336 |
| Pfifferling | 3.64 | 1 | 1 | 1.083 | 1.083 |
| Feldsalat (Nüssler) | 1.80 | 1 | 1 | 1.168 | 1.168 |
| Endivie | 1.90 | 1 | 1 | 1.159 | 1.159 |
| Chinakohl | 1.70 | 1 | 1 | 1.178 | 1.178 |
| Spargel | 1.31 | 1 | 1 | 1.231 | 1.231 |
| Kopfsalat | 1.40 | 1 | 1 | 1.216 | 1.216 |

**Figur 6B**

| Gemüse und Salat | f (Folsäure in µg) | kf | vmax | =(kf*kf+f)/f | =(kf*kf+f)/f / vmax |
|---|---|---|---|---|---|
| Kichererbsen getrocknet | 340 | 1.6 | 1 | 1.01 | 1.01 |
| Kidney-Bohnen | 182 | 1.6 | 1 | 1.01 | 1.01 |
| Oliven schwarz gesäuert | 44 | 1.6 | 1 | 1.06 | 1.06 |
| Oliven grün gesäuert | 50 | 1.6 | 1 | 1.05 | 1.05 |
| Sojabohnen reif | 55 | 1.6 | 1 | 1.05 | 1.05 |
| Avocado | 20 | 1.6 | 1 | 1.13 | 1.13 |
| Zuckermais | 43 | 1.6 | 1 | 1.06 | 1.06 |
| Lattich | 42.9 | 1.6 | 1 | 1.06 | 1.06 |
| Sojasprossen | 16 | 1.6 | 1 | 1.16 | 1.16 |
| Meerrettich | 26 | 1.6 | 1 | 1.10 | 1.10 |
| Zwiebel | 11 | 1.6 | 1 | 1.23 | 1.23 |
| Pepperoni | 55 | 1.6 | 1 | 1.05 | 1.05 |
| Karotten | 17 | 1.6 | 1 | 1.15 | 1.15 |
| Grünkohl | 187 | 1.6 | 1 | 1.01 | 1.01 |
| Zucchini | 10 | 1.6 | 1 | 1.26 | 1.26 |
| Grünkohl | 98 | 1.6 | 1 | 1.03 | 1.03 |
| Rhabarber | 2 | 1.6 | 1 | 2.28 | 2.28 |
| Kohlrabi | 70 | 1.6 | 1 | 1.04 | 1.04 |
| Lauch gegart | 68 | 1.6 | 1 | 1.04 | 1.04 |
| Schnittlauch frisch | 80 | 1.6 | 1 | 1.03 | 1.03 |
| Weisskohl | 27 | 1.6 | 1 | 1.09 | 1.09 |
| Blumenkohl | 35 | 1.6 | 1 | 1.07 | 1.07 |
| Austernpilze | 34 | 1.6 | 1 | 1.08 | 1.08 |
| Rotkohl | 35 | 1.6 | 1 | 1.07 | 1.07 |
| Romanesco | 33 | 1.6 | 1 | 1.08 | 1.08 |
| Tomaten | 33 | 1.6 | 1 | 1.08 | 1.08 |
| Artischocke | 38 | 1.6 | 1 | 1.07 | 1.07 |
| Fenchel | 37 | 1.6 | 1 | 1.07 | 1.07 |
| Spinat | 141 | 1.6 | 1 | 1.02 | 1.02 |
| Rettich | 24 | 1.6 | 1 | 1.11 | 1.11 |
| Chicorée | 50 | 1.6 | 1 | 1.05 | 1.05 |
| Sauerkraut | 31 | 1.6 | 1 | 1.08 | 1.08 |
| Radieschen | 24 | 1.6 | 1 | 1.11 | 1.11 |
| Knollensellerie | 76 | 1.6 | 1 | 1.03 | 1.03 |
| Gurke | 14 | 1.6 | 1 | 1.18 | 1.18 |
| Pfifferling | 14 | 1.6 | 1 | 1.18 | 1.18 |
| Feldsalat (Nüssler) | 145 | 1.6 | 1 | 1.02 | 1.02 |
| Endivie | 109 | 1.6 | 1 | 1.02 | 1.02 |
| Chinakohl | 66 | 1.6 | 1 | 1.04 | 1.04 |
| Spargel | 60 | 1.6 | 1 | 1.04 | 1.04 |
| Kopfsalat | 41 | 1.6 | 1 | 1.06 | 1.06 |

**Figur 6C**

| Gemüse und Salat | d (Dichte kcal/100g) | kd | vmax | =kd/(kd+d) | =vmax / kd/(kd+d) /100 | EPIIndex |
|---|---|---|---|---|---|---|
| Kichererbsen getrocknet | 310 | 1 | 1 | 0.0034 | 2.9624 | 3 |
| Kidney-Bohnen | 251 | 1 | 1 | 0.0042 | 2.4005 | 2 |
| Oliven schwarz gesäuert | 185 | 1 | 1 | 0.0056 | 1.7719 | 2 |
| Oliven grün gesäuert | 143 | 1 | 1 | 0.0073 | 1.3719 | 2 |
| Sojabohnen reif | 147 | 1 | 1 | 0.0071 | 1.4100 | 2 |
| Avocado | 130 | 1 | 1 | 0.0080 | 1.2481 | 2 |
| Zuckermais | 89 | 1 | 1 | 0.0117 | 0.8576 | 1 |
| Lattich | 66 | 1 | 1 | 0.0157 | 0.6386 | 1 |
| Sojasprossen | 55 | 1 | 1 | 0.0187 | 0.5338 | 1 |
| Meerrettich | 63 | 1 | 1 | 0.0164 | 0.6100 | 1 |
| Zwiebel | 28 | 1 | 1 | 0.0361 | 0.2767 | 1 |
| Pepperoni | 37 | 1 | 1 | 0.0276 | 0.3624 | 1 |
| Karotten | 33 | 1 | 1 | 0.0308 | 0.3243 | 1 |
| Grünkohl | 37 | 1 | 1 | 0.0276 | 0.3624 | 1 |
| Zucchini | 21 | 1 | 1 | 0.0476 | 0.2100 | 1 |
| Grünkohl | 31 | 1 | 1 | 0.0332 | 0.3014 | 1 |
| Rhabarber | 13 | 1 | 1 | 0.0747 | 0.1338 | 1 |
| Kohlrabi | 25 | 1 | 1 | 0.0403 | 0.2481 | 1 |
| Lauch gegart | 26 | 1 | 1 | 0.0388 | 0.2576 | 1 |
| Schnittlauch frisch | 28 | 1 | 1 | 0.0361 | 0.2767 | 1 |
| Weisskohl | 24 | 1 | 1 | 0.0419 | 0.2386 | 1 |
| Blumenkohl | 24 | 1 | 1 | 0.0419 | 0.2386 | 1 |
| Austernpilze | 25 | 1 | 1 | 0.0403 | 0.2481 | 1 |
| Rotkohl | 23 | 1 | 1 | 0.0437 | 0.2290 | 1 |
| Romanesco | 23 | 1 | 1 | 0.0437 | 0.2290 | 1 |
| Tomaten | 18 | 1 | 1 | 0.0551 | 0.1814 | 1 |
| Artischocke | 22 | 1 | 1 | 0.0456 | 0.2195 | 1 |
| Fenchel | 19 | 1 | 1 | 0.0524 | 0.1910 | 1 |
| Spinat | 19 | 1 | 1 | 0.0524 | 0.1910 | 1 |
| Rettich | 16 | 1 | 1 | 0.0616 | 0.1624 | 1 |
| Chicorée | 17 | 1 | 1 | 0.0582 | 0.1719 | 1 |
| Sauerkraut | 17 | 1 | 1 | 0.0582 | 0.1719 | 1 |
| Radieschen | 15 | 1 | 1 | 0.0654 | 0.1529 | 1 |
| Knollensellerie | 18 | 1 | 1 | 0.0551 | 0.1814 | 1 |
| Gurke | 12 | 1 | 1 | 0.0805 | 0.1243 | 1 |
| Pfifferling | 15 | 1 | 1 | 0.0654 | 0.1529 | 1 |
| Feldsalat (Nüssler) | 15 | 1 | 1 | 0.0654 | 0.1529 | 1 |
| Endivie | 15 | 1 | 1 | 0.0654 | 0.1529 | 1 |
| Chinakohl | 14 | 1 | 1 | 0.0698 | 0.1433 | 1 |
| Spargel | 13 | 1 | 1 | 0.0747 | 0.1338 | 1 |
| Kopfsalat | 11 | 1 | 1 | 0.0871 | 0.1148 | 1 |

**Figur 7A**

| Früchte/Obst/Beeren | b (Ballaststoffe in g) | kb | vmax | =(kb*kb+b)/b | =(kb*kb+b)/b/vmax |
|---|---|---|---|---|---|
| Datteln | 8.7 | 1 | 1 | 1.035 | 1.035 |
| Weinbeeren getrocknet | 6.7 | 1 | 1 | 1.045 | 1.045 |
| Wassermelone | 0.2 | 1 | 1 | 2.513 | 2.513 |
| Banane reif | 2.0 | 1 | 1 | 1.151 | 1.151 |
| Honigmelonen | 0.7 | 1 | 1 | 1.414 | 1.414 |
| Trauben blau | 1.6 | 1 | 1 | 1.189 | 1.189 |
| Kirschen, süss | 1.9 | 1 | 1 | 1.159 | 1.159 |
| Banane gelb/grün | 2.0 | 1 | 1 | 1.151 | 1.151 |
| Ananas frisch | 1.4 | 1 | 1 | 1.210 | 1.210 |
| Banane grün | 2.0 | 1 | 1 | 1.151 | 1.151 |
| Trauben grün | 1.6 | 1 | 1 | 1.189 | 1.189 |
| Mango | 1.7 | 1 | 1 | 1.178 | 1.178 |
| Granatapfel | 2.2 | 1 | 1 | 1.135 | 1.135 |
| Passionsfrucht | 1.4 | 1 | 1 | 1.216 | 1.216 |
| Kiwi | 3.9 | 1 | 1 | 1.078 | 1.078 |
| Aprikose | 1.5 | 1 | 1 | 1.202 | 1.202 |
| Feige | 2.0 | 1 | 1 | 1.151 | 1.151 |
| Apfel | 2.0 | 1 | 1 | 1.151 | 1.151 |
| Mandarine | 1.8 | 1 | 1 | 1.173 | 1.173 |
| Kirschen, sauer | 1.9 | 1 | 1 | 1.159 | 1.159 |
| Grapefruit | 0.6 | 1 | 1 | 1.504 | 1.504 |
| Birne | 2.8 | 1 | 1 | 1.108 | 1.108 |
| Pflaumen | 1.7 | 1 | 1 | 1.178 | 1.178 |
| Papaya | 1.9 | 1 | 1 | 1.159 | 1.159 |
| Orange | 2.2 | 1 | 1 | 1.138 | 1.138 |
| Pfirsich | 1.7 | 1 | 1 | 1.178 | 1.178 |
| Heidelbeeren | 4.9 | 1 | 1 | 1.062 | 1.062 |
| Johannisbeeren schwarz | 3.5 | 1 | 1 | 1.086 | 1.086 |
| Stachelbeere | 3.0 | 1 | 1 | 1.103 | 1.103 |
| Erdbeeren | 2.0 | 1 | 1 | 1.151 | 1.151 |
| Himbeeren | 4.7 | 1 | 1 | 1.064 | 1.064 |
| Johannisbeeren rot | 3.5 | 1 | 1 | 1.086 | 1.086 |

**Figur 7B**

| Früchte/Obst/Beeren | g (Glykämie) | kg | vmax | $=kg/(g^3+k_g)$ | =vmax / $kg/(g^3+k_g)$ |
|---|---|---|---|---|---|
| Datteln | 105 | 300000 | 1 | 0.206 | 4.859 |
| Weinbeeren getrocknet | 65 | 300000 | 1 | 0.522 | 1.915 |
| Wassermelone | 75 | 300000 | 1 | 0.416 | 2.406 |
| Banane reif | 60 | 300000 | 1 | 0.581 | 1.720 |
| Honigmelonen | 65 | 300000 | 1 | 0.522 | 1.915 |
| Trauben blau | 60 | 300000 | 1 | 0.581 | 1.720 |
| Kirschen, süss | 65 | 300000 | 1 | 0.522 | 1.915 |
| Banane gelb/grün | 42 | 300000 | 1 | 0.802 | 1.247 |
| Ananas frisch | 59 | 300000 | 1 | 0.594 | 1.685 |
| Banane grün | 30 | 300000 | 1 | 0.917 | 1.090 |
| Trauben grün | 45 | 300000 | 1 | 0.767 | 1.304 |
| Mango | 55 | 300000 | 1 | 0.643 | 1.555 |
| Granatapfel | 35 | 300000 | 1 | 0.875 | 1.143 |
| Passionsfrucht | 35 | 300000 | 1 | 0.875 | 1.143 |
| Kiwi | 53 | 300000 | 1 | 0.668 | 1.496 |
| Aprikose | 57 | 300000 | 1 | 0.618 | 1.617 |
| Feige | 35 | 300000 | 1 | 0.875 | 1.143 |
| Apfel | 34 | 300000 | 1 | 0.884 | 1.131 |
| Mandarine | 42 | 300000 | 1 | 0.802 | 1.247 |
| Kirschen, sauer | 20 | 300000 | 1 | 0.974 | 1.027 |
| Grapefruit | 26 | 300000 | 1 | 0.945 | 1.059 |
| Birne | 38 | 300000 | 1 | 0.845 | 1.183 |
| Pflaumen | 39 | 300000 | 1 | 0.835 | 1.198 |
| Papaya | 59 | 300000 | 1 | 0.594 | 1.685 |
| Orange | 42 | 300000 | 1 | 0.802 | 1.247 |
| Pfirsich | 43 | 300000 | 1 | 0.790 | 1.265 |
| Heidelbeeren | 40 | 300000 | 1 | 0.824 | 1.213 |
| Johannisbeeren schwarz | 15 | 300000 | 1 | 0.989 | 1.011 |
| Stachelbeere | 25 | 300000 | 1 | 0.950 | 1.052 |
| Erdbeeren | 25 | 300000 | 1 | 0.950 | 1.052 |
| Himbeeren | 25 | 300000 | 1 | 0.950 | 1.052 |
| Johannisbeeren rot | 25 | 300000 | 1 | 0.950 | 1.052 |

**Figur 7C**

| Früchte/Obst/Beeren | d (Dichte kcal/100g) | kd | vmax | =kd/(kd+d) | =vmax / kd/(kd+d) /100 | EPIIndex |
|---|---|---|---|---|---|---|
| Datteln | 280 | 1 | 1 | 0.0037 | 2.6767 | 13 |
| Weinbeeren getrocknet | 291 | 1 | 1 | 0.0036 | 2.7814 | 6 |
| Wassermelone | 38 | 1 | 1 | 0.0269 | 0.3719 | 2 |
| Banane reif | 90 | 1 | 1 | 0.0115 | 0.8671 | 2 |
| Honigmelonen | 55 | 1 | 1 | 0.0187 | 0.5338 | 1 |
| Trauben blau | 70 | 1 | 1 | 0.0148 | 0.6767 | 1 |
| Kirschen, süss | 61 | 1 | 1 | 0.0169 | 0.5910 | 1 |
| Banane gelb/grün | 90 | 1 | 1 | 0.0115 | 0.8671 | 1 |
| Ananas frisch | 56 | 1 | 1 | 0.0184 | 0.5433 | 1 |
| Banane grün | 90 | 1 | 1 | 0.0115 | 0.8671 | 1 |
| Trauben grün | 70 | 1 | 1 | 0.0148 | 0.6767 | 1 |
| Mango | 59 | 1 | 1 | 0.0175 | 0.5719 | 1 |
| Granatapfel | 76 | 1 | 1 | 0.0136 | 0.7338 | 1 |
| Passionsfrucht | 64 | 1 | 1 | 0.0161 | 0.6195 | 1 |
| Kiwi | 54 | 1 | 1 | 0.0191 | 0.5243 | 1 |
| Aprikose | 43 | 1 | 1 | 0.0238 | 0.4195 | 1 |
| Feige | 63 | 1 | 1 | 0.0164 | 0.6100 | 1 |
| Apfel | 61 | 1 | 1 | 0.0169 | 0.5910 | 1 |
| Mandarine | 50 | 1 | 1 | 0.0206 | 0.4862 | 1 |
| Kirschen, sauer | 61 | 1 | 1 | 0.0169 | 0.5910 | 1 |
| Grapefruit | 44 | 1 | 1 | 0.0233 | 0.4290 | 1 |
| Birne | 53 | 1 | 1 | 0.0194 | 0.5148 | 1 |
| Pflaumen | 45 | 1 | 1 | 0.0228 | 0.4386 | 1 |
| Papaya | 32 | 1 | 1 | 0.0318 | 0.3148 | 1 |
| Orange | 44 | 1 | 1 | 0.0233 | 0.4290 | 1 |
| Pfirsich | 41 | 1 | 1 | 0.0250 | 0.4005 | 1 |
| Heidelbeeren | 37 | 1 | 1 | 0.0276 | 0.3624 | 1 |
| Johannisbeeren schwarz | 40 | 1 | 1 | 0.0256 | 0.3910 | 1 |
| Stachelbeere | 37 | 1 | 1 | 0.0276 | 0.3624 | 1 |
| Erdbeeren | 33 | 1 | 1 | 0.0308 | 0.3243 | 1 |
| Himbeeren | 34 | 1 | 1 | 0.0300 | 0.3338 | 1 |
| Johannisbeeren rot | 33 | 1 | 1 | 0.0308 | 0.3243 | 1 |

**Figur 8**

**Figur 9**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- *J Nutr.,* Januar 2013, vol. 143 (1), 93-9
- **KRUMWIDE K H.** Kohlenhydrate richtig schätzen und einschätzen. *MMW-Fortschritte der Medizin Originalien Nr. III,* 2007, vol. 149, 91-96
- **SVEN DAVID MÜLLER ; KATRIN RASCHKE.** Das Kalorien -Nährwert Lexikon. Schlütersche Verlag, 2004